(19) Europäisches Patentamt
European Patent Office
Office européen des brevets

(11) EP 4 112 607 A1

(12) EUROPEAN PATENT APPLICATION
published in accordance with Art. 153(4) EPC

(43) Date of publication:
**04.01.2023 Bulletin 2023/01**

(21) Application number: **21759547.9**

(22) Date of filing: **25.02.2021**

(51) International Patent Classification (IPC):
***C07D 251/52*** (2006.01) ***C07D 251/46*** (2006.01)
***C07D 251/70*** (2006.01) ***A61K 31/53*** (2006.01)
***A61K 9/127*** (2006.01) ***C12N 15/88*** (2006.01)
***A61P 35/00*** (2006.01) ***A61P 31/12*** (2006.01)
***A61P 37/00*** (2006.01)

(52) Cooperative Patent Classification (CPC):
**A61K 9/127; A61K 31/53; A61P 31/12; A61P 35/00; A61P 37/00; C07D 251/46; C07D 251/52; C07D 251/70; C12N 15/88**

(86) International application number:
**PCT/CN2021/077852**

(87) International publication number:
**WO 2021/170034 (02.09.2021 Gazette 2021/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.02.2020 CN 202010128563**

(71) Applicant: **Shenzhen Shenxin Biotechnology Co., Ltd.**
**Shenzhen, Guangdong 518000 (CN)**

(72) Inventor: **LI, Linxian**
**Shenzhen, Guangdong 518000 (CN)**

(74) Representative: **Nederlandsch Octrooibureau**
**P.O. Box 29720**
**2502 LS The Hague (NL)**

# (54) AMINO LIPID COMPOUND, PREPARATION METHOD THEREFOR, AND APPLICATION THEREOF

(57) The present invention discloses an amino lipid compound, a preparation method and use of the same. The present invention also provides lipid particles comprising the amino lipid compound, and use thereof for delivering bioactive agents into cells. The present invention also provides a use of lipid particles comprising the amino lipid as a medicament.

EP 4 112 607 A1

## Description

### Field of the Invention

The present invention relates to an amino lipid compound, a preparation method for the same and lipid particles comprising the amino lipid compound. The lipid particles can be used to deliver bioactive agents into cells. The invention also relates to a use of amino lipid containing lipid particles as a medicament.

### Background of the Invention

Gene therapy refers to introducing target genetic materials into cells of specific tissues through appropriate vectors for proper expression, thereby replacing or correcting the disorder of the structure or function of its own genes, killing diseased cells or enhancing the ability of the body to eliminate diseased cells, etc., so as to achieve the therapeutic purpose. However, nucleic acids are easily hydrolyzed by nucleases, and have a large number of negative charges, which is disadvantageous to phagocytosis by cells. Therefore, it is particularly important to research and develop a gene delivery system with high efficiency, safety and tissue specificity.

The current gene vectors in research can be divided into viral vectors and non-viral vectors. Although viral vectors have high transfection efficiency, the application thereof in clinical treatment is limited by its toxicity, immunogenicity and severe safety issues. Instead, non-viral vectors have attracted the attention of more and more experts and scholars because of their advantages such as easy preparation, transportation and storage, as well as safety, effectiveness and non-immunogenicity.

Non-viral vectors are mainly divided into cationic polymers and cationic liposomes. A cationic polymer refers to an artificially synthesized or naturally formed polymer containing cations. Polyethyleneimine (PEI) is one of the most common cationic polymeric non-viral gene vectors. It can encapsulate negatively charged genes and form nanocomposites which can enter cells by endocytosis and escape from lysosomes to show good transfection efficiency. However, PEI is highly cytotoxic, with the toxicity being directly proportional to the transfection efficiency. Chitosan has good biological safety but low transfection efficiency. Cationic lipid particles are artificially prepared phospholipid vesicles with bilayered membranes. These lipid particles are principally different in their charges carried and fine structures. When the target DNA is mixed with such lipid particles, DNA will be concentrated and form a relatively stable lipid particle-DNA complex (lipoplex) with the particles. Since lipid particles are similar to biomembranes in nature, when the lipid particle-DNA complex comes into contact with the cell membrane, it enters the cell through endocytosis. In addition to high transfection efficiency, lipid particles have no immunogenicity, low cytotoxicity and are easy to prepare. Therefore, cationic lipid particles have become one of the most commonly used vectors for gene transfection.

Although cationic lipid particles have been reported widely and commercial transfection reagents are available, the minor differences between the gene structures to be delivered need to be matched with the charges and fine structures of lipid particles, and it is difficult for one or several transfection reagents to be suitable for delivery of all genetic materials. The minor structural differences in cells into which genetic materials will be transferred also need to be matched with lipid particles of different properties. Therefore, compared with the rapid development of gene therapy and enormous market demands, there is an urgent need to develop a minitype delivery system as a platform to meet the needs of delivering nucleic acid substances with different structures.

### Summary of the Invention

In one aspect, the present invention provides an amino lipid compound which is a compound represented by the following Formula I:

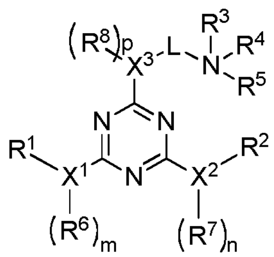

(I)

wherein:

$R^1$ and $R^2$ are the same or different from each other, and are each independently selected from the group consisting of $C_6$-$C_{24}$ alkyl, $C_6$-$C_{24}$ alkenyl, $C_6$-$C_{24}$ alkynyl and $C_4$-$C_{24}$ acyl, wherein the $C_6$-$C_{24}$ alkyl, the $C_6$-$C_{24}$ alkenyl, the $C_6$-$C_{24}$ alkynyl and the $C_4$-$C_{24}$ acyl are optionally substituted with $C_1$-$C_6$ hydrocarbyl;

$X^1$, $X^2$ and $X^3$ are the same or different from one another, and are each independently selected from the group consisting of C, N, O, S, S=O, $S(=O)_2$ and S-S;

when $X^1$ is C, m=2, and two $R^6$ are the same or different from each other; when $X^1$ is N, m=1; when $X^1$ is O, S, S=O, $S(=O)_2$ or S-S, m=0;

when $X^2$ is C, n=2, and two $R^7$ are the same or different from each other; when $X^2$ is N, n=1; when $X^2$ is O, S, S=O, $S(=O)_2$ or S-S, n=0;

when $X^3$ is C, p=2, and two $R^8$ are the same or different from each other; when $X^3$ is N, p=1; when $X^3$ is O, S, S=O, $S(=O)_2$ or S-S, p=0;

$R^3$ and $R^4$ are the same or different from each other, and are each independently selected from the group consisting of $C_1$-$C_{12}$ alkyl, $C_2$-$C_{12}$ alkenyl and $C_2$-$C_{12}$ alkynyl, wherein the $C_1$-$C_{12}$ alkyl, the $C_2$-$C_{12}$ alkenyl and the $C_2$-$C_{12}$ alkynyl are optionally substituted with $C_1$-$C_6$ hydrocarbyl, or $R^3$ and $R^4$ bind to each other to form an optionally substituted 4-10 membered heterocycle containing 1 to 6 heteroatoms selected from nitrogen, sulfur, and oxygen;

$R^5$ is absent, or $R^5$ is hydrogen or $C_1$-$C_{12}$ alkyl to provide a quaternary amine;

$R^6$, $R^7$, $R^8$ are hydrogen or $C_1$-$C_{12}$ alkyl;

L is $C_1$-$C_{12}$ alkylene, $C_2$-$C_{12}$ alkenylene or $C_2$-$C_{12}$ alkynylene, wherein the $C_1$-$C_{12}$ alkylene, the $C_2$-$C_{12}$ alkenylene and the $C_2$-$C_{12}$ alkynylene are optionally substituted with one or more substituents selected from the group consisting of hydrocarbyl, carboxyl, acyl and alkoxy, or L is an optionally substituted 4-10 membered heterocycle containing heteroatoms selected from nitrogen, sulfur, and oxygen.

In another aspect, the present invention provides a method for preparing the amino lipid compound.

In another aspect, the present invention provides a use of the amino lipid compound for preparing lipid particles.

In another aspect, the present invention provides lipid particles comprising the amino lipid compound.

In another aspect, the present invention provides a use of the lipid particles in the preparation of medicaments.

**Brief Description of the Drawings**

Fig. 1 shows the humoral antibody titer produced by subcutaneous administration of ovalbumin mRNA (OVA mRNA) by representative amino lipid compounds.

Fig. 2 shows the microscopic images of HEK293 cells after treatment with a reference reagent (Lipofectamine2000) and S8N12D6 according to Example 4, wherein Fig. 2(a) shows the bright field image of HEK293 cells after treatment with the reference reagent (Lipofectamine2000); Fig. 2(b) shows the bright field image of HEK293 cells after treatment with S8N12D6 according to Example 4; Fig. 2(c) shows the Hoechst staining image of HEK293 nuclei after treatment with the reference reagent (Lipofectamine2000); Fig. 2(d) shows the Hoechst staining image of HEK293 nuclei after treatment with S8N12D6 according to Example 4; Fig. 2(e) shows the GFP image of HEK293 cells after treatment with the reference reagent (Lipofectamine2000); and Fig. 2(f) shows the GFP image of HEK293 cells after treatment with S8N12D6 according to Example 4.

**Detailed Description of the Invention**

The present invention will be further described in detail hereinafter.

As used herein, the term "optionally substituted" means that one or more hydrogen atoms linked to an atom or group are independently unsubstituted or substituted with one or more, e.g., one, two, three or four substituents which are independently selected from: deuterium (D), halogen, -OH, mercapto, cyano, $-CD_3$, $C_1$-$C_6$ alkyl (preferably $C_1$-$C_3$ alkyl), $C_2$-$C_6$ alkenyl, $C_2$-$C_6$ alkynyl, cycloalkyl (preferably $C_3$-$C_8$ cycloalkyl), aryl, heterocyclyl (preferably 3-8 membered heterocyclyl), heteroaryl, aryl-$C_1$-$C_6$ alkyl-, heteroaryl-$C_1$-$C_6$ alkyl, $C_1$-$C_6$ haloalkyl, -$OC_1$-$C_6$ alkyl (preferably -$OC_1$-$C_3$ alkyl), -$OC_2$-$C_6$ alkenyl, $OC_1$-$C_6$ alkylphenyl, $C_1$-$C_6$ alkyl-OH (preferably $C_1$-$C_4$ alkyl-OH) , $C_1$-$C_6$ alkyl-SH, $C_1$-$C_6$ alkyl-O-$C_1$-$C_6$ alkyl, $OC_1$-$C_6$ haloalkyl, NH2, $C_1$-$C_6$ alkyl-$NH_2$ (preferably $C_1$-$C_3$ alkyl-$NH_2$), -N($C_1$-$C_6$ alkyl)$_2$ (preferably -N($C_1$-$C_3$ alkyl)$_2$), -NH($C_1$-$C_6$ alkyl) (preferably -NH($C_1$-$C_3$ alkyl)), -N($C_1$-$C_6$ alkyl)($C_1$-$C_6$ alkylphenyl), -NH($C_1$-$C_6$ alkylphenyl), nitro, -C(O)-OH, -C(O)$OC_1$-$C_6$ alkyl (preferably -C(O)$OC_1$-$C_3$ alkyl), -CONRiRii (wherein Ri and Rii are H, D and $C_1$-$C_6$ alkyl, preferably $C_1$-$C_3$ alkyl), -NHC(O)($C_1$-$C_6$ alkyl), -NHC(O)(phenyl), -N($C_1$-$C_6$ alkyl)C(O)($C_1$-$C_6$ alkyl), -N($C_1$-$C_6$ alkyl)C(O)(phenyl), -C(O)$C_1$-$C_6$ alkyl, -C(O)heteroaryl (preferably -C(O)-5-7 membered heteroaryl), -C(O)$C_1$-$C_6$ alkylphenyl, -C(O)$C_1$-$C_6$ haloalkyl, -OC(O)$C_1$-$C_6$ alkyl (preferably -OC(O)$C_1$-$C_3$ alkyl), -$S(O)_2$-$C_1$-$C_6$ alkyl, -S(O)-$C_1$-$C_6$ alkyl, -$S(O)_2$-phenyl, -$S(O)_2$-$C_1$-$C_6$ haloalkyl, -$S(O)_2NH_2$, -$S(O)_2NH$($C_1$-$C_6$ alkyl), -$S(O)_2NH$(phenyl),

-NHS(O)$_2$($C_1$-$C_6$ alkyl), -NHS(O)$_2$(phenyl) and -NHS(O)$_2$($C_1$-$C_6$ haloalkyl), wherein each of the alkyl, cycloalkyl, phenyl, aryl, heterocyclyl and heteroaryl groups is optionally further substituted with one or more substituents selected from the group consisting of halogen, -OH, -NH2, cycloalkyl, 3-8 membered heterocyclyl, $C_1$-$C_4$ alkyl, $C_1$-$C_4$ haloalkyl-, -O$C_1$-$C_4$ alkyl, -$C_1$-$C_4$ alkyl-OH, -$C_1$-$C_4$ alkyl-O-$C_1$-$C_4$ alkyl, -O$C_1$-$C_4$ haloalkyl, cyano, nitro, -C(O)-OH, -C(O)O$C_1$-$C_6$ alkyl, -CON($C_1$-$C_6$ alkyl)$_2$, -CONH($C_1$-$C_6$ alkyl), -CON$H_2$, -NHC(O)($C_1$-$C_6$ alkyl), -NH($C_1$-$C_6$ alkyl)C(O)($C_1$-$C_6$ alkyl), -$SO_2$($C_1$-$C_6$ alkyl), -$SO_2$(phenyl), -$SO_2$($C_1$-$C_6$ haloalkyl), -$SO_2NH_2$, -$SO_2$NH($C_1$-$C_6$ alkyl), -$SO_2$NH(phenyl), -NH$SO_2$($C_1$-$C_6$ alkyl), -NH$SO_2$(phenyl) and -NH$SO_2$($C_1$-$C_6$ haloalkyl). When an atom or group is substituted with multiple substituents, said multiple substituents may be the same or different.

As used herein, the term "hydrocarbyl" means the remaining group of an aliphatic hydrocarbon after losing one hydrogen atom, including linear or branched, saturated or unsaturated hydrocarbyl. The hydrocarbyl includes alkyl, alkenyl and alkynyl; preferably, the hydrocarbyl is $C_1$-$C_{10}$ hydrocarbyl, $C_1$-$C_6$ hydrocarbyl, or $C_1$-$C_3$ hydrocarbyl.

As used herein, the term "alkyl" refers to $C_1$-$C_{24}$ alkyl, $C_1$-$C_{20}$ alkyl, $C_1$-$C_{18}$ alkyl, $C_1$-$C_{12}$ alkyl, $C_1$-$C_6$ alkyl, $C_3$-$C_{24}$ alkyl, $C_3$-$C_{20}$ alkyl, $C_3$-$C_{18}$ alkyl, $C_3$-$C_{12}$ alkyl, $C_3$-$C_6$ alkyl, $C_6$-$C_{24}$ alkyl, $C_6$-$C_{20}$ alkyl, $C_6$-$C_{18}$ alkyl or $C_6$-$C_{12}$ alkyl.

As used herein, the term "alkenyl" refers to $C_2$-$C_{24}$ alkenyl, $C_2$-$C_{20}$ alkenyl, $C_2$-$C_{18}$ alkenyl, $C_2$-$C_{12}$ alkenyl, $C_2$-$C_6$ alkenyl, $C_3$-$C_{20}$ alkenyl, $C_3$-$C_{18}$ alkenyl, $C_3$-$C_{12}$ alkenyl, $C_3$-$C_6$ alkenyl, $C_6$-$C_{24}$ alkenyl, $C_6$-$C_{20}$ alkenyl, $C_6$-$C_{18}$ alkenyl or $C_6$-$C_{12}$ alkenyl. Alkenyl may contain one or more (for example, 2, 3 or 4) C=C double bonds.

As used herein, the term "alkynyl" refers to $C_2$-$C_{24}$ alkynyl, $C_2$-$C_{20}$ alkynyl, $C_2$-$C_{18}$ alkynyl, $C_2$-$C_{12}$ alkynyl, $C_2$-$C_6$ alkynyl, $C_3$-$C_{20}$ alkynyl, $C_3$-$C_{18}$ alkynyl, $C_3$-$C_{12}$ alkynyl, $C_3$-$C_6$ alkynyl, $C_6$-$C_{24}$ alkynyl, $C_6$-$C_{20}$ alkynyl, $C_6$-$C_{18}$ alkynyl or $C_6$-$C_{12}$ alkynyl.

As used herein, the term "acyl" means hydrocarbyl-carbonyl. Preferably, the acyl is $C_4$-$C_{24}$ acyl, $C_6$-$C_{18}$ acyl, $C_6$-$C_{12}$ acyl, $C_6$-$C_{10}$ acyl, $C_4$-$C_6$ acyl.

As used herein, the term "alkoxy" means alkyl-oxy. Preferably, the alkoxy is $C_1$-$C_{10}$ alkoxy; more preferably, the alkoxy is $C_1$-$C_6$ alkoxy; and most preferably, the alkoxy is $C_1$-$C_3$ alkoxy.

As used herein, the term "heterocycle" means a saturated or unsaturated cyclic group containing heteroatoms selected from N, O and S. Preferably, the heterocycle is an optionally substituted 4-10 membered heterocycle containing 1 to 6 heteroatoms selected from N, O and S, or an optionally substituted 4-6 membered saturated heterocycle containing 1, 2 or 3 heteroatoms selected from N, O and S. Examples of heterocycles include, but are not limited to, azetidine, oxetanyl, tetrahydrofuran, pyrrolidine, imidazolidine, pyrazolidine, tetrahydropyran, piperidine, morpholine, thiomorpholine, piperazine, preferably pyrrolidine, piperidine, piperazine and morpholine. The heterocycle may be optionally substituted with one or more substituents, and the types of the substituents are as defined for "optionally substituted" above to which reference is made.

In one aspect, the present invention provides an amino lipid compound which is a compound represented by the following Formula I:

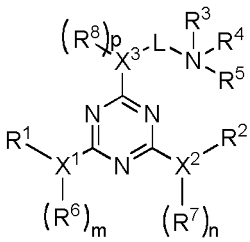

(I)

wherein:

$R^1$ and $R^2$ are the same or different from each other, and are each independently selected from the group consisting of $C_6$-$C_{24}$ alkyl, $C_6$-$C_{24}$ alkenyl, $C_6$-$C_{24}$ alkynyl and $C_4$-$C_{24}$ acyl, wherein the $C_6$-$C_{24}$ alkyl, the $C_6$-$C_{24}$ alkenyl, the $C_6$-$C_{24}$ alkynyl and the $C_4$-$C_{24}$ acyl are optionally substituted with $C_1$-$C_6$ hydrocarbyl;

preferably, $R^1$ and $R^2$ are the same or different from each other, and are each independently $C_6$-$C_{18}$ alkyl or $C_6$-$C_{18}$ alkenyl.

$X^1$, $X^2$ and $X^3$ are the same or different from one another, and are each independently selected from the group consisting of C, N, O, S, S=O, S(=O)$_2$ and S-S;

when $X^1$ is C, m=2, and two $R^6$ are the same or different from each other; when $X^1$ is N, m=1; when $X^1$ is O, S, S=O, S(=O)$_2$ or S-S, m=0;

when $X^2$ is C, n=2, and two $R^7$ are the same or different from each other; when $X^2$ is N, n=1; when $X^2$ is O, S, S=O, S(=O)$_2$ or S-S, n=0;

when $X^3$ is C, p=2, and two $R^8$ are the same or different from each other; when $X^3$ is N, p=1;when $X^3$ is O, S, S=O, S(=O)$_2$ or S-S, p=0;

$R^3$ and $R^4$ are the same or different from each other, and are each independently selected from the group consisting of $C_1$-$C_{12}$ alkyl, $C_2$-$C_{12}$ alkenyl and $C_2$-$C_{12}$ alkynyl, wherein the $C_1$-$C_{12}$ alkyl, the $C_2$-$C_{12}$ alkenyl and the $C_2$-$C_{12}$ alkynyl are optionally substituted with $C_1$-$C_6$ hydrocarbyl, or $R^3$ and $R^4$ bind to each other to form an optionally substituted 4-10 membered heterocycle containing 1 to 6 heteroatoms selected from nitrogen, sulfur, and oxygen;
$R^5$ is absent, or $R^5$ is hydrogen or $C_1$-$C_{12}$ alkyl to provide a quaternary amine;
$R^6$, $R^7$, $R^8$ are hydrogen or $C_1$-$C_{12}$ alkyl;
L is $C_1$-$C_{12}$ alkylene, $C_2$-$C_{12}$ alkenylene or $C_2$-$C_{12}$ alkynylene, wherein the $C_1$-$C_{12}$ alkylene, the $C_2$-$C_{12}$ alkenylene and the $C_2$-$C_{12}$ alkenylene are optionally substituted with one or more substituents selected from the group consisting of hydrocarbyl, carboxyl, acyl and alkoxy, or L is an optionally substituted 4-10 membered heterocycle containing heteroatoms selected from nitrogen, sulfur, and oxygen.

Preferably, L is $C_1$-$C_8$ alkylene, $C_2$-$C_6$ alkylene, $C_2$-$C_4$ alkylene or $C_3$-$C_4$ alkylene, wherein the $C_1$-$C_8$ alkylene, the $C_2$-$C_6$ alkylene, the $C_2$-$C_4$ alkylene or the $C_3$-$C_4$ alkylene is optionally substituted with $C_1$-$C_6$ hydrocarbyl.

In some embodiments, the present invention provides the amino lipid compound of Formula I, wherein $X^1$ is C, $R^6$ is H, and m=2; or $X^1$ is N, $R^6$ is H and m=1; or $X^1$ is O, S, S=O, $S(=O)_2$ or S-S, and m=0; alternatively or further, $X^2$ is C, $R^7$ is H, and n=2; or $X^2$ is N, $R^7$ is H, and n=1; or $X^2$ is O, S, S=O, $S(=O)_2$ or S-S, n=0; alternatively or further, $X^3$ is C, $R^8$ is H, and p=2; or $X^3$ is N, $R^8$ is H, and p=1; or $X^3$ is O, S, S=O, $S(=O)_2$ or S-S, and p=0.

In some other embodiments, the present invention provides the amino lipid compound of Formula I, wherein $X^1$ and $X^2$ are the same or different from each other, and are each independently N or S; when $X^1$ is N, m=1; when $X^1$ is S, m=0; when $X^2$ is N, n=1; when $X^2$ is S, n=0; alternatively or further, $X^3$ is N and p=1, or $X^3$ is O and p=0; alternatively or further, $R^5$ is absent; alternatively or further, L is $C_1$-$C_{12}$ alkylene which is optionally substituted with $C_1$-$C_6$ hydrocarbyl, for example, L is $(CH_2)_q$, wherein q is an integer of from 1 to 12, for example, an integer of from 1 to 8 or from 1 to 6. In some of such embodiments, when m=1, $R^6$ is hydrogen; alternatively or further, when n=1, $R^7$ is hydrogen; alternatively or further, $R^8$ is hydrogen. In other such embodiments, the present invention provides the amino lipid compound of Formula I, wherein: $R^1$ and $R^2$ are the same or different from each other, and are each independently $C_6$-$C_{24}$ alkyl or $C_6$-$C_{24}$ alkenyl (for example, $C_{12}$-$C_{18}$ alkenyl); $X^1$ is N, $R^6$ is H, and m=1; or $X^1$ is S, and m=0; $X^2$ is N, $R^7$ is H, and n=1; or $X^2$ is S, and n=0; $X^3$ is N, $R^8$ is H, and p=1; $R^3$ and $R^4$ are the same or different from each other, and are each independently $C_1$-$C_{12}$ alkyl, wherein the $C_1$-$C_{12}$ alkyl is optionally substituted with $C_1$-$C_6$ hydrocarbyl, or $R^3$ and $R^4$ bind to each other to form an optionally substituted 4-10 membered heterocycle containing 1 to 6 heteroatoms selected from nitrogen, sulfur, and oxygen; and $R^5$ is absent.

In some embodiments, the present invention provides the amino lipid compound of Formula I described above, wherein L is $C_1$-$C_4$ alkylene, wherein the $C_1$-$C_4$ alkylene is optionally substituted with $C_1$-$C_6$ hydrocarbyl. For example, L is $C_2$-$C_4$ alkylene, such as $(CH_2)_q$, wherein q is 1, 2, 3 or 4.

In some embodiments, the present invention provides the amino lipid compound of Formula I described above, wherein $R^1$ and $R^2$ are the same or different from each other, and are each independently $C_6$-$C_{18}$ alkyl or $C_6$-$C_{18}$ alkenyl. In some embodiments, $R^1$ and $R^2$ are the same or different from each other, and are each independently $C_6$-$C_{18}$ alkyl. In some embodiments, one of $R^1$ and $R^2$ is $C_6$-$C_{18}$ alkyl and the other is $C_6$-$C_{18}$ alkenyl.

In some embodiments, the present invention provides the amino lipid compound of Formula I described above, wherein:
m=0 or 1, n=0 or 1, and

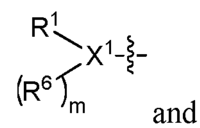
and
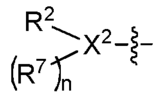

are the same or different from each other, and are each independently one selected from S6, S7, S8, S9, S10, S11, S12, S14, S15, S16, S18, S19, S20, N6, N7, N8, N9, N11, N12, N13, N15, N16, N18 and $^{2}N_{12}$:

| | | |
|---|---|---|
| S6: $CH_3(CH_2)_5S$-; | S7: $CH_3(CH_2)_6S$-; | S8: $CH_3(CH_2)_7S$-; |
| S9: $CH_3(CH_2)_8S$-; | S10: $CH_3(CH_2)_9S$-; | S11: $CH_3(CH_2)_{10}S$-; |
| S12: $CH_3(CH_2)_{11}S$-; | S14: $CH_3(CH_2)_{13}S$-; | S15: $CH_3(CH_2)_{14}S$-; |
| S16: $CH_3(CH_2)_{15}S$-; | S18: $CH_3(CH_2)_{17}S$-; | |

S19: 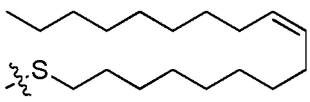 ; S20: 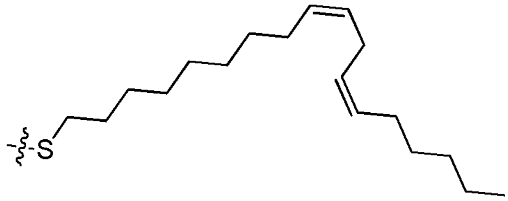 ;

N6: $CH_3(CH_2)_5NH$-; N7: $CH_3(CH_2)_6NH$-; N8: $CH_3(CH_2)_7NH$-;
N9: $CH_3(CH_2)_8NH$-; N11: $CH_3(CH_2)_{10}NH$-; N12: $CH_3(CH_2)_{11}NH$-;
N13: $CH_3(CH_2)_{12}NH$-; N15: $CH_3(CH_2)_{14}NH$-; N16: $CH_3(CH_2)_{15}NH$-;
N18: $CH_3(CH_2)_{17}NH$-; $^2N_{12}$: $(CH_3(CH_2)_{11})_2N$-.

In some embodiments, the present invention provides the amino lipid compound of Formula I described above, wherein:
p=1, and

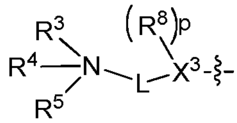

is one selected from D1, D2, D3, D4, D5, D6, D7, D8, D9 and D10 below:

D1: 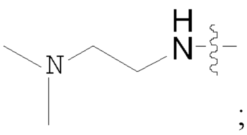 ; D2: 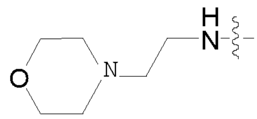 ; D3: 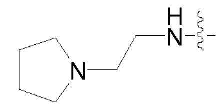 ;

D4: 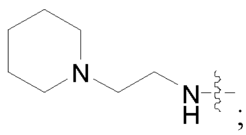 ; D5: 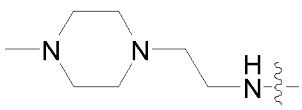 ; D6: 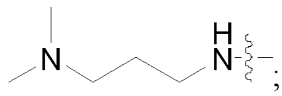 ;

D7: 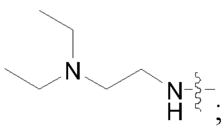 ; D8: 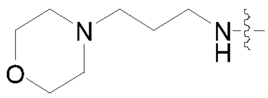 ; D9: 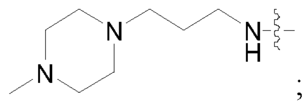 ;

D10: 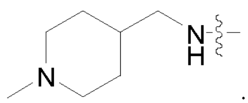 .

In some embodiments, the present invention provides the amino lipid compound of Formula I described above, wherein:

$X^3$ is O;
p is 0;
$R^5$ is absent; and

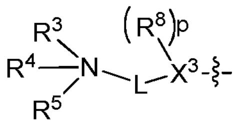

is one selected from O1, O2, O3, O4, O5, O6, O7, O8, O9 and O10 below:

O1: 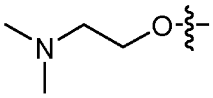 ; O2: 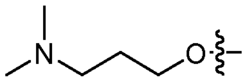 ; O3: 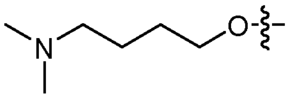 ;

O4: 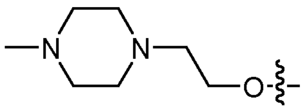 ; O5: 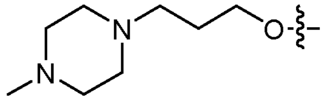 ; O6: 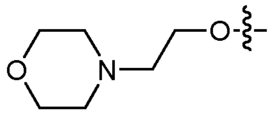 ;

O7: 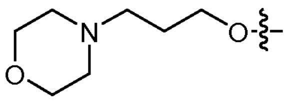 ; O8: 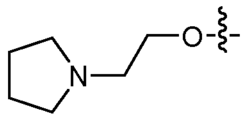 ; O9: 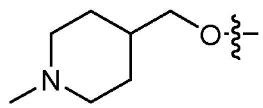 ;

O10: 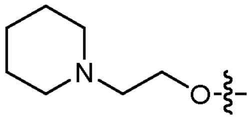 .

In some other embodiments, the present invention provides the amino lipid compound of Formula I described above, wherein the amino lipid compound is a compound represented by the following Formula I':

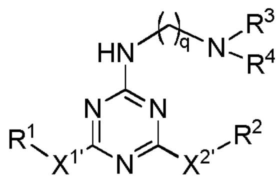 ( I' )

wherein:

$X^{1'}$ and $X^{2'}$ are the same or different from each other, and are each independently NH, O or S, preferably NH or S; the definitions of $R^1$, $R^2$, $R^3$, $R^4$ and q are the same as the definitions thereof in the above description of the amino lipid compound of Formula I.

In some other embodiments, the present invention provides the amino lipid compound of Formula I described above, wherein the amino lipid compound is a compound represented by the following Formula I":

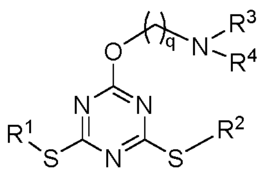 ( I" )

wherein:
the definitions of $R^1$, $R^2$, $R^3$, $R^4$ and q are the same as the definitions thereof in the above description of the amino lipid

compound of Formula I.

In another aspect, the present invention provides a method for preparing the amino lipid compound of Formula I described above, comprising the steps of:

(1) performing a first reaction between cyanuric chloride and a compound represented by $R^1(R^6)_m$-$X^1H$ at a temperature of -40°C to 30°C in the presence of a base as an acid-binding agent to obtain a first intermediate of Formula I-1;

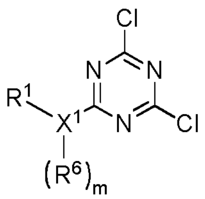

( **I-1** )

(2) with or (preferably) without separating the first intermediate, performing a second reaction between the first intermediate and a compound represented by $R^2(R^7)_n$-$X^2H$ at room temperature or under heating condition in the presence of a base as an acid-binding agent to obtain a second intermediate of Formula 1-2;

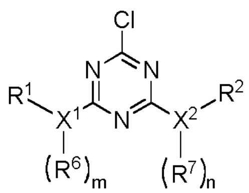

( **I-2** )

(3) with or (preferably) without separating the second intermediate, performing a third reaction between the second intermediate and a diamine represented by $HX^3(R^8)_p$-L-$NR^3R^4R^5$ under heating condition to obtain the amino lipid compound of Formula I;

wherein the definitions of $X^1$, $X^2$, $X^3$, $R^1$, $R^2$, $R^3$, $R^4$, $R^5$, $R^6$, $R^7$, $R^8$, L, m, n and p are the same as the definitions thereof in the above description of the amino lipid compound of Formula I.

For example, in some embodiments, $X^1$ and $X^2$ are the same or different from each other, and are each independently N or S. When $X^1$ is N, m = 1; when $X^1$ is S, m=0. When $X^2$ is N, n = 1; when $X^2$ is S, n=0. In some embodiments, $X^3$ is N, and p=1. In some embodiments, $R^5$ is absent. In some embodiments, L is $C_1$-$C_{12}$ alkylene which is optionally substituted with $C_1$-$C_6$ hydrocarbyl, for example, L is $(CH_2)_q$, wherein q is an integer of from 1 to 12, such as an integer of from 1 to 6. In some embodiments, when m=1, $R^6$ is hydrogen. In some embodiments, when n=1, $R^7$ is hydrogen. In some embodiments, $R^8$ is hydrogen.

Preferably, in step (3), the third reaction is carried out in the presence of a base as an acid-binding agent.

In some embodiments, the present invention provides a method for preparing the amino lipid compound of Formula I', comprising the steps of:

(1) performing a first reaction between cyanuric chloride and a compound represented by $R^1$-$X^{1'}H$ at a temperature of -40°C to 30 °C in the presence of a base as an acid-binding agent to obtain a first intermediate of Formula I'-1;

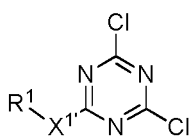

( **I'-1** )

(2) with or (preferably) without separating the first intermediate, performing a second reaction between the first intermediate and a compound represented by $R^2$-$X^{2'}H$ at room temperature or under heating condition in the presence of a base as an acid-binding agent to obtain a second intermediate of Formula I'-2;

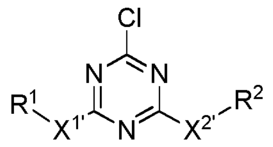

( **I'-2** )

(3) with or (preferably) without separating the second intermediate, performing a third reaction between the second intermediate and a diamine represented by $H_2N\text{-}(CH_2)_q\text{-}NR^3R^4$ under heating condition to obtain the amino lipid compound of Formula I';

wherein the definitions of $X^{1'}$, $X^{2'}$, $R^1$, $R^2$, $R^3$, $R^4$ and q are the same as the definitions thereof in the above description of the amino lipid compound of Formula I'.

Preferably, in step (3), the third reaction is carried out in the presence of a base as an acid-binding agent.

In some embodiments, the present invention provides a method for preparing the amino lipid compound of Formula I" described above,

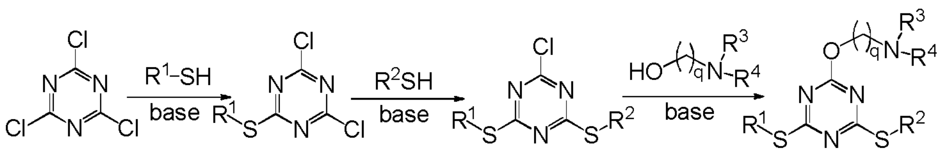


( **I"-1** ) ( **I"-2** ) ( **I"** )

comprising the steps of:

(1) performing a first reaction between cyanuric chloride with a compound represented by $R^1$-SH at a temperature of -40°C to 30°C in the presence of a base as an acid-binding agent to obtain a first intermediate of Formula I"-1;
(2) with or (preferably) without separating the first intermediate, performing a second reaction between the first intermediate and a compound represented by $R^2$-SH at room temperature or under heating condition in the presence of a base as an acid-binding agent to obtain a second intermediate of Formula I"-2;

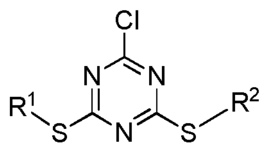

( **I"-2** )

(3) with or (preferably) without separating the second intermediate, performing a third reaction between the second intermediate and a hydroxylamine represented by $HO\text{-}(CH_2)_q\text{-}NR^3R^4$ at room temperature or under heating condition in the presence of a base as an acid-binding agent to obtain the amino lipid compound of Formula I";

wherein the definitions of $R^1$, $R^2$, $R^3$, $R^4$ and q are the same as the definitions thereof in the above description of the amino lipid compound of Formula I".

In preferred embodiments of any of the methods described above, the first reaction is carried out at a temperature less than 20°C (for example, less than 15°C, less than 10°C, less than 5°C, less than 0°C, less than -10°C, or less than -20°C, without particular limitation to the lower limit of the reaction temperature) and greater than -40°C (for example, greater than -35°C, or preferably equal to or greater than -30°C).

As used herein, the term "room temperature" refers to the normal temperature at atmospheric pressure, and may refer to a temperature ranging from 0°C to 30°C, from 0°C to 25°C, from 0°C to 20°C, from 5°C to 30°C, from 5°C to 20°C, from 10°C to 30°C, from 10°C to 25°C, from 15°C to 30°C, or from 15°C to 25°C.

As used herein, the term "heating condition" specifically refers to heating to a temperature ranging from 50°C to 120°C, from 50°C to 110°C, from 50°C to 80°C, from 60°C to 120°C, from 60°C to 110°C, from 60°C to 100°C, from 70°C to 120°C, from 70°C to 100°C, or from 70°C to 90°C.

As used herein, the term "base" can particularly be a base compound commonly used in the art, such as but not limited to organic bases, such as triethylamine, DIPEA, pyridine, DMAP; or inorganic bases, such as sodium hydroxide, potassium hydroxide, sodium carbonate, potassium carbonate.

Preferably, q is an integer of from 1 to 8, preferably an integer of from 1 to 6; more preferably, q is an integer

of from 1 to 4, such as 1, 2, 3 or 4.

In some embodiments, the present invention provides a method for preparing an amino lipid compound of Formula II which can be carried out according to the following Equation 1:

[Equation 1]

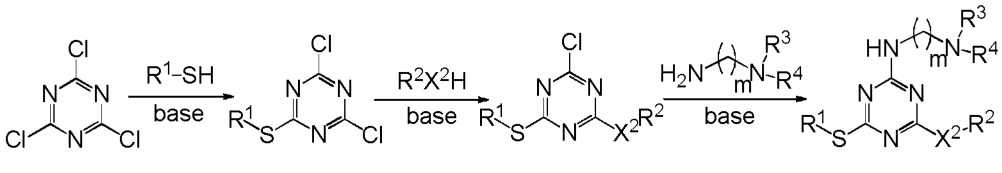


( **II** )

wherein the definitions of $R^1$, $R^2$, $R^3$, $R^4$ and $X^2$ are the same as the definitions thereof in the above description of the method for preparing the amino lipid compound of Formula I.

In some embodiments, the present invention provides a method for preparing an amino lipid compound of Formula III which can be carried out according to the following Equation 2:

[Equation 2]

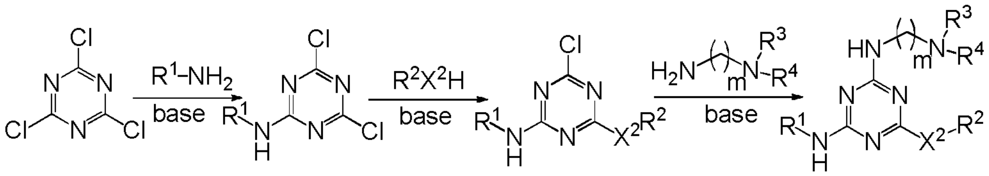


( **III** )

wherein the definitions of $R^1$, $R^2$, $R^3$, $R^4$ and $X^2$ are the same as the definitions thereof in the above description of the method for preparing the amino lipid compound of Formula I.

In some embodiments, the present invention provides a method for preparing an amino lipid compound of Formula IV which can be carried out according to the following Equation 3:

[Equation 3]

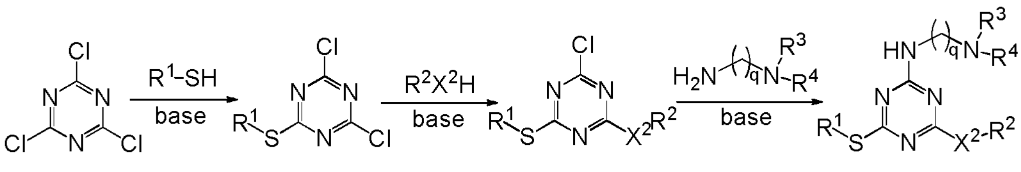


( **IV** )

wherein the definitions of $R^1$, $R^2$, $R^3$, $R^4$, $X^2$ and q are the same as the definitions thereof in the above description of the amino lipid compound of Formula I;

preferably, $X^2$ is selected from the group consisting of O, S, S=O, $S(=O)_2$ and S-S, preferably O or S, and more preferably S.

In some embodiments, the present invention provides a method for preparing an amino lipid compound of Formula V which can be carried out according to the following Equation 4:

[Equation 4]

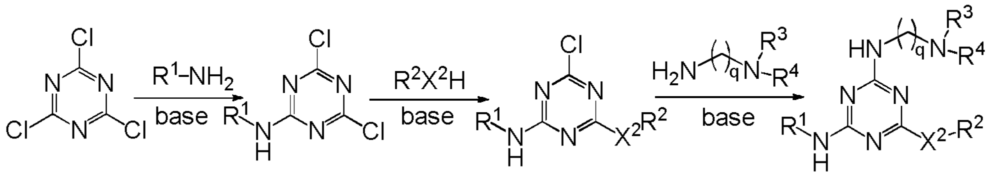


( V )

wherein the definitions of $R^1$, $R^2$, $R^3$, $R^4$, $X^2$ and q are the same as the definitions thereof in the above description of the amino lipid compound of Formula I;

preferably, $X^2$ is selected from the group consisting of O, S, S=O, $S(=O)_2$ and S-S, preferably O or S, and more preferably S.

It can be seen that the method for preparing the amino lipid compound according to the present invention is very universal, and can be used for rapid synthesis of amino lipid compound library and for rapid cell-based screening experiments at a low cost.

In another aspect, the present invention provides a use of the amino lipid compound according to any one of the formulae I, I', II, III, IV and V for the preparation of lipid particles.

In another aspect, the present invention provides lipid particles comprising the amino lipid compound according to the present invention as described above.

Due to the long nonpolar residues contained in the amino lipid compound of the present invention, all the obtained compounds have hydrophobic characteristic, and simultaneously have hydrophilic characteristic due to the amine group. This amphoteric characteristic can be useful for forming lipid particles, such as lipid nanoparticles, lipid bilayers, micelles, liposomes, etc.

Within the scope of the present invention, the term "lipid particle" means nano-sized substances prepared by placing amino lipid compounds in an aqueous solution. These particles are particularly lipid nanoparticles, bilayered lipid vesicles (liposomes), multilayered vesicles or micelles.

In a preferred embodiment of the present invention, the lipid particles are liposomes containing the amino lipid compound according to the present invention as described above. Within the scope of the present invention, liposomes are micro vesicles consisting of bilayers of lipid amphiphilic molecules surrounding aqueous compartments.

The formation of liposome is not a spontaneous process. lipid vesicles are formed firstly when lipids are added to water, thus forming a bilayer or a series of bilayers, each being separated by water molecules. Liposomes can be formed by ultrasonic treatment of lipid vesicles in water.

Within the scope of the present invention, the term "lipid bilayer" means a thin film formed by two layers of lipid molecules. The term "micelle" means an aggregate of surfactant molecules dispersed in a liquid colloid. Typical micelles in an aqueous solution form aggregates with the hydrophilic head regions upon contact with water, and chelate the hydrophobic single tail regions at the center of the micelles.

Within the scope of the present invention, the term "cell" means a general term, and includes the culture of individual cells, tissues, organs, insect cells, avian cells, fish cells, amphibian cells, mammalian cells, primary cells, continuous cell lines, stem cells and/or genetically engineered cells (such as recombinant cells expressing heterologous polypeptides or proteins). Recombinant cells include, for example, cells expressing heterologous polypeptides or proteins such as growth factors or blood factors.

In some preferred embodiments, the lipid particles or liposomes further contain one or more of a helper lipid, a sterol and a bioactive agent.

For example, in some embodiments, the lipid particles or liposomes contain a helper lipid. In preferred embodiments, the helper lipid is a non-cationic lipid. In more preferred embodiments, the helper lipid is a non-cationic phospholipid. Within the scope of the present invention, a non-cationic lipid can contain a cationic functional group (for example, ammonium), but should contain anionic functional group to at least neutralize the molecule. The total of all functional groups in the lipid molecule should be non-cationic. Liposomes consisting of the mixture of the amino lipid according to the present invention and a non-cationic (neutral) phospholipid are most effective for delivering nucleic acids into cells. In even more preferred embodiments, the non-cationic lipid is DOPE, DSPC or a combination thereof. In some preferred embodiments, the molar ratio of the amino lipid compound to the helper lipid in the lipid particles is about (2 to 10):1, preferably about (3 to 8):1, more preferably about (4 to 6): 1, such as about 4:1, about 4.5:1 or about 5:1.

In some alternative or further embodiments, the lipid particles or liposomes comprise a sterol. Sterols, such as cholesterol, are natural components in cell membranes. It can be useful for stabilizing the particles and helping integration with cell membranes. The sterol may be one or more selected from the group consisting of cholesterol, sitosterol,

stigmasterol and ergosterol, preferably cholesterol. In some preferred embodiments, the molar ratio of the amino lipid compound to the sterol in the lipid particles is about (1 to 1.5): 1, preferably about (1 to 1.4): 1, such as about (1 to 1.3): 1.

In some alternative or further embodiments, the lipid particles or liposomes contain a bioactive agent. Within the scope of the present invention, bioactive agents are substances that have biological effects when introduced into cells or hosts, for example, by stimulating immune responses or inflammatory responses, by exerting enzyme activity or by complementary mutations, etc. The bioactive agent is particularly a nucleic acid, a peptide, a protein, an antibody and a small molecule. The term "lipid particle drug" can be used when liposomes are used to wrap a drug into lipid bilayers or into the inner aqueous space in the liposomes.

In the most preferred embodiments, the bioactive agent is a nucleic acid, including but not limited to messenger RNA (mRNA), antisense oligonucleotide, DNA, plasmid, ribosomal RNA (rRNA), microRNA (miRNA), transfer RNA (tRNA), small interfering RNA (siRNA) and small nuclear RNA (snRNA). In some other preferred embodiments, the bioactive agent is selected from the group consisting of an antitumor agent, an antibiotic, an immunomodulator, an anti-inflammatory agent, an agent acting on the central nervous system, an antigen or a fragment thereof, a protein, a peptide, a polypeptide, a polypeptoid, a vaccine and a small molecule, and mixtures thereof.

In some other embodiments, the lipid particles or liposomes further contain at least one polyethylene glycol (PEG)-lipid. PEG lipids contribute to protect particles and their contents from degradation *in vitro* or *in vivo*. In addition, PEG forms a protective layer on the liposome surface, and increases the circulation time *in vivo*. It can be used in the delivery of liposome drugs (PEG-liposome). Preferably, the PEG-lipid can be one or more selected from PEG1000-DMG, PEG5000-DMG, PEG2000-DMG and PEG2000-DSPE, preferably PEG2000-DMG In some preferred embodiments, the molar ratio of the amino lipid compound to the PEG-lipid in the lipid particles is about (9 to 42): 1, preferably about (12 to 38):1, more preferably about (16 to 36):1, such as about (18 to 34):1.

The lipid particles or liposomes according to the present invention have excellent performances in encapsulating bioactive agents. The lipid particles or liposomes containing a bioactive agent can be used to deliver any of a variety of therapeutic agents into a cell. The present invention includes the use of the lipid particles (particularly, liposomes) as described above for delivering a bioactive agent into a cell. The present invention also provides a method for delivering a bioactive agent into a cell, comprising contacting lipid particles or liposomes containing the bioactive agent according to the present invention with the cell.

As shown above, the lipid particles or liposomes containing the amino lipid compound of the present invention is suitable for delivering a bioactive agent into a cell. Specific characteristics of liposomes imparted by various amino lipid compounds synthesized by the general synthesis method can be screened. The important characteristics are, e.g., transfection efficiency, cytotoxicity, adhesion of agents to be delivered into cells, stability of liposomes, size of liposomes, etc. The method of the invention can be used to form specific adaptive liposomes for specific applications.

For example, lipid particles or liposomes can be used to transfect multicellular tissues or organs. Therefore, the present invention also provides a method for transfecting a cell, a multicellular tissue or organ, comprising contacting lipid particles or liposomes containing the nucleic acid according to the present invention with the cell. This provides patients with the possibility for new therapeutic treatment.

According to the present invention, patients can be any mammal, preferably selected from mice, rats, pigs, cats, dogs, horses, goats, cows and monkeys and/or others. In some preferred embodiments, the patient is human.

In another aspect, the present invention still provides a use of lipid particles or liposomes comprising any one of the amino lipid compounds of formulae I, I', I", II, III, IV and V as a medicament. In another aspect, the present invention still provides a use of the lipid particles or liposomes comprising any one of the amino lipid compounds of formulae I, I', I", II, III, IV and V in the preparation of a medicament. For example, in some embodiments, the lipid particles are used as vectors for encapsulating a bioactive agent.

Particularly, the lipid particles or liposomes, or the medicament can be administered to patients for gene therapy, gene vaccination, antisense therapy or a therapy by RNA interference. Specific applications include but are not limited to:

(1) The lipid particles of the present invention can deliver nucleic acids for gene therapy. Exogenous genes are introduced into target cells through the amino lipid of the present invention to correct or remedy diseases caused by defective and abnormal genes, so as to achieve the therapeutic purpose. Among them, also included is the application of such technologies as transgene, that is, exogenous genes are inserted into appropriate recipient cells of a patient by gene transfer technology, so that the products produced by the exogenous genes can treat a certain disease, such as common lung cancer, gastric cancer, liver cancer, esophageal cancer, colon cancer, pancreatic cancer, brain cancer, lymphatic cancer, hematologic cancer, prostate cancer, etc. Gene-edited nucleic acid substances can also be introduced for the treatment of various genetic diseases, such as hemophilia, thalassemia, Gaucher disease, etc.

(2) The lipid particles of the present invention can be used in vaccination. The lipid particles or liposomes of the present invention can be used for delivering antigens or nucleic acids encoding antigens. The lipid particles of the present invention can also be used for the initiation of immune responses against various antigens which are used

for treating and/or preventing various disorders such as cancer, allergy, toxicity and pathogens (for example, viruses, bacteria, fungi and other pathogenic organisms) infection.

The present invention also provides a method for gene therapy, gene vaccination, antisense therapy or a therapy by RNA interference, comprising administering to a patient in need thereof lipid particles or liposomes of the present invention containing a bioactive agent (for example, a desired nucleic acid or antigen).

The lipid particles of the present invention can be used for preparing a medicament for nucleic acid transfer. Preferably, the nucleic acid is RNA, messenger RNA (mRNA), antisense oligonucleotide, DNA, plasmid, ribosomal RNA (rRNA), microRNA (Mirna), transfer RNA (tRNA), small interfering RNA (siRNA) and small nuclear RNA (snRNA).

Encapsulation of non-bioactive agents is also one purpose of the lipid particles or liposomes of the present invention. Therefore, the present invention also provides such lipid particles or liposomes comprising a non-bioactive agent. Examples of non-bioactive agent include, but are not limited to, antioxidants, pigments, dyes, perfumes and flavoring agents, such as those used in cosmetics. It is expected that the lipid particles or liposomes according to the present invention also have excellent performances in encapsulating non-bioactive agents.

The present invention also includes the following embodiments:

1. An amino lipid compound which is a compound represented by the following formula I:

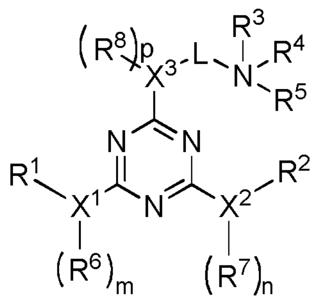

(I)

wherein $R^1$ and $R^2$ are the same or different from each other, and are each independently selected from the group consisting of $C_6$-$C_{24}$ alkyl, $C_6$-$C_{24}$ alkenyl, $C_6$-$C_{24}$ alkynyl and $C_4$-$C_{24}$ acyl, wherein the $C_6$-$C_{24}$ alkyl, the $C_6$-$C_{24}$ alkenyl, the $C_6$-$C_{24}$ alkynyl and the $C_4$-$C_{24}$ acyl are optionally substituted with $C_1$-$C_6$ hydrocarbyl;

$X^1$, $X^2$ and $X^3$ are the same or different from one another, and are each independently selected from the group consisting of C, N, O, S, S=O, $S(=O)_2$ and S-S;
when $X^1$ is C, m=2, and two $R^6$ are the same or different from each other; when $X^1$ is N, m=1; when $X^1$ is O, S, S=O, $S(=O)_2$ or S-S, m=0;
when $X^2$ is C, n=2, and two $R^7$ are the same or different from each other; when $X^2$ is N, n=1; when $X^2$ is O, S, S=O, $S(=O)_2$ or S-S, n=0;
when $X^3$ is C, p=2, and two $R^8$ are the same or different from each other; when $X^3$ is N, p=1; when $X^3$ is O, S, S=O, $S(=O)_2$ or S-S, p=0;
$R^3$ and $R^4$ are the same or different from each other, and are each independently selected from the group consisting of $C_1$-$C_{12}$ alkyl, $C_2$-$C_{12}$ alkenyl and $C_2$-$C_{12}$ alkynyl, wherein the $C_1$-$C_{12}$ alkyl, the $C_2$-$C_{12}$ alkenyl and the $C_2$-$C_{12}$ alkynyl are optionally substituted with $C_1$-$C_6$ hydrocarbyl, or $R^3$ and $R^4$ bind to each other to form an optionally substituted 4-10 membered heterocycle containing 1 to 6 heteroatoms selected from nitrogen, sulfur, and oxygen;
$R^5$ is absent, or $R^5$ is hydrogen or $C_1$-$C_{12}$ alkyl to provide a quaternary amine;
$R^6$, $R^7$, $R^8$ are hydrogen or $C_1$-$C_{12}$ alkyl;
L is $C_1$-$C_{12}$ alkylene, $C_2$-$C_{12}$ alkenylene or $C_2$-$C_{12}$ alkynylene, wherein the $C_1$-$C_{12}$ alkylene, the $C_2$-$C_{12}$ alkenylene and the $C_2$-$C_{12}$ alkynylene are optionally substituted with one or more substituents selected from the group consisting of hydrocarbyl, carboxyl, acyl and alkoxy, or L is an optionally substituted 4-10 membered heterocycle containing heteroatoms selected from nitrogen, sulfur, and oxygen.

2. The amino lipid compound according to item 1, wherein:

$X^1$ is C, $R^6$ is H, and m=2; or $X^1$ is N, $R^6$ is H, and m=1; or $X^1$ is O, S, S=O, $S(=O)_2$ or S-S, and m=0;
$X^2$ is C, $R^7$ is H, and n=2; or $X^2$ is N, $R^7$ is H, and n=1; or $X^2$ is O, S, S=O, $S(=O)_2$ or S-S, and n=0;
$X^3$ is C, $R^8$ is H, and p=2; or $X^3$ is N, $R^8$ is H, and p=1; or $X^3$ is O, S, S=O, $S(=O)_2$ or S-S, and p=0.

3. The amino lipid compound according to item 1, wherein:

$R^1$ and $R^2$ are the same or different from each other, and are each independently $C_6$-$C_{24}$ alkyl;
$X^1$ is N, $R^6$ is H, and m=1; or $X^1$ is S, and m=0;
$X^2$ is N, $R^7$ is H, and n=1; or $X^2$ is S, and n=0;
$X^3$ is N, $R^8$ is H, and p=1;
$R^3$ and $R^4$ are the same or different from each other, and are each independently $C_1$-$C_{12}$ alkyl, wherein the $C_1$-$C_{12}$ alkyl is optionally substituted with $C_1$-$C_6$ hydrocarbyl, or $R^3$ and $R^4$ bind to each other to form an optionally substituted 4-10 membered heterocycle containing 1 to 6 heteroatoms selected from nitrogen, sulfur, and oxygen;
$R^5$ is absent.

4. The amino lipid compound according to any one of items 1 to 3, wherein L is $C_1$-$C_4$ alkylene, wherein the $C_1$-$C_4$ alkylene is optionally substituted with $C_1$-$C_6$ hydrocarbyl.
5. The amino lipid compound according to any one of items 1 to 3, wherein $R^1$ and $R^2$ are the same or different from each other, and are each independently $C_6$-$C_{18}$ alkyl.
6. The amino lipid compound according to item 1, wherein:

m=0, n=0, p=0,
$R^1$-$X^1$- and $R^2$-$X^2$- are the same or different from each other, and are each independently one selected from S6, S7, S8, S9, S10, S11, S12, S14, S15, S16, S18, N6, N7, N8, N9, N11, N12, N13, N15, N16, N18, $^2N_{12}$ described above;
-$X^3$-L-N($R^3$)($R^4$)($R^5$) is one selected from D1, D2, D3, D4, D5, D6, D7, D8, D9, D10 described above.

7. A method for preparing the amino lipid compound according to any one of items 1-6, comprising the steps of:

(1) performing a first reaction between cyanuric chloride and a compound represented by $R^1$-$X^1$H at a temperature of -30°C to 30°C in the presence of a base as an acid-binding agent to obtain a first intermediate, wherein $X^1$ is NH or S;
(2) performing a second reaction by adding a compound represented by $R^2$-$X^2$H without separating the first intermediate, at room temperature or under heating condition in the presence of a base as an acid-binding agent to obtain a second intermediate, wherein $X^2$ is NH or S;
(3) performing a third reaction by adding a diamine represented by $H_2N$-$(CH_2)_q$-$NR^3R^4$ without separating the second intermediate, under heating condition to obtain the amino lipid compound;

wherein q is an integer of from 1 to 12, and the definitions of $R^1$, $R^2$, $R^3$ and $R^4$ are the same as the definitions thereof in any one of items 1 to 6.
8. A method for preparing the amino lipid compound according to the present invention, which can be carried out according to the following Equation 1:

[Equation 1]

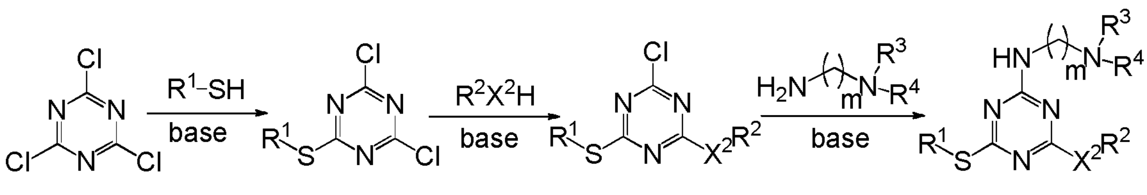


wherein the definitions of $R^1$, $R^2$, $R^3$, $R^4$, $X^2$ are the same as the definitions thereof in the above description of the method for preparing the amino lipid compound.
9. A method for preparing the amino lipid compound according to the present invention, which can be carried out according to the following Equation 2:

[Equation 2]

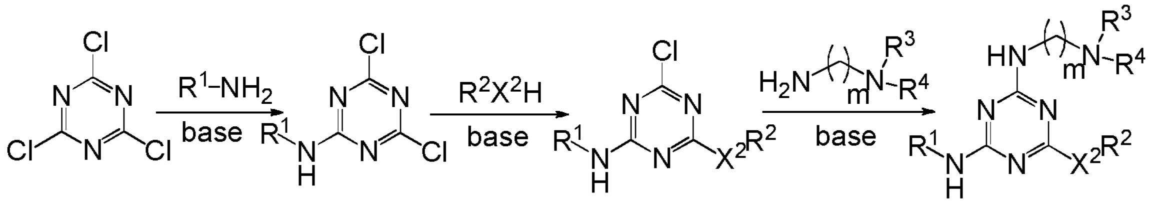


wherein the definitions of $R^1$, $R^2$, $R^3$, $R^4$, $X^2$ are the same as the definitions thereof in the above description of the method for preparing the amino lipid compound.

10. Use of the amino lipid compound according to any one of items 1 to 6 for preparing lipid particles, wherein the lipid particles are lipid nanoparticles, liposomes, multilayered vesicles or micelles; more preferably, the lipid particles are lipid nanoparticles.

11. Lipid particles comprising the amino lipid compound according to any one of items 1 to 6; preferably, the lipid particles are lipid nanoparticles, liposomes, multilayered vesicles or micelles; more preferably, the lipid particles are lipid nanoparticles.

12. The lipid particles according to item 11, further containing one or more of a helper lipid, a sterol and a bioactive agent;

preferably, the helper lipid is a non-cationic lipid; more preferably, the helper lipid is a non-cationic phospholipid; and even more preferably, the non-cationic lipid is DOPE;
preferably, the sterol is cholesterol;
preferably, the bioactive agent is one or more of a nucleic acid, an antitumor agent, an antibiotic, an immunomodulator, an anti-inflammatory agent, an agent acting on the central nervous system, an antigen or fragments thereof, a peptide, a protein, an antibody, a vaccine and a small molecule; more preferably, the nucleic acid is RNA, messenger RNA (mRNA), antisense oligonucleotide, DNA, plasmid, ribosomal RNA (rRNA), microRNA (miRNA), transfer RNA (tRNA), small interfering RNA (siRNA) and small nuclear RNA (snRNA).

13. Use of the lipid particles according to item 11 or 12 in the preparation of a medicament for gene therapy, gene vaccination, antisense therapy or a therapy by RNA interference;

preferably, the gene therapy is useful for the treatment of a cancer and a genetic disease; more preferably, the cancer is one or more of lung cancer, gastric cancer, liver cancer, esophageal cancer, colon cancer, pancreatic cancer, brain cancer, lymphatic cancer, hematologic cancer or prostate cancer, and the genetic disease is one or more of hemophilia, thalassemia and Gaucher disease;
preferably, the gene vaccination is useful for the treatment of cancer, allergy, toxicity and pathogen infection; more preferably, the pathogen is one or more of viruses, bacteria or fungi.

14. Use of the lipid particles according to item 11 or 12 in the preparation of a medicament for nucleic acid transfer, preferably, the nucleic acid is RNA, messenger RNA (mRNA), antisense oligonucleotide, DNA, plasmid, ribosomal RNA (rRNA), microRNA (miRNA), transfer RNA (tRNA), small interfering RNA (siRNA) and small nuclear RNA (snRNA).

## Beneficial effects

The amino lipid compound of the present invention is characterized in having functional groups of a wide diversity and a large quantity; the preparation method of the amino lipid compound has advantages such as easily available raw materials, mild reaction conditions, excellent reaction selectivity, high reaction yield, low equipment requirements and simple operations; and the preparation method of the present invention is very universal, and can be used for rapid synthesis of libraries of ionizable amino lipid compounds and for rapid cell-based screening experiments at a low cost.

The amino lipid compound provided by the present invention has such functional groups that include various types and combination modes, and can be conveniently adapted to the delivery of different nucleic acid substances to different cells through conventional screening with regard to transfection efficiency, cytotoxicity, adhesion of agents to be delivered to cells, stability of lipid particles, size of lipid particles and other characteristics, thereby improving the specificity and effectiveness of the delivery system.

The amino lipid compound of the present invention can be synthesized by way of combinatorial chemistry with

high throughput and high efficiency, and the synthesis method is simple and high-yield.

Lipid particles or liposomes comprising the amino lipid compound of the present invention have excellent performances in encapsulating bioactive agents, and can be used for the delivery of bioactive agents, especially poorly water-soluble drugs or easily decomposable or degradable active agents (such as nucleic acids) to improve the bioavailability and efficacy thereof.

To make the objects, technical solutions and advantages of the present invention clearer, the present invention will be described below with reference to specific examples. The described examples are part of the embodiments of the present invention, but not all of them. Based on the examples of the present invention, all other embodiments obtained by those of ordinary skill in the art without creative work are within the scope of the present invention.

## Examples

Unless otherwise specified, all chemical reagents used in the examples are analytically pure and purchased from TCI, Aladdin, J&K, etc.

### Example 1: Synthesis of amino lipid compounds

In order to systematically study the relationship between structure such as the length of alkyl chains, the combination modes of alkyl chains, ionizable head groups, and transfection capacity, 11 linear alkylthiols with chains of 6-18 carbons (S6-S18), 11 alkylamines with chains of 6-24 carbons (N6-$^{2}$N12), and 10 diamines with different structures from one another (D1-D6) were selected. Libraries of 2530 compounds comprising both two alkyl chains and ionizable head groups were synthesized by way of combinatorial chemistry with high throughput, the synthesis route being as follows:

**(a) triazine as a linker**

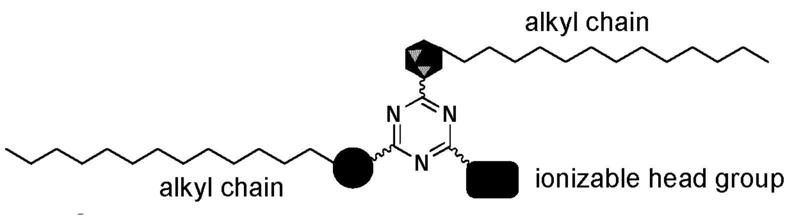


**(b) typical example**

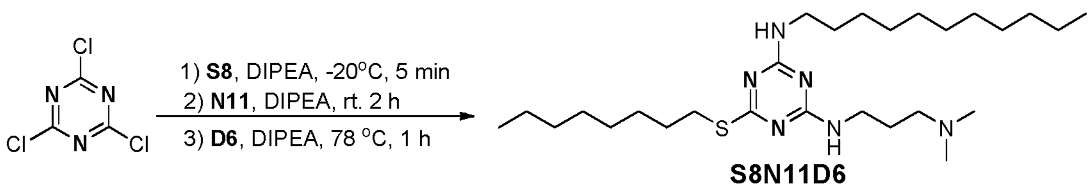


**S8N11D6**

**(c) Synthetic building blocks**

| Diamines | | | | | Thiols and amines | |
|---|---|---|---|---|---|---|
| **D1** | 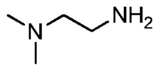 | **D6** | 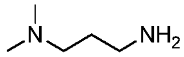 | | 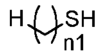 | 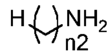 |
| **D2** | 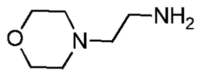 | **D7** | 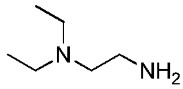 | | **S6** n1 = 6 | **N6** n2 = 6 |
| | | | | | **S7** n1 = 7 | **N7** n2 = 7 |
| | | | | | **S8** n1 =8 | **N8** n2 =8 |
| **D3** | 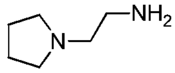 | **D8** | 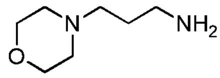 | | **S9** n1 = 9 | **N9** n2 = 9 |
| | | | | | **S10** n1 =10 | **N11** n2 = 11 |
| | | | | | **S11** n1 = 11 | **N12** n2 = 12 |
| **D4** | 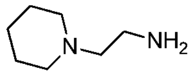 | **D9** | 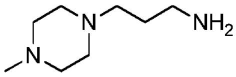 | | **S12** n1 = 12 | **N13** n2 = 13 |
| | | | | | **S14** n1 = 14 | **N15** n2 = 15 |
| **D5** | 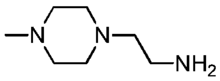 | **D10** | 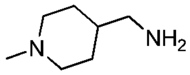 | | **S15** n1 = 15 | **N16** n2 = 16 |
| | | | | | **S16** n1 = 16 | **N18** n2 = 18 |
| | | | | | **S18** n1 = 18 | $^{2}$**N**$_{12}$ $(n\text{-}C_{12}H_{25})_2NH$ |

**(d) Compound libraries**

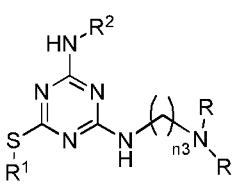

**Compound library S-N-D**

**11×11×10=1210**

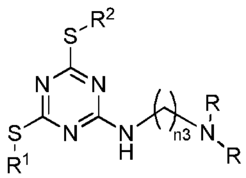

**Compound library S-S-D**

**66×10=660**

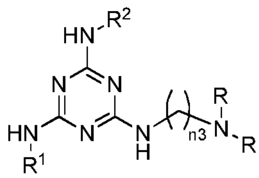

**Compound library N-N-D**

**66×10=660**

wherein n3=1, 2 or 3; -NRR represents the dialkylamino or cyclic amino moiety in the diamines D1 to D10 described above; and $R^1$ and $R^2$ are as defined above.

The synthesized compounds have one of the structures shown in the following table I:

Table I. Synthesized compounds

| Compound SzNxDy | Compound SzSzDy | Compound NxNxDy |
|---|---|---|
| 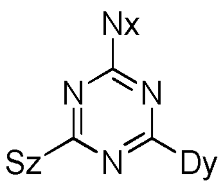 | 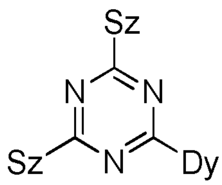 | 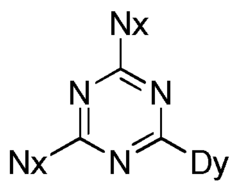 |

wherein each Sz independently represents a group selected from S6 to S12, S14 to S16 and S18 listed in Table II below;

each Nx independently represents a group selected from N6 to N9, N11 to N13, N15, N16, N18 and $^{2}N_{12}$ listed in Table II below; and

each Dy independently represents a group selected from D1 to D10 listed in the following Table II:

Table II

| Group Sz | | Group Nx | | Group Dy | |
|---|---|---|---|---|---|
| **S6** | $-S-(CH_2)_6-H$ | **N6** | $-NH-(CH_2)_6-H$ | **D1** | 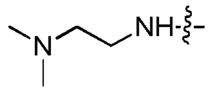 |
| **S7** | $-S-(CH_2)_7-H$ | **N7** | $-NH-(CH_2)_7-H$ | **D2** | 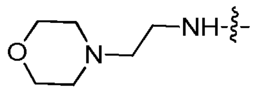 |
| **S8** | $-S-(CH_2)_8-H$ | **N8** | $-NH-(CH_2)_8-H$ | **D3** | 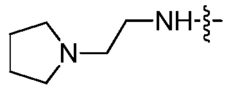 |
| **S9** | $-S-(CH_2)_9-H$ | **N9** | $-NH-(CH_2)_9-H$ | **D4** | 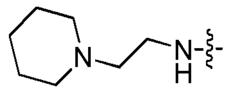 |
| **S10** | $-S-(CH_2)_{10}-H$ | **N11** | $-NH-(CH_2)_{11}-H$ | **D5** | 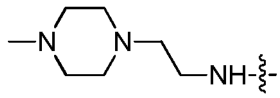 |
| **S11** | $-S-(CH_2)_{11}-H$ | **N12** | $-NH-(CH_2)_{12}-H$ | **D6** | 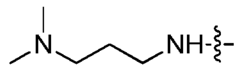 |
| **S12** | $-S-(CH_2)_{12}-H$ | **N13** | $-NH-(CH_2)_{13}-H$ | **D7** | 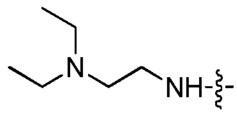 |
| **S14** | $-S-(CH_2)_{14}-H$ | **N15** | $-NHS-(CH_2)_{15}-H$ | **D8** | 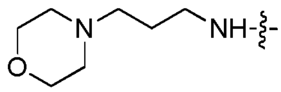 |
| **S15** | $-S-(CH_2)_{15}-H$ | **N16** | $-NH-(CH_2)_{16}-H$ | **D9** | 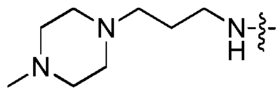 |
| **S16** | $-S-(CH_2)_{16}-H$ | **N18** | $-NH-(CH_2)_{18}-H$ | **D10** | 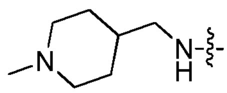 |
| **S18** | $-S-(CH_2)_{18}-H$ | **$^2N_{12}$** | $-N-((CH_2)_{12}-H)_2$ | | |

The mass spectrum ($[M+H]^+$) data measured for the synthesized compounds are shown in Table III below:

Table III

| | D1 | D2 | D3 | D4 | D5 | D6 | D7 | D8 | D9 | D10 |
|---|---|---|---|---|---|---|---|---|---|---|
| **S6N6** | 383.4 | 425.3 | 409.3 | 423.4 | 438.4 | 397.3 | 411.4 | 439.3 | 452.4 | 423.4 |
| **S6N7** | 397.3 | 439.3 | 423.3 | 437.4 | 452.4 | 411.3 | 425.4 | 453.3 | 466.4 | 437.5 |
| **S6N8** | 411.4 | 453.3 | 437.4 | 451.4 | 466.6 | 425.4 | 439.4 | 467.3 | 480.6 | 451.4 |
| **S6N9** | 425.3 | 467.4 | 451.3 | 465.4 | 480.4 | 439.3 | 453.4 | 481.3 | 494.4 | 465.4 |
| **S6N11** | 453.3 | 495.3 | 479.3 | 493.4 | 508.4 | 467.3 | 481.5 | 509.4 | 522.4 | 493.4 |
| **S6N12** | 467.4 | 509.3 | 493.4 | 507.4 | 522.4 | 481.3 | 495.4 | 523.3 | 536.4 | 507.6 |
| **S6N13** | 481.3 | 523.5 | 507.3 | 521.5 | 536.4 | 495.3 | 509.4 | 537.5 | 550.6 | 521.4 |

(continued)

| | D1 | D2 | D3 | D4 | D5 | D6 | D7 | D8 | D9 | D10 |
|---|---|---|---|---|---|---|---|---|---|---|
| **S6N15** | 509.6 | 551.3 | 535.5 | 549.4 | 564.4 | 523.3 | 537.5 | 565.3 | 578.4 | 549.4 |
| **S6N16** | 523.3 | 565.3 | 549.3 | 563.4 | 578.6 | 537.3 | 551.4 | 579.5 | 592.4 | 563.4 |
| **S6N18** | 551.3 | 593.3 | 577.3 | 591.4 | 606.4 | 565.3 | 579.4 | 607.3 | 620.4 | 591.6 |
| **S6²N12** | 635.6 | 677.4 | 661.4 | 675.5 | 690.5 | 649.6 | 663.5 | 691.4 | 704.5 | 675.5 |
| **S7N6** | 397.3 | 439.6 | 423.7 | 437.4 | 452.4 | 411.3 | 425.4 | 453.3 | 466.4 | 437.6 |
| **S7N7** | 411.3 | 453.3 | 437.3 | 451.4 | 466.4 | 425.3 | 439.4 | 467.3 | 480.6 | 451.4 |
| **S7N8** | 425.5 | 467.3 | 451.3 | 465.4 | 480.6 | 439.3 | 453.5 | 481.3 | 494.4 | 465.4 |
| **S7N9** | 439.3 | 481.3 | 465.3 | 479.4 | 494.4 | 453.5 | 467.4 | 495.5 | 508.4 | 479.4 |
| **S7N11** | 467.5 | 509.3 | 493.7 | 507.7 | 522.4 | 481.3 | 495.4 | 523.3 | 536.4 | 507.4 |
| **S7N12** | 481.3 | 523.3 | 507.6 | 521.4 | 536.7 | 495.3 | 509.6 | 537.3 | 550.6 | 521.4 |
| **S7N13** | 495.6 | 537.6 | 521.3 | 535.4 | 550.4 | 50963 | 523.4 | 551.3 | 564.4 | 535.4 |
| **S7N15** | 523.3 | 565.3 | 549.3 | 563.4 | 578.4 | 537.3 | 551.4 | 579.3 | 592.4 | 563.4 |
| **S7N16** | 537.3 | 579.3 | 563.3 | 577.4 | 592.4 | 551.3 | 565.4 | 593.3 | 606.4 | 577.4 |
| **S7N18** | 565.3 | 607.3 | 591.3 | 605.4 | 620.4 | 579.3 | 593.4 | 621.3 | 634.4 | 605.4 |
| **S7²N12** | 649.4 | 691.4 | 675.4 | 689.5 | 704.5 | 663.4 | 677.5 | 705.4 | 718.5 | 689.5 |
| **S8N6** | 411.3 | 453.3 | 437.3 | 451.4 | 466.4 | 425.3 | 439.4 | 467.3 | 480.4 | 451.4 |
| **S8N7** | 425.3 | 467.3 | 451.3 | 465.4 | 480.6 | 439.3 | 453.4 | 481.3 | 494.4 | 465.4 |
| **S8N8** | 439.8 | 481.3 | 465.3 | 479.4 | 494.4 | 453.6 | 467.4 | 495.3 | 508.6 | 479.4 |
| **S8N9** | 453.3 | 495.8 | 479.8 | 493.6 | 508.4 | 467.3 | 481.5 | 509.5 | 522.4 | 493.6 |
| **S8N12** | 495.7 | 537.3 | 521.3 | 535.4 | 550.6 | 509.3 | 523.4 | 551.3 | 564.4 | 535.4 |
| **S8N13** | 509.3 | 551.3 | 535.6 | 549.4 | 564.4 | 523.3 | 537.6 | 565.3 | 578.5 | 549.6 |
| **S8N15** | 537.3 | 579.3 | 563.3 | 577.4 | 592.4 | 551.3 | 565.4 | 593.3 | 606.4 | 577.4 |
| **S8N16** | 551.3 | 593.3 | 577.3 | 591.4 | 606.4 | 565.3 | 579.4 | 607.3 | 620.4 | 591.4 |
| **S8N18** | 579.6 | 621.3 | 605.3 | 619.4 | 634.4 | 593.3 | 607.4 | 635.3 | 648.4 | 619.4 |
| **S8²N12** | 663.4 | 705.4 | 689.4 | 703.5 | 718.5 | 677.4 | 691.5 | 719.4 | 732.5 | 703.5 |
| **S9N6** | 425.3 | 467.3 | 451.3 | 465.4 | 480.7 | 439.3 | 453.4 | 481.3 | 494.4 | 465.6 |
| **S9N7** | 439.3 | 481.3 | 465.3 | 479.6 | 494.4 | 453.3 | 467.4 | 495.3 | 508.4 | 479.4 |
| **S9N8** | 453.6 | 495.3 | 479.3 | 493.4 | 508.4 | 467.3 | 481.4 | 509.6 | 522.4 | 493.4 |
| **S9N9** | 467.3 | 509.3 | 493.3 | 507.4 | 522.4 | 481.3 | 495.4 | 523.3 | 536.4 | 507.4 |
| **S9N11** | 495.3 | 537.3 | 521.6 | 535.4 | 550.4 | 509.3 | 523.4 | 551.3 | 564.4 | 535.6 |
| **S9N12** | 509.3 | 551.6 | 535.3 | 549.4 | 564.4 | 523.3 | 537.4 | 565.3 | 578.4 | 549.4 |
| **S9N13** | 523.6 | 565.6 | 549.3 | 563.4 | 578.4 | 537.3 | 551.4 | 579.3 | 592.4 | 563.4 |
| **S9N15** | 551.3 | 593.3 | 577.3 | 591.4 | 606.4 | 565.3 | 579.4 | 607.3 | 620.4 | 591.4 |
| **S9N16** | 565.3 | 607.3 | 591.3 | 605.4 | 620.4 | 579.3 | 593.4 | 621.3 | 634.4 | 605.4 |
| **S9N18** | 593.3 | 635.3 | 619.6 | 633.4 | 648.4 | 607.3 | 621.4 | 649.3 | 662.4 | 633.4 |
| **S9²N12** | 677.4 | 719.4 | 703.4 | 717.5 | 732.5 | 691.4 | 705.5 | 733.4 | 746.5 | 717.5 |
| **S10N6** | 439.5 | 481.6 | 465.6 | 479.4 | 494.4 | 453.5 | 467.4 | 495.3 | 508.4 | 479.4 |
| **S10N7** | 453.3 | 495.3 | 479.3 | 493.5 | 508.4 | 467.3 | 481.4 | 509.3 | 522.4 | 493.4 |

(continued)

| | D1 | D2 | D3 | D4 | D5 | D6 | D7 | D8 | D9 | D10 |
|---|---|---|---|---|---|---|---|---|---|---|
| **S10N8** | 467.3 | 509.3 | 493.3 | 507.7 | 522.4 | 481.3 | 495.4 | 523.3 | 536.4 | 507.4 |
| **S10N9** | 481.3 | 523.3 | 507.3 | 521.4 | 536.4 | 495.5 | 509.4 | 537.3 | 550.4 | 521.4 |
| **S10N11** | 509.3 | 551.3 | 535.3 | 549.4 | 564.4 | 523.3 | 537.4 | 565.3 | 578.4 | 549.4 |
| **S10N12** | 523.3 | 565.3 | 549.3 | 563.4 | 578.4 | 537.3 | 551.4 | 579.3 | 592.4 | 563.4 |
| **S10N13** | 537.3 | 579.3 | 563.3 | 577.4 | 592.4 | 551.3 | 565.4 | 593.3 | 606.7 | 577.7 |
| **S10N15** | 565.3 | 607.3 | 591.3 | 605.4 | 620.4 | 579.3 | 593.4 | 621.3 | 634.4 | 605.4 |
| **S10N16** | 579.3 | 621.3 | 605.3 | 619.4 | 634.4 | 593.3 | 607.4 | 635.6 | 648.4 | 619.4 |
| **S10N18** | 607.3 | 649.3 | 633.3 | 647.4 | 662.4 | 621.3 | 635.4 | 663.3 | 676.4 | 647.4 |
| **S10$^2$N12** | 691.4 | 733.4 | 717.4 | 731.5 | 746.5 | 705.4 | 719.7 | 747.4 | 760.5 | 731.5 |
| **S11N6** | 453.3 | 495.3 | 479.3 | 493.4 | 508.4 | 467.3 | 481.4 | 509.3 | 522.4 | 493.4 |
| **S11N7** | 467.3 | 509.3 | 493.3 | 507.4 | 522.4 | 481.3 | 495.4 | 523.3 | 536.4 | 507.4 |
| **S11N8** | 481.3 | 523.3 | 507.3 | 521.4 | 536.4 | 495.3 | 509.4 | 537.3 | 550.4 | 521.4 |
| **S11N9** | 495.3 | 537.3 | 521.6 | 535.4 | 550.4 | 509.3 | 523.4 | 551.3 | 564.4 | 535.4 |
| **S11N11** | 523.3 | 565.3 | 549.3 | 563.4 | 578.6 | 537.3 | 551.4 | 579.3 | 592.4 | 563.4 |
| **S11N12** | 537.6 | 579.3 | 563.3 | 577.4 | 592.4 | 551.3 | 565.4 | 593.3 | 606.4 | 577.6 |
| **S11N13** | 551.3 | 593.3 | 577.3 | 591.4 | 606.4 | 565.3 | 579.4 | 607.3 | 620.4 | 591.4 |
| **S11N15** | 579.6 | 621.3 | 605.6 | 619.4 | 634.4 | 593.3 | 607.4 | 635.3 | 648.4 | 619.4 |
| **S11N16** | 593.3 | 635.3 | 619.3 | 633.4 | 648.4 | 607.3 | 621.4 | 649.3 | 662.4 | 633.4 |
| **S11N18** | 621.3 | 663.3 | 647.3 | 661.4 | 676.4 | 635.3 | 649.4 | 677.3 | 690.4 | 661.4 |
| **S11$^2$N12** | 705.4 | 747.4 | 731.6 | 745.7 | 760.5 | 719.4 | 733.5 | 761.4 | 774.5 | 745.5 |
| **S12N6** | 467.3 | 509.3 | 493.3 | 507.4 | 522.4 | 481.3 | 495.4 | 523.3 | 536.4 | 507.4 |
| **S12N7** | 481.3 | 523.3 | 507.3 | 521.4 | 536.4 | 495.3 | 509.4 | 537.3 | 550.4 | 521.4 |
| **S12N8** | 495.3 | 537.3 | 521.3 | 535.4 | 550.4 | 509.3 | 523.4 | 551.3 | 564.4 | 535.4 |
| **S12N9** | 509.3 | 551.3 | 535.3 | 549.4 | 564.4 | 523.3 | 537.4 | 565.3 | 578.4 | 549.4 |
| **S12N11** | 537.3 | 579.3 | 563.3 | 577.5 | 592.7 | 551.3 | 565.4 | 593.3 | 606.4 | 577.4 |
| **S12N12** | 551.3 | 593.3 | 577.3 | 591.4 | 606.4 | 565.3 | 579.7 | 607.3 | 620.4 | 591.4 |
| **S12N13** | 565.3 | 607.3 | 591.3 | 605.4 | 620.4 | 579.3 | 593.4 | 621.3 | 634.4 | 605.4 |
| **S12N15** | 593.3 | 635.3 | 619.3 | 633.4 | 648.4 | 607.3 | 621.4 | 649.3 | 662.4 | 633.4 |
| **S12N16** | 607.3 | 649.3 | 633.3 | 647.4 | 662.4 | 621.3 | 635.4 | 663.3 | 676.4 | 647.4 |
| **S12N18** | 635.3 | 677.3 | 661.3 | 675.4 | 690.4 | 649.3 | 663.4 | 691.5 | 704.4 | 675.4 |
| **S12$^2$N12** | 719.5 | 761.4 | 745.4 | 759.5 | 774.5 | 733.4 | 747.5 | 775.4 | 788.5 | 759.5 |
| **S14N6** | 495.3 | 537.3 | 521.3 | 535.4 | 550.4 | 509.3 | 523.4 | 551.3 | 564.5 | 535.5 |
| **S14N7** | 509.3 | 551.5 | 535.3 | 549.4 | 564.4 | 523.3 | 537.4 | 565.3 | 578.4 | 549.4 |
| **S14N8** | 523.5 | 565.3 | 549.3 | 563.4 | 578.4 | 537.5 | 551.4 | 579.3 | 592.4 | 563.4 |
| **S14N9** | 537.3 | 579.6 | 563.3 | 577.4 | 592.5 | 5516 | 565.4 | 593.6 | 606.6 | 577.4 |
| **S14N11** | 565.3 | 607.3 | 591.6 | 605.4 | 620.4 | 579.3 | 593.4 | 621.3 | 634.4 | 605.4 |
| **S14N12** | 579.5 | 621.3 | 605.3 | 619.4 | 634.4 | 593.3 | 607.4 | 635.3 | 648.4 | 619.4 |
| **S14N13** | 593.3 | 635.6 | 619.3 | 633.4 | 648.4 | 607.3 | 621.4 | 649.3 | 662.4 | 633.4 |

(continued)

| | D1 | D2 | D3 | D4 | D5 | D6 | D7 | D8 | D9 | D10 |
|---|---|---|---|---|---|---|---|---|---|---|
| **S14N15** | 621.3 | 663.3 | 647.3 | 661.4 | 676.4 | 635.3 | 649.4 | 677.3 | 690.4 | 661.4 |
| **S14N16** | 635.6 | 677.3 | 661.3 | 675.4 | 690.4 | 649.3 | 663.4 | 691.3 | 704.4 | 675.4 |
| **S14N18** | 663.3 | 705.3 | 689.3 | 703.4 | 718.4 | 677.3 | 691.4 | 719.3 | 732.4 | 703.6 |
| **S14$^2$N12** | 747.4 | 789.4 | 773.4 | 787.5 | 802.5 | 761.4 | 775.6 | 803.4 | 816.5 | 787.5 |
| **S15N6** | 509.3 | 551.7 | 535.3 | 549.4 | 564.4 | 523.6 | 537.4 | 565.3 | 578.6 | 549.4 |
| **S15N7** | 523.7 | 565.3 | 549.3 | 563.4 | 578.4 | 537.3 | 551.4 | 5796 | 592.4 | 563.4 |
| **S15N8** | 537.3 | 579.3 | 563.3 | 577.4 | 592.4 | 551.3 | 565.4 | 593.3 | 606.4 | 577.4 |
| **S15N9** | 551.3 | 593.3 | 577.7 | 591.4 | 606.4 | 565.3 | 579.4 | 607.3 | 620.4 | 591.4 |
| **S15N11** | 579.7 | 621.3 | 605.3 | 619.7 | 634.6 | 593.3 | 607.7 | 635.3 | 648.8 | 619.9 |
| **S15N12** | 593.3 | 635.3 | 619.3 | 633.4 | 648.4 | 607.3 | 621.4 | 649.3 | 662.4 | 633.4 |
| **S15N13** | 607.5 | 649.3 | 633.3 | 647.7 | 662.4 | 621.3 | 635.4 | 663.3 | 676.4 | 647.4 |
| **S15N15** | 635.3 | 677.3 | 6615 | 675.4 | 690.4 | 649.3 | 663.4 | 691.3 | 704.4 | 675.4 |
| **S15N16** | 649.3 | 691.5 | 675.3 | 689.4 | 704.7 | 663.3 | 677.4 | 705.3 | 718.4 | 689.4 |
| **S15N18** | 677.3 | 719.3 | 703.3 | 717.4 | 732.4 | 691.3 | 705.4 | 733.3 | 746.4 | 717.4 |
| **S15$^2$N12** | 761.4 | 803.4 | 787.4 | 801.5 | 816.5 | 775.4 | 789.5 | 817.4 | 830.5 | 801.5 |
| **S16N6** | 523.3 | 565.3 | 549.3 | 563.4 | 578.4 | 537.3 | 551.4 | 579.3 | 592.4 | 563.4 |
| **S16N7** | 537.3 | 579.3 | 563.7 | 577.4 | 592.4 | 551.3 | 565.4 | 593.3 | 606.4 | 577.4 |
| **S16N8** | 551.6 | 593.6 | 577.3 | 591.4 | 606.4 | 565.3 | 579.4 | 607.3 | 620.4 | 591.4 |
| **S16N9** | 565.3 | 607.3 | 591.5 | 605.6 | 620.4 | 579.3 | 593.4 | 621.3 | 634.4 | 605.4 |
| **S16N11** | 593.3 | 635.3 | 619.3 | 633.4 | 648.7 | 607.3 | 621.4 | 649.3 | 662.4 | 633.4 |
| **S16N12** | 607.3 | 649.3 | 633.3 | 647.4 | 662.4 | 621.3 | 635.4 | 663.3 | 676.4 | 647.4 |
| **S16N13** | 621.3 | 663.6 | 647.3 | 661.4 | 676.4 | 635.3 | 649.4 | 677.3 | 690.4 | 661.4 |
| **S16N15** | 649.3 | 691.3 | 675.6 | 689.4 | 704.4 | 663.7 | 677.4 | 705.3 | 718.4 | 689.4 |
| **S16N16** | 663.3 | 705.3 | 689.3 | 703.4 | 718.4 | 677.3 | 691.4 | 719.3 | 732.4 | 703.4 |
| **S16N18** | 691.3 | 733.3 | 717.3 | 731.4 | 746.4 | 705.3 | 719.4 | 747.3 | 760.4 | 731.4 |
| **S16$^2$N12** | 775.4 | 817.4 | 801.4 | 815.5 | 830.5 | 789.4 | 803.5 | 831.7 | 844.7 | 815.5 |
| **S18N6** | 551.3 | 593.3 | 577.3 | 591.4 | 606.4 | 565.3 | 579.4 | 607.3 | 620.4 | 591.4 |
| **S18N7** | 565.3 | 607.3 | 591.3 | 605.4 | 620.4 | 579.3 | 593.4 | 621.3 | 634.4 | 605.8 |
| **S18N8** | 579.3 | 621.3 | 605.6 | 619.4 | 634.7 | 593.3 | 607.4 | 635.3 | 648.4 | 619.4 |
| **S18N9** | 593.3 | 635.3 | 619.3 | 633.4 | 648.4 | 607.5 | 621.6 | 649.7 | 662.8 | 633.8 |
| **S18N11** | 621.3 | 663.3 | 647.3 | 661.4 | 676.4 | 635.3 | 649.4 | 677.3 | 690.4 | 661.4 |
| **S18N12** | 635.3 | 677.3 | 661.3 | 675.4 | 690.4 | 649.3 | 663.4 | 691.3 | 704.4 | 675.4 |
| **S18N13** | 649.5 | 691.3 | 675.3 | 689.4 | 704.4 | 663.3 | 677.4 | 705.3 | 718.4 | 689.4 |
| **S18N15** | 677.3 | 719.3 | 703.3 | 717.4 | 732.4 | 691.3 | 705.4 | 733.3 | 746.4 | 717.4 |
| **S18N16** | 691.3 | 733.3 | 717.3 | 731.4 | 746.4 | 705.3 | 719.4 | 747.3 | 760.4 | 731.4 |
| **S18N18** | 719.3 | 761.6 | 745.3 | 759.4 | 774.4 | 733.3 | 747.4 | 775.3 | 788.4 | 759.4 |
| **S18$^2$N12** | 803.4 | 845.4 | 829.7 | 843.7 | 858.5 | 817.4 | 831.5 | 859.4 | 872.5 | 843.5 |
| **S6S6** | 400.3 | 442.3 | 426.3 | 440.4 | 455.7 | 414.3 | 428.4 | 456.3 | 469.4 | 440.4 |

(continued)

| | D1 | D2 | D3 | D4 | D5 | D6 | D7 | D8 | D9 | D10 |
|---|---|---|---|---|---|---|---|---|---|---|
| **S6S7** | 414.3 | 456.3 | 440.3 | 454.4 | 469.4 | 428.3 | 442.4 | 470.3 | 483.4 | 454.4 |
| **S6S8** | 428.3 | 470.3 | 454.3 | 468.4 | 483.4 | 442.3 | 456.4 | 484.3 | 497.4 | 468.4 |
| **S6S9** | 442.3 | 484.3 | 468.3 | 482.4 | 497.4 | 456.3 | 470.4 | 498.3 | 511.4 | 482.4 |
| **S6S10** | 456.3 | 498.3 | 482.3 | 496.4 | 511.4 | 470.3 | 484.4 | 512.3 | 525.4 | 496.4 |
| **S6S11** | 470.3 | 512.3 | 496.3 | 521.4 | 525.4 | 484.3 | 484.3 | 526.5 | 539.4 | 510.4 |
| **S6S12** | 484.6 | 526.6 | 510.3 | 524.4 | 539.4 | 498.3 | 512.4 | 540.3 | 553.4 | 524.4 |
| **S6S14** | 512.3 | 554.3 | 538.5 | 552.4 | 567.4 | 526.3 | 540.4 | 568.3 | 581.4 | 552.4 |
| **S6S15** | 526.5 | 568.3 | 552.3 | 566.4 | 581.4 | 540.4 | 554.4 | 582.3 | 595.4 | 566.4 |
| **S6S16** | 540.3 | 582.3 | 566.3 | 580.4 | 595.4 | 554.3 | 568.4 | 596.3 | 609.4 | 580.4 |
| **S6S18** | 568.3 | 610.3 | 594.3 | 608.4 | 623.4 | 582.3 | 596.4 | 624.3 | 637.4 | 608.4 |
| **S7S7** | 428.5 | 470.3 | 454.3 | 468.4 | 483.4 | 442.3 | 456.4 | 484.3 | 497.4 | 468.4 |
| **S7S8** | 442.3 | 484.3 | 468.3 | 482.4 | 497.4 | 456.3 | 470.4 | 498.3 | 511.4 | 482.4 |
| **S7S9** | 456.3 | 498.3 | 482.3 | 496.4 | 511.4 | 470.3 | 484.4 | 512.3 | 525.4 | 496.4 |
| **S7S10** | 470.3 | 512.5 | 496.3 | 510.4 | 525.4 | 484.3 | 498.4 | 526.3 | 539.4 | 510.4 |
| **S7S11** | 484.3 | 526.3 | 510.3 | 524.4 | 539.4 | 498.3 | 512.4 | 540.3 | 553.4 | 524.4 |
| **S7S12** | 498.7 | 540.3 | 524.3 | 538.4 | 553.4 | 512.3 | 526.4 | 554.3 | 567.4 | 538.4 |
| **S7S14** | 526.3 | 568.3 | 552.3 | 566.4 | 581.4 | 540.3 | 554.4 | 582.3 | 595.4 | 566.4 |
| **S7S15** | 540.3 | 582.3 | 566.3 | 580.4 | 595.4 | 554.3 | 568.4 | 596.3 | 609.4 | 580.4 |
| **S7S16** | 554.6 | 596.3 | 580.3 | 594.4 | 609.4 | 568.3 | 582.4 | 610.3 | 623.4 | 594.4 |
| **S7S18** | 582.3 | 624.3 | 608.3 | 622.4 | 637.4 | 596.3 | 610.4 | 638.3 | 651.4 | 622.4 |
| **S8S8** | 456.8 | 498.3 | 482.3 | 496.4 | 511.4 | 470.3 | 484.4 | 512.3 | 525.4 | 496.4 |
| **S8S9** | 470.3 | 512.3 | 496.3 | 510.6 | 525.6 | 484.3 | 498.6 | 526.3 | 539.4 | 510.4 |
| **S8S10** | 484.7 | 526.3 | 510.3 | 524.4 | 539.4 | 498.3 | 512.4 | 540.3 | 553.8 | 524.7 |
| **S8S11** | 498.3 | 540.3 | 524.3 | 538.4 | 553.4 | 512.3 | 526.4 | 554.3 | 567.4 | 538.4 |
| **S8S12** | 512.3 | 554.3 | 538.3 | 552.4 | 567.4 | 526.3 | 540.4 | 568.3 | 581.4 | 552.4 |
| **S8S14** | 540.3 | 582.3 | 566.3 | 580.4 | 595.4 | 554.3 | 568.4 | 596.3 | 609.4 | 580.4 |
| **S8S15** | 554.3 | 596.3 | 580.3 | 594.4 | 609.4 | 568.3 | 582.4 | 610.3 | 623.4 | 594.4 |
| **S8S16** | 568.3 | 610.3 | 594.3 | 608.4 | 623.4 | 582.3 | 596.4 | 624.3 | 637.4 | 608.4 |
| **S8S18** | 596.3 | 638.3 | 622.3 | 636.4 | 651.4 | 610.3 | 624.4 | 652.3 | 665.4 | 636.4 |
| **S9S9** | 484.3 | 526.3 | 510.3 | 524.4 | 539.4 | 498.3 | 512.4 | 540.3 | 553.4 | 524.4 |
| **S9S10** | 498.3 | 540.7 | 524.6 | 538.4 | 553.4 | 512.3 | 526.4 | 554.3 | 567.4 | 538.4 |
| **S9S11** | 512.3 | 554.3 | 538.3 | 552.4 | 567.4 | 526.3 | 540.4 | 568.3 | 581.4 | 552.4 |
| **S9S12** | 526.3 | 568.3 | 552.3 | 566.4 | 581.4 | 540.3 | 554.4 | 582.3 | 595.4 | 566.4 |
| **S9S14** | 554.3 | 596.3 | 580.3 | 594.4 | 609.4 | 568.3 | 582.4 | 610.3 | 623.4 | 594.4 |
| **S9S15** | 568.3 | 610.6 | 594.3 | 608.5 | 623.4 | 582.5 | 596.6 | 624.3 | 637.4 | 608.4 |
| **S9S16** | 582.3 | 624.3 | 608.3 | 622.4 | 637.4 | 596.3 | 610.4 | 638.7 | 651.4 | 622.5 |
| **S9S18** | 610.3 | 652.3 | 636.3 | 650.4 | 665.4 | 624.3 | 638.4 | 666.3 | 679.4 | 650.4 |
| **S10S10** | 512.3 | 554.3 | 538.3 | 552.4 | 567.4 | 526.3 | 540.4 | 568.3 | 581.4 | 552.4 |

(continued)

| | D1 | D2 | D3 | D4 | D5 | D6 | D7 | D8 | D9 | D10 |
|---|---|---|---|---|---|---|---|---|---|---|
| **S10S11** | 526.3 | 568.3 | 552.3 | 566.4 | 581.4 | 540.3 | 554.4 | 582.3 | 595.4 | 566.4 |
| **S10S12** | 540.3 | 582.3 | 566.3 | 580.4 | 595.4 | 554.3 | 568.4 | 596.3 | 609.4 | 580.4 |
| **S10S14** | 568.3 | 610.6 | 594.3 | 608.4 | 623.4 | 582.3 | 596.4 | 624.3 | 637.4 | 608.4 |
| **S10S15** | 582.3 | 624.3 | 608.3 | 622.4 | 637.4 | 596.3 | 610.4 | 638.3 | 651.4 | 622.4 |
| **S10S16** | 596.3 | 638.3 | 622.3 | 636.4 | 651.4 | 610.3 | 624.4 | 652.3 | 665.4 | 636.4 |
| **S10S18** | 624.3 | 666.3 | 650.3 | 664.4 | 679.4 | 638.3 | 652.4 | 680.3 | 693.4 | 664.4 |
| **S11S11** | 540.5 | 582.3 | 566.5 | 580.4 | 595.4 | 554.3 | 568.4 | 596.3 | 609.4 | 580.4 |
| **S11S12** | 554.3 | 596.3 | 580.3 | 594.4 | 609.4 | 568.3 | 582.4 | 610.3 | 623.4 | 594.4 |
| **S11S14** | 582.3 | 624.5 | 608.3 | 622.4 | 637.4 | 596.3 | 610.4 | 638.3 | 651.4 | 622.4 |
| **S11S15** | 596.3 | 638.3 | 622.6 | 636.4 | 651.4 | 610.3 | 624.4 | 652.3 | 665.4 | 636.4 |
| **S11S16** | 610.3 | 652.3 | 636.3 | 650.7 | 665.4 | 624.3 | 638.4 | 666.3 | 679.4 | 650.4 |
| **S11S18** | 638.3 | 680.3 | 664.3 | 678.4 | 693.4 | 652.3 | 666.4 | 694.3 | 707.4 | 678.4 |
| **S12S12** | 568.3 | 610.5 | 594.3 | 608.4 | 623.6 | 582.7 | 596.4 | 624.3 | 637.4 | 608.4 |
| **S12S14** | 596.3 | 638.3 | 622.3 | 636.4 | 651.4 | 610.3 | 624.4 | 652.3 | 665.4 | 636.4 |
| **S12S15** | 610.3 | 652.3 | 636.3 | 650.4 | 665.4 | 624.3 | 638.4 | 666.3 | 679.4 | 650.4 |
| **S12S16** | 624.3 | 666.3 | 650.3 | 664.4 | 679.4 | 638.3 | 652.4 | 680.3 | 693.4 | 664.4 |
| **S12S18** | 652.3 | 694.3 | 678.3 | 692.4 | 707.4 | 666.3 | 680.8 | 708.9 | 721.4 | 692.4 |
| **S14S14** | 624.3 | 666.3 | 650.3 | 664.4 | 679.4 | 638.3 | 652.4 | 680.3 | 693.4 | 664.4 |
| **S14S15** | 638.3 | 680.6 | 664.3 | 678.4 | 693.4 | 652.5 | 666.4 | 694.3 | 707.4 | 678.4 |
| **S14S16** | 652.3 | 694.3 | 678.3 | 692.4 | 707.4 | 666.3 | 680.4 | 708.3 | 721.4 | 692.4 |
| **S14S18** | 680.4 | 722.3 | 706.3 | 720.8 | 735.7 | 694.3 | 708.4 | 736.3 | 749.4 | 720.4 |
| **S15S15** | 652.3 | 694.5 | 678.3 | 692.4 | 707.4 | 666.3 | 680.4 | 708.3 | 721.4 | 692.4 |
| **S15S16** | 666.3 | 708.3 | 692.3 | 706.4 | 721.4 | 680.3 | 694.4 | 722.3 | 735.4 | 706.4 |
| **S15S18** | 694.3 | 736.3 | 720.3 | 734.4 | 749.4 | 708.3 | 722.4 | 750.3 | 763.4 | 734.4 |
| **S16S16** | 680.3 | 722.3 | 706.3 | 720.4 | 735.4 | 694.3 | 708.4 | 736.3 | 749.4 | 720.4 |
| **S16S18** | 708.3 | 750.3 | 734.3 | 748.4 | 763.4 | 722.3 | 736.4 | 764.3 | 777.4 | 748.4 |
| **S18S18** | 736.3 | 778.3 | 762.3 | 776.4 | 791.4 | 750.3 | 764.4 | 792.3 | 805.4 | 776.4 |
| **N6N6** | 366.4 | 408.4 | 392.4 | 406.8 | 421.5 | 380.4 | 394.5 | 422.4 | 435.5 | 406.5 |
| **N6N7** | 380.4 | 422.4 | 406.4 | 420.5 | 435.5 | 394.4 | 408.5 | 436.4 | 449.5 | 420.5 |
| **N6N8** | 394.4 | 436.4 | 420.4 | 434.5 | 449.6 | 408.4 | 422.5 | 450.4 | 463.5 | 434.5 |
| **N6N9** | 408.4 | 450.4 | 434.4 | 448.5 | 463.5 | 422.4 | 436.5 | 464.4 | 477.5 | 448.5 |
| **N6N11** | 436.4 | 478.4 | 462.4 | 476.4 | 491.4 | 450.4 | 464.4 | 492.4 | 492.4 | 476.4 |
| **N6N12** | 450.4 | 492.4 | 476.4 | 490.5 | 505.5 | 464.4 | 478.5 | 506.4 | 519.5 | 490.5 |
| **N6N13** | 464.4 | 506.4 | 490.4 | 504.5 | 519.5 | 478.4 | 492.5 | 520.4 | 533.5 | 504.5 |
| **N6N15** | 492.4 | 534.4 | 518.4 | 532.5 | 547.5 | 506.4 | 520.5 | 548.4 | 561.5 | 532.5 |
| **N6N16** | 506.4 | 548.4 | 532.4 | 546.5 | 561.5 | 520.4 | 534.5 | 562.4 | 575.5 | 546.5 |
| **N6N18** | 534.4 | 576.4 | 560.4 | 574.5 | 589.5 | 548.4 | 562.5 | 590.4 | 603.5 | 574.5 |
| **$N6^2N12$** | 618.5 | 660.5 | 644.5 | 658.6 | 673.6 | 632.5 | 646.6 | 674.5 | 687.6 | 658.6 |

(continued)

| | D1 | D2 | D3 | D4 | D5 | D6 | D7 | D8 | D9 | D10 |
|---|---|---|---|---|---|---|---|---|---|---|
| **N7N7** | 394.4 | 436.4 | 420.4 | 434.5 | 449.5 | 408.4 | 422.5 | 450.4 | 463.5 | 434.5 |
| **N7N8** | 408.4 | 450.4 | 434.4 | 448.5 | 463.5 | 422.4 | 436.5 | 464.4 | 477.5 | 448.5 |
| **N7N9** | 422.4 | 464.4 | 448.7 | 462.5 | 477.5 | 436.4 | 450.5 | 478.4 | 491.5 | 462.5 |
| **N7N11** | 450.4 | 492.4 | 476.4 | 490.5 | 505.5 | 464.4 | 478.5 | 506.4 | 519.5 | 490.5 |
| **N7N12** | 464.4 | 506.4 | 490.4 | 504.5 | 519.5 | 478.4 | 492.5 | 520.4 | 533.5 | 504.5 |
| **N7N13** | 478.4 | 520.4 | 504.4 | 518.5 | 533.5 | 492.4 | 506.5 | 534.4 | 547.5 | 518.5 |
| **N7N15** | 506.8 | 548.4 | 532.8 | 546.5 | 561.5 | 520.5 | 534.5 | 562.4 | 575.5 | 546.5 |
| **N7N16** | 520.4 | 562.4 | 546.4 | 560.5 | 575.5 | 534.4 | 548.5 | 576.4 | 589.5 | 560.5 |
| **N7N18** | 548.4 | 590.4 | 574.4 | 588.5 | 603.5 | 562.4 | 576.5 | 604.4 | 617.5 | 588.5 |
| **N7$^2$N12** | 632.7 | 674.7 | 658.5 | 672.6 | 687.6 | 646.5 | 660.6 | 688.5 | 701.6 | 672.6 |
| **N8N8** | 422.4 | 464.8 | 448.4 | 462.5 | 477.5 | 436.4 | 450.5 | 478.4 | 491.5 | 462.5 |
| **N8N9** | 436.4 | 478.4 | 462.4 | 476.5 | 491.5 | 450.4 | 464.5 | 492.4 | 505.5 | 476.5 |
| **N8N11** | 464.7 | 506.4 | 490.4 | 504.5 | 519.5 | 478.4 | 492.5 | 520.4 | 533.5 | 504.5 |
| **N8N12** | 478.5 | 520.4 | 504.4 | 518.5 | 533.8 | 492.4 | 506.5 | 534.4 | 547.5 | 518.5 |
| **N8N13** | 492.4 | 534.7 | 518.4 | 532.5 | 547.5 | 506.4 | 520.5 | 548.4 | 561.5 | 532.5 |
| **N8N15** | 520.4 | 562.6 | 546.4 | 560.5 | 575.5 | 534.4 | 548.5 | 576.4 | 589.5 | 560.5 |
| **N8N16** | 534.4 | 576.8 | 560.4 | 574.5 | 589.5 | 548.4 | 562.5 | 590.4 | 603.5 | 574.5 |
| **N8N18** | 562.4 | 604.4 | 588.4 | 602.5 | 617.5 | 576.4 | 590.5 | 618.4 | 631.5 | 602.5 |
| **N8$^2$N12** | 646.5 | 688.5 | 672.5 | 686.6 | 701.6 | 660.5 | 674.6 | 702.5 | 715.6 | 686.6 |
| **N9N9** | 450.4 | 492.4 | 476.4 | 490.5 | 505.5 | 464.4 | 478.5 | 506.4 | 519.5 | 490.5 |
| **N9N11** | 478.4 | 520.4 | 504.4 | 518.5 | 533.5 | 492.4 | 506.5 | 534.4 | 547.5 | 518.5 |
| **N9N12** | 492.4 | 534.4 | 518.7 | 532.5 | 547.5 | 506.4 | 520.5 | 548.4 | 561.5 | 532.5 |
| **N9N13** | 506.4 | 548.4 | 532.4 | 546.5 | 561.5 | 520.4 | 534.5 | 562.4 | 575.5 | 546.5 |
| **N9N15** | 534.4 | 576.4 | 560.4 | 574.9 | 589.5 | 548.4 | 562.5 | 590.4 | 603.5 | 574.5 |
| **N9N16** | 548.4 | 590.4 | 574.4 | 588.5 | 603.5 | 562.9 | 576.5 | 604.4 | 617.5 | 588.5 |
| **N9N18** | 576.8 | 618.4 | 602.4 | 616.5 | 631.5 | 590.4 | 604.5 | 632.4 | 645.9 | 616.5 |
| **N9$^2$N12** | 660.5 | 702.5 | 686.9 | 700.6 | 715.6 | 674.5 | 688.6 | 716.5 | 729.6 | 700.6 |
| **N11N11** | 506.4 | 548.4 | 532.4 | 546.5 | 561.5 | 520.4 | 534.6 | 562.4 | 575.5 | 546.5 |
| **N11N12** | 520.4 | 562.4 | 546.4 | 560.5 | 575.5 | 534.4 | 548.5 | 576.4 | 589.5 | 560.5 |
| **N11N13** | 534.4 | 576.4 | 560.4 | 574.5 | 589.5 | 548.4 | 562.5 | 590.4 | 603.5 | 574.5 |
| **N11N15** | 562.4 | 604.4 | 588.4 | 602.5 | 617.5 | 576.4 | 590.5 | 618.4 | 631.5 | 602.5 |
| **N11N16** | 576.4 | 618.4 | 602.4 | 616.5 | 631.5 | 590.4 | 604.5 | 632.4 | 645.5 | 616.5 |
| **N11N18** | 604.4 | 646.4 | 630.4 | 644.5 | 659.5 | 618.4 | 632.5 | 660.4 | 673.5 | 644.5 |
| **N11$^2$N12** | 688.5 | 730.5 | 714.5 | 728.6 | 743.6 | 702.5 | 716.6 | 744.5 | 757.6 | 728.6 |
| **N12N12** | 534.4 | 576.4 | 560.4 | 574.5 | 589.5 | 548.4 | 562.5 | 590.4 | 603.5 | 574.5 |
| **N12N13** | 548.4 | 590.4 | 574.4 | 588.5 | 603.5 | 562.4 | 576.5 | 604.4 | 617.8 | 588.5 |
| **N12N15** | 576.4 | 618.4 | 602.4 | 616.5 | 631.5 | 590.4 | 604.5 | 632.8 | 645.5 | 616.5 |
| **N12N16** | 590.4 | 632.4 | 616.4 | 630.5 | 645.5 | 604.4 | 618.8 | 646.4 | 659.5 | 630.8 |

(continued)

| | D1 | D2 | D3 | D4 | D5 | D6 | D7 | D8 | D9 | D10 |
|---|---|---|---|---|---|---|---|---|---|---|
| **N12N18** | 618.4 | 660.4 | 644.4 | 658.5 | 673.5 | 632.4 | 646.5 | 674.4 | 687.5 | 658.5 |
| **N12$^2$N12** | 702.5 | 744.5 | 728.8 | 742.6 | 757.6 | 716.5 | 730.8 | 758.5 | 771.6 | 742.6 |
| **N13N13** | 562.4 | 604.4 | 588.4 | 602.8 | 617.5 | 576.4 | 590.5 | 618.4 | 631.5 | 602.5 |
| **N13N15** | 590.4 | 632.4 | 616.4 | 630.5 | 645.5 | 604.4 | 618.5 | 646.4 | 659.5 | 630.5 |
| **N13N16** | 604.4 | 646.4 | 630.4 | 644.5 | 659.8 | 618.4 | 632.5 | 660.4 | 673.5 | 644.5 |
| **N13N18** | 632.4 | 674.4 | 658.4 | 672.5 | 687.5 | 646.4 | 660.5 | 688.4 | 701.5 | 672.5 |
| **N13$^2$N12** | 716.5 | 758.5 | 742.5 | 756.6 | 771.6 | 730.6 | 744.6 | 772.5 | 785.6 | 756.6 |
| **N15N15** | 618.4 | 660.4 | 644.4 | 658.5 | 673.5 | 632.4 | 646.5 | 674.4 | 687.5 | 658.5 |
| **N15N16** | 632.4 | 674.4 | 658.4 | 672.5 | 687.9 | 646.4 | 660.5 | 688.4 | 701.5 | 672.5 |
| **N15N18** | 660.4 | 702.4 | 686.4 | 700.5 | 715.5 | 674.4 | 688.5 | 716.4 | 729.5 | 700.5 |
| **N15$^2$N12** | 744.5 | 786.5 | 770.5 | 784.6 | 799.6 | 758.5 | 772.6 | 800.5 | 813.6 | 784.6 |
| **N16N16** | 646.9 | 688.4 | 672.4 | 686.9 | 701.5 | 660.4 | 674.5 | 702.4 | 715.5 | 686.5 |
| **N16N18** | 674.4 | 716.9 | 700.4 | 714.7 | 729.5 | 688.4 | 702.5 | 730.4 | 743.5 | 714.5 |
| **N16$^2$N12** | 758.5 | 800.5 | 784.5 | 798.6 | 813.6 | 772.5 | 786.6 | 814.5 | 827.6 | 798.6 |
| **N18N18** | 702.9 | 744.4 | 728.4 | 742.5 | 757.5 | 716.8 | 730.5 | 758.4 | 771.5 | 742.5 |
| **N18$^2$N12** | 786.5 | 828.5 | 812.5 | 826.6 | 841.6 | 800.5 | 814.6 | 842.5 | 855.6 | 826.6 |
| **$^2$N12$^2$N12** | 870.6 | 912.6 | 896.9 | 910.7 | 926.1 | 884.6 | 898.9 | 926.6 | 939.7 | 911.2 |

**Example 1.1: Parallel synthesis and characterization of the library** of **amino lipid compounds of S8NxDy series**

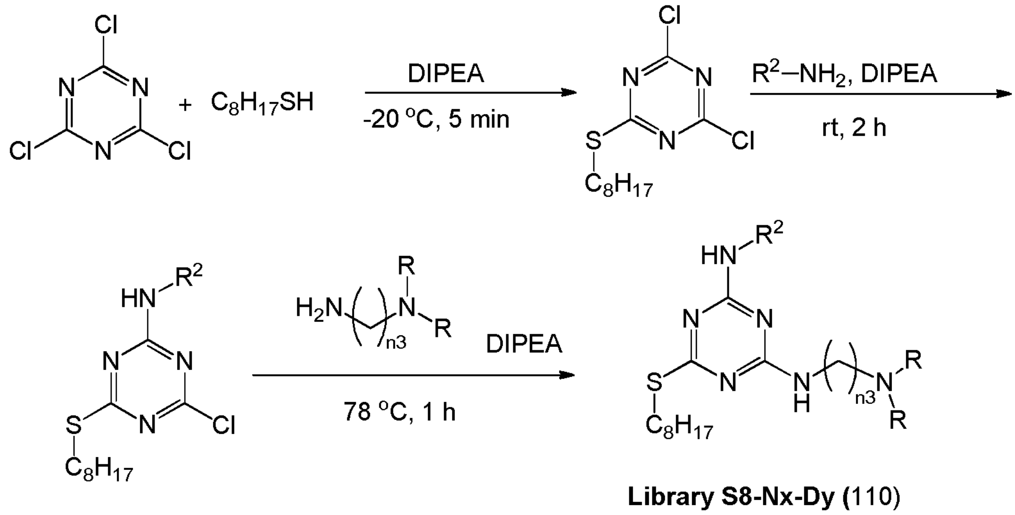


**Library S8-Nx-Dy (**110)

wherein n3 -NRR, R$^1$ and R$^2$ are defined above.

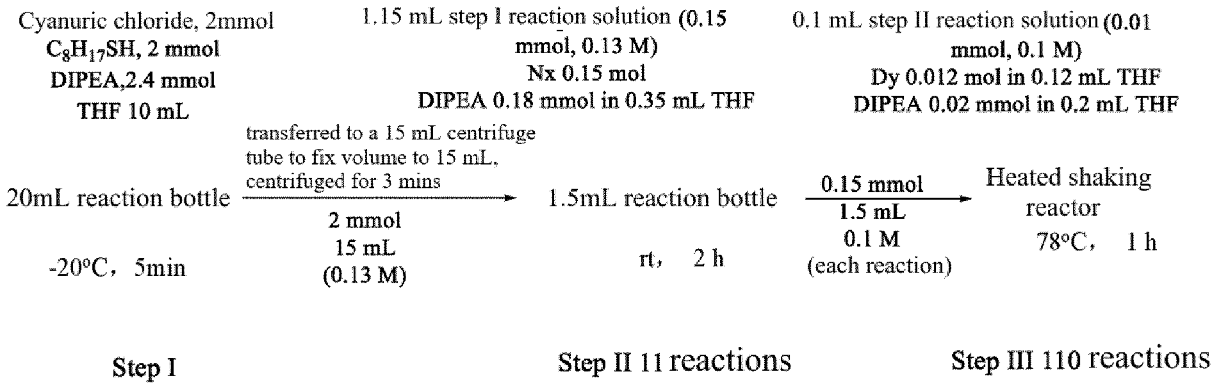


Cyanuric chloride (2 mmol) and THF (10 mL) were added to a 50 mL dry reaction bottle, and cooled to -20°C under stirring in a cold bath, followed by successive addition of 1-octanethiol (2 mmol) and DIPEA (2.4 mmol), and a large amount of precipitate immediately occurred in the reaction solution. TLC detection was performed after reaction for 5 min, the reaction was complete. The reaction solution was transferred to a 15 mL centrifuge tube, fixed to a volume of 15 mL, and then centrifuged in a centrifuge (2000 r/min, 1 min). Step I reaction solution (15 mL, 0.13 M) was then obtained.

The Step I reaction solution was transferred to 11 1.5 mL reaction bottles (each 1.15 mL, 0.15 mmol) with a pipette, and a linear alkylamine (0.15 mmol) and a solution of DIPEA in THF (0.35 mL, 0.5 M) were added to the corresponding reaction bottle, and reacted for 2 h under stirring at room temperature. TLC detection showed no Step I raw material.

Each Step II reaction solution was transferred to 10 1.5 mL EP tubes (each 0.1mL, 0.01 mmol) with a pipette, and a solution of a diamine in THF (0.12 mL, 0.012 mmol, 0.1M) and a solution of DIPEA in THF (0.02 mL, 0.1M) were added to the corresponding EP tube, followed by reaction for 1 h in a heated shaking reactor (Thermo-Shaker) at 78°C. TLC detection showed no Step II raw material. After the reaction was complete, the solvent in the reaction tube was evaporated to dryness at normal temperature to obtain 110 amino lipid compounds **S8NxDy.** 10 representative compounds among them were selected for measurement by mass spectrometry. The results are shown in Table 1 below.

Table 1: MW/z values of representative compounds

| | Compound No. | Structural formula | Molecular formula | Molecular weight | Found $(M+H)^+$ |
|---|---|---|---|---|---|
| 1 | **S8N11D1** | 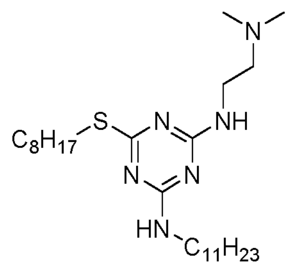 | C26H52N6S | 480.4 | 481.5 |
| 2 | **S8N11D2** | 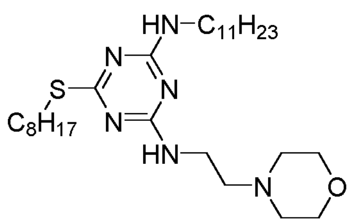 | C28H54N60S | 522.4 | 523.4 |

(continued)

| | Compound No. | Structural formula | Molecular formula | Molecular weight | Found (M+H)+ |
|---|---|---|---|---|---|
| 3 | **S8N11D3** | 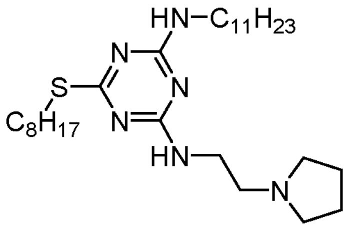 | C28H54N6S | 506.4 | 507.6 |
| 4 | **S8N11D4** | 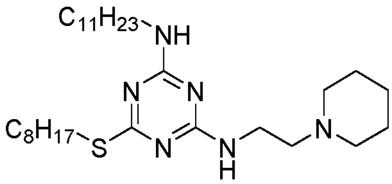 | C29H56N6S | 520.4 | 521.7 |
| 5 | **S8N11D5** | 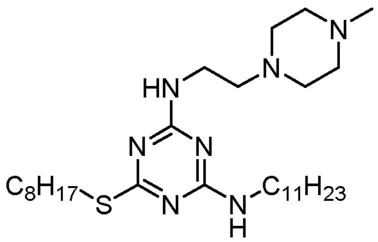 | C29H57N7S | 535.4 | 536.5 |
| 6 | **S8N11D6** | 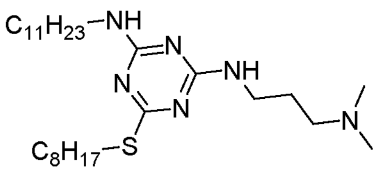 | C27H54N6S | 494.4 | 495.6 |
| 7 | **S8N11D7** | 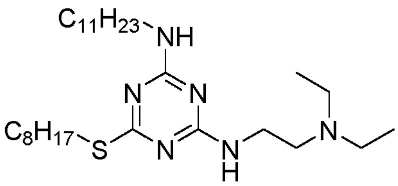 | C28H56N6S | 508.4 | 509.6 |
| 8 | **S8N11D8** | 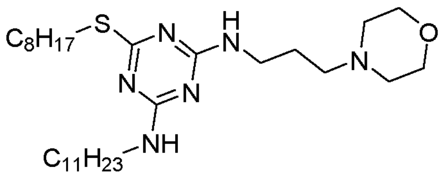 | C29H56N6OS | 536.4 | 537.7 |
| 9 | **S8N11D9** | 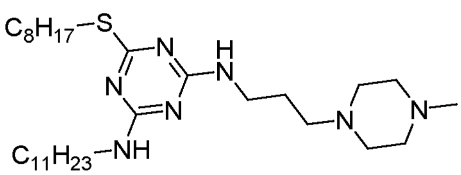 | C30H59N7S | 549.5 | 550.6 |

(continued)

| | Compound No. | Structural formula | Molecular formula | Molecular weight | Found $(M+H)^+$ |
|---|---|---|---|---|---|
| 10 | **S8N11D10** | 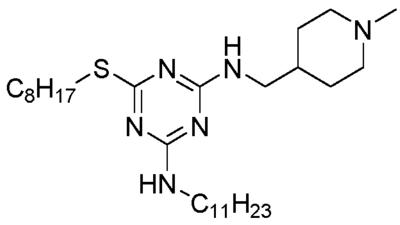 | C29H56N6S | 520.4 | 521.6 |

**Example 1.1.1: One-pot synthesis and characterization of representative amino lipid compound S8N11D6**

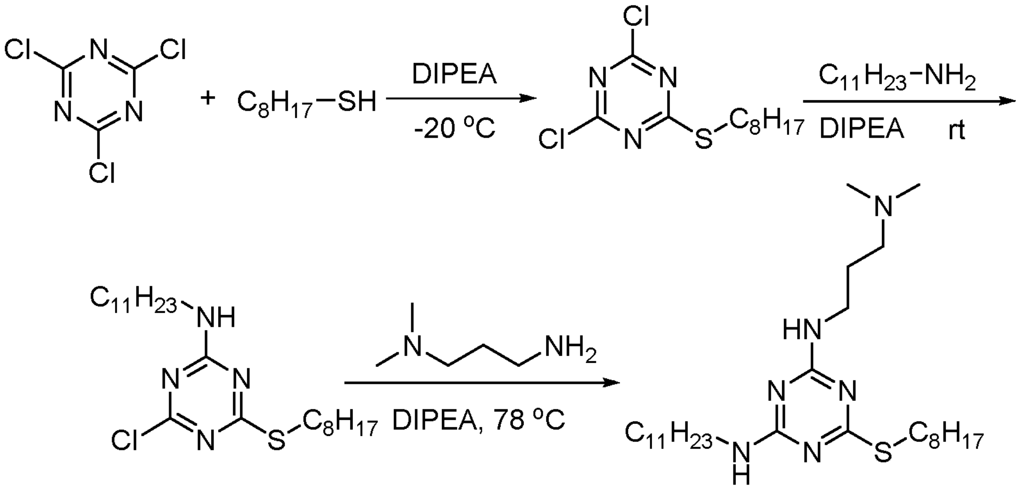


**S8N11D6**

Cyanuric chloride (1 mmol) and THF (10 mL) were added to a 20 mL dry reaction tube and cooled to -20°C under stirring in a cold bath, followed by successive addition of 1-octanethiol (1 mmol) and DIPEA (1.2 mmol), and a large amount of precipitate immediately occurred in the reaction solution. TLC detection was performed after reaction for 5 min, the reaction was complete. After the reaction solution was warmed to room temperature, undecylamine (1 mmol) and DIPEA (1.2 mol) were added, and reacted for 2 h under stirring at room temperature. TLC detection showed no Step I raw material. N,N-dimethylpropanediamine (1.2 mmol) and DIPEA (2 mol) were then added, warmed to 78°C under stirring and reacted for 1 h. TLC detection showed no Step II raw material. Purification by an automatic purification system (forward silica column, eluting with a gradient of 0 to 10% DCM:MeOH) to obtain 440.4 mg of a white solid, with a yield of 89% for the reactions of three steps, and a purity of > 99%. $^1H$ NMR (400 MHz, $CDCl_3$) $\delta$ 3.54 (m, 2H), 3.34 (m, 2H), 3.01 (m, 4H), 2.80-2.73 (m, 6H), 2.07 (m, 2H), 1.67 (m, 2H), 1.53 (m, 2H), 1.38 (m, 2H), 1.28 (m, 2H), 1.24 (m, 24H), 0.88-0.84 (m, 6H). ESI-MS calculated for C27H55N6S+ [M+H]+ 495.4, found 495.6.

**Example 1.2: Parallel synthesis and characterization of the library of amino lipid compound of S6SxDy series**

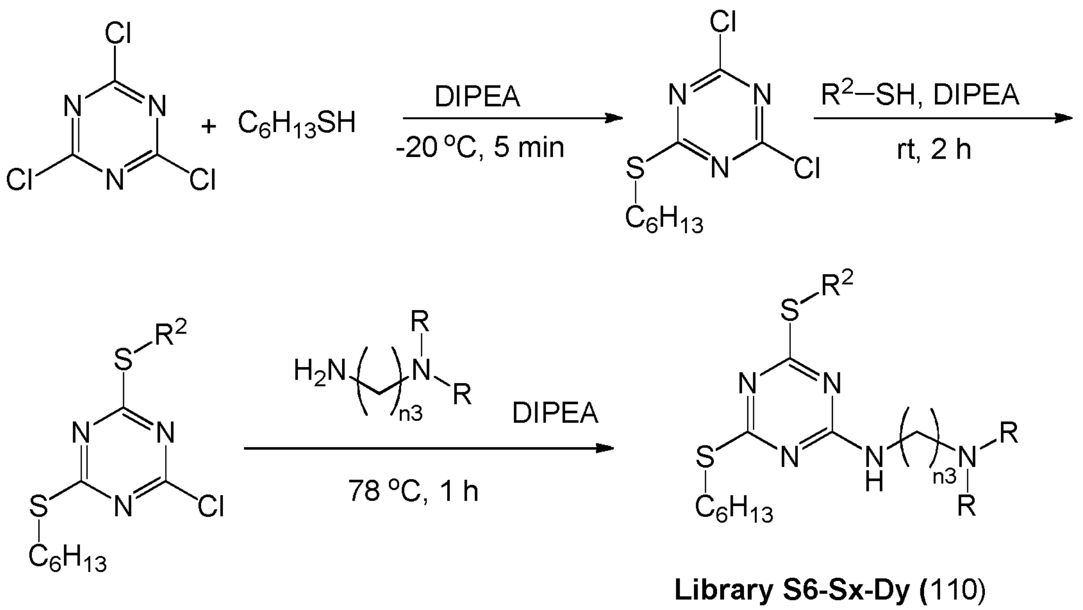


**Library S6-Sx-Dy (**110)

wherein n3, -NRR, $R^1$ and $R^2$ are as defined above.

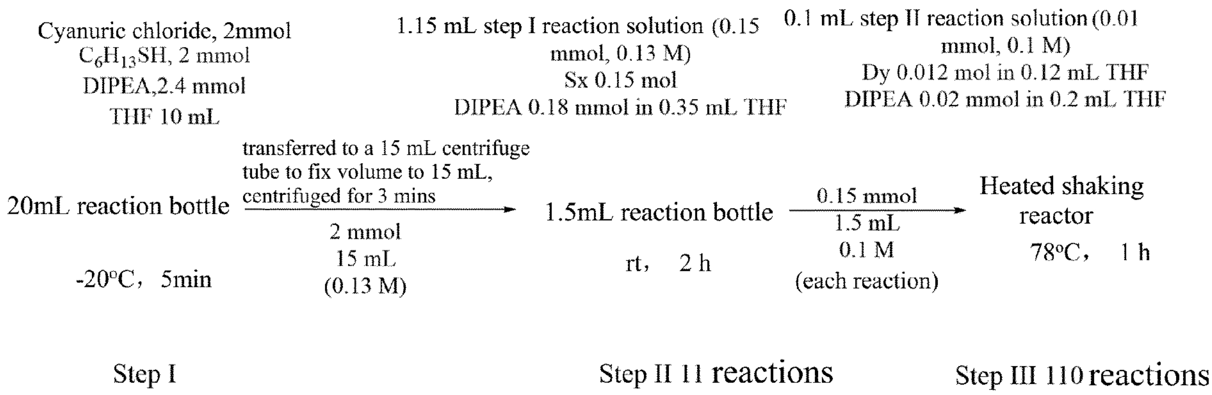


Cyanuric chloride (2 mmol) and THF (10 mL) were added to a 50 mL dry reaction bottle and cooled to -20°C under stirring in a cold bath, followed by successive addition of 1-hexanethiol (2 mmol) and DIPEA (2.4 mmol), and a large amount of precipitate immediately occurred in the reaction solution. TLC detection was performed after reaction for 5 min, the reaction was complete. The reaction solution was transferred to a 15 mL centrifuge tube, fixed to a volume of 15 mL, and then centrifuged in a centrifuge (2000 r/min, 1 min). Step I reaction solution (15 mL, 0.13 M) was then obtained.

The Step I reaction solution was transferred to 11 1.5 mL reaction bottles (each 1.15 mL, 0.15 mmol) with a pipette, and a linear alkylthiol (0.15 mmol) and a solution of DIPEA in THF (0.35 mL, 0.5 M) were added to the corresponding reaction bottle, and reacted for 2 h under stirring at room temperature. TLC detection showed no Step I raw material.

Each Step II reaction solution was transferred to 10 1.5 mL EP tubes (each 0.1mL, 0.01 mmol) with a pipette, then a solution of a diamine in THF (0.12 mL, 0.012 mmol, 0.1M) and a solution of DIPEA in THF (0.02 mL, 0.1M) were added to the corresponding EP tube, followed by reaction for 1 h in a heated shaking reactor (Thermo-Shaker) at 78°C. TLC detection showed no Step II raw material. After the reaction was complete, the solvent in the reaction tube was evaporated to dryness at normal temperature to obtain 110 amino lipid compounds **S6SxDy.** 10 representative compounds among them were selected for measurement by mass spectrometry. The results are shown in Table 2 below.

Table 2: MW/z values of representative compounds

| | Compound No. | Structural formula | Molecular formula | Molecular weight | Found $(M+H)^+$ |
|---|---|---|---|---|---|
| 1 | **S6S11D1** | 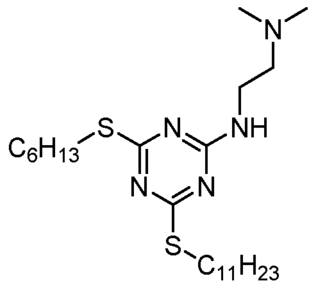 | C24H47N5 S2 | 469.3 | 470.3 |
| 2 | **S6S11D2** | 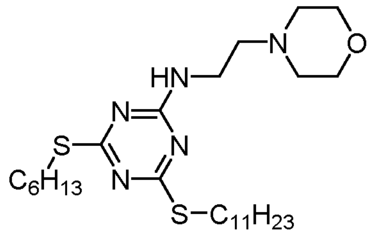 | C26H49N5 OS2 | 511.3 | 512.3 |
| 3 | **S6S11D3** | 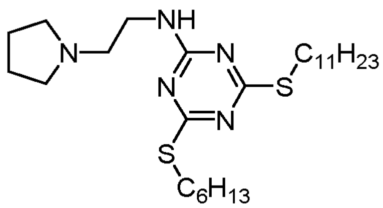 | C26H49N5 S2 | 495.3 | 496.3 |
| 4 | **S6S11D4** | 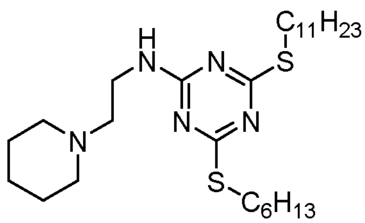 | C27H51N5 S2 | 520.4 | 521.4 |
| 5 | **S6S11D5** | 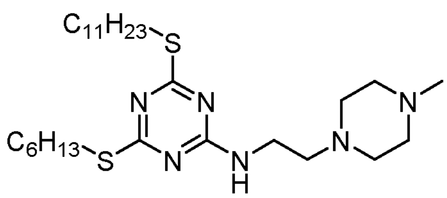 | C27H52N6 S2 | 524.4 | 525.4 |
| 6 | **S6S11D6** | 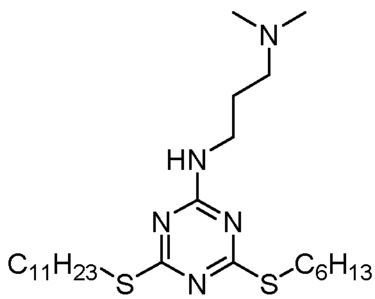 | C25H49N5 S2 | 483.3 | 484.3 |

(continued)

| | Compound No. | Structural formula | Molecular formula | Molecular weight | Found (M+H)+ |
|---|---|---|---|---|---|
| 7 | **S6S11D7** | 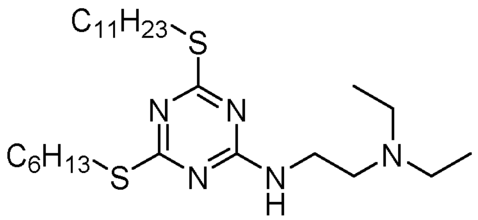 | C26H51N5 S2 | 497.4 | 498.5 |
| 8 | **S6S11D8** | 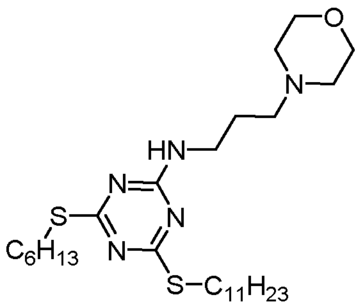 | C27H51N5 OS2 | 525.4 | 526.5 |
| 9 | **S6S11D9** | 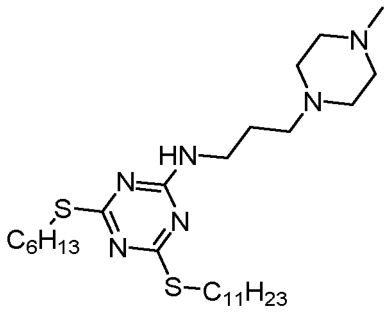 | C28H54N6 S2 | 538.4 | 539.4 |
| 10 | **S6S11D10** | 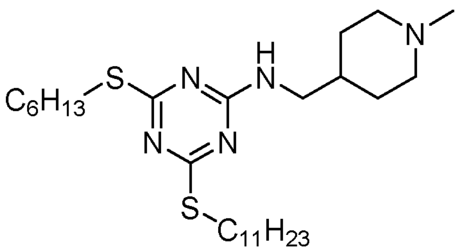 | C27H51N5 S2 | 509.4 | 510.4 |

**Example 1.2.1: One-pot synthesis and characterization of representative amino lipid compound S6S11D6**

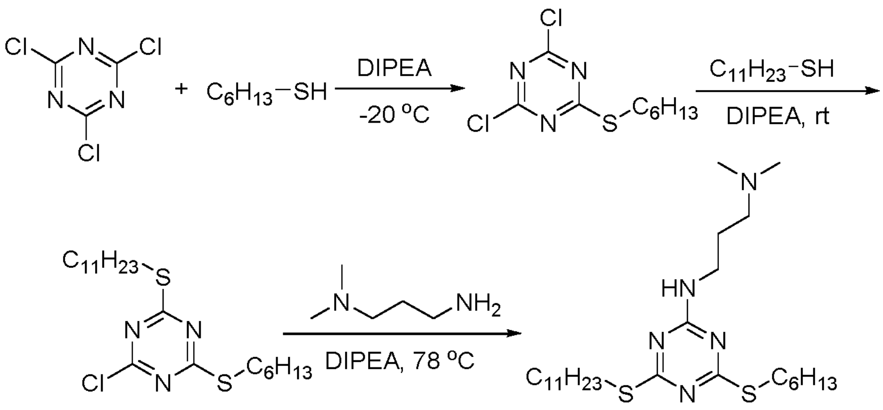


**S6S11D6**

Cyanuric chloride (1 mmol) and THF (10 mL) were added to a 20 mL dry reaction bottle and cooled to -20°C under stirring in a cold bath, followed by successive addition of 1-hexanethiol (1 mmol) and DIPEA (1.2 mmol), and a large amount of precipitate immediately occurred in the reaction solution. TLC detection was performed after reaction for 5 min, the reaction was complete. After the reaction solution was warmed to room temperature, undecylthiol (1 mmol) and DIPEA(1.2 mol) were added, and reacted for 2 h under stirring at room temperature. TLC detection showed no Step I raw material. N,N-dimethylpropanediamine (1.2 mmol) and DIPEA (2 mol) were then added, warmed to 78°C with stirring and reacted for 1 h. TLC detection showed no Step II raw material. Purification by an automatic purification system (forward silica column, eluting with a gradient of 0 to 10% DCM:MeOH) to obtain 445.1 mg of a white solid with a yield of 92% of the reactions of three steps and a purity of 99%. $^1H$ NMR (400 MHz, $CDCl_3$) $\delta$ 3.51 (m, 2H), 3.04-3.01 (m, 4H), 2.81-2.74 (m, 6H), 2.09 (m, 2H), 1.68 (m, 2H), 1.55 (m, 2H), 1.33 (m, 2H), 1.29 (m, 2H), 1.23 (m, 22H), 0.88-0.84 (m, 6H). ESI-MS calculated for $C_{25}H_{50}N_5S_2^+$ $[M+H]^+$ 484.4, found 484.4.

**Example 1.3: Parallel synthesis and characterization of the library of amino lipid compounds of N6NxDy series**

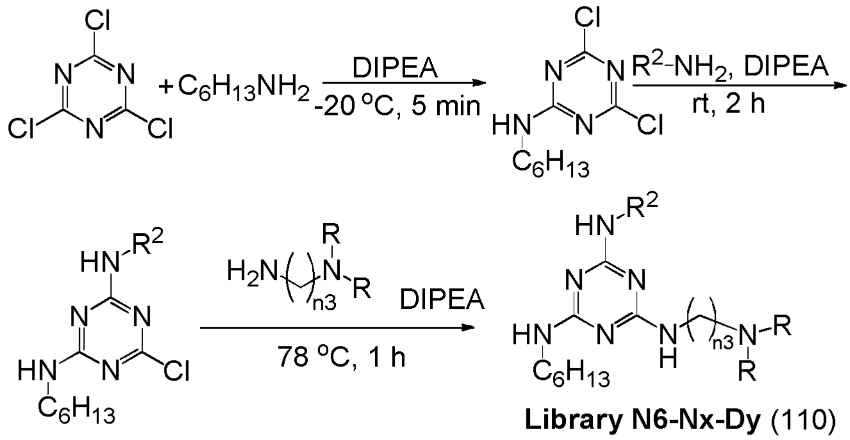


**Library N6-Nx-Dy** (110)

wherein n3, -NRR, $R^1$ and $R^2$ are as defined above.

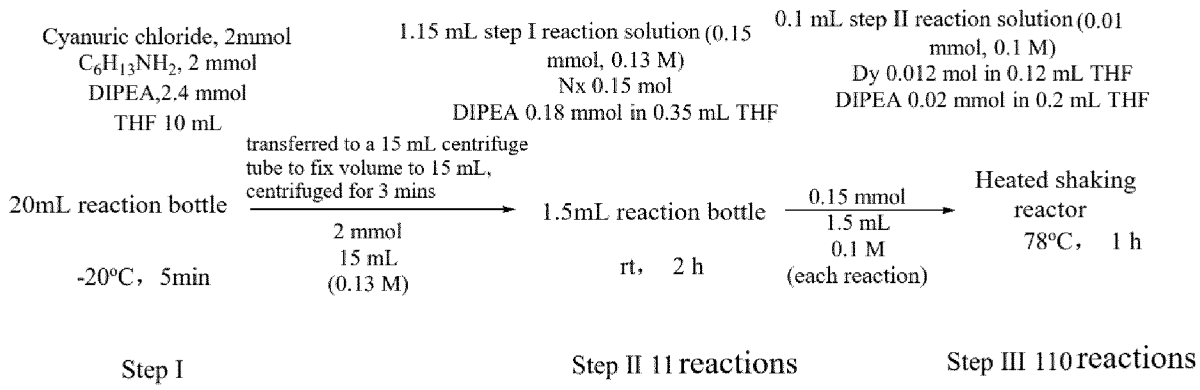


Cyanuric chloride (2 mmol) and THF (10 mL) were added to a 50 mL dry reaction tube and cooled to -20°C under stirring in a cold bath, followed by successive addition of 1-hexylamine (2 mmol) and DIPEA (2.4 mmol), and a large amount of precipitate immediately occurred in the reaction solution. TLC detection was performed after reaction for 5 min, the reaction was complete. The reaction solution was transferred to a 15 mL centrifuge tube, fixed to a volume of 15 mL, and then centrifuged in a centrifuge (2000 r/min, 1 min). Step I reaction solution (15 mL, 0.13 M) was then obtained.

The Step I reaction solution was transferred to 11 1.5 mL reaction bottles (each 1.15 mL, 0.15 mmol) with a pipette, and a linear alkylamine (0.15 mmol) and a solution of DIPEA in THF (0.35 mL, 0.5 M) were added to the corresponding reaction bottle, and reacted for 2 h under stirring at room temperature. TLC detection showed no Step I raw material.

Each Step II reaction solution was transferred to 10 1.5 mL EP tubes (each 0.1mL, 0.01 mmol) with a pipette, and a solution of a diamine in THF (0.12 mL, 0.012 mmol, 0.1M) and a solution of DIPEA in THF (0.02 mL, 0.1M) were added to the corresponding EP tube, followed by reaction for 1 h in a heated shaking reactor (Thermo-Shaker) at 78°C. TLC detection showed no Step II raw material. After the reaction was complete, the solvent in the reaction tube was evaporated to dryness at normal temperature to obtain 110 amino lipid compounds **N6NxDy.** 10 representative compounds among them were selected for measurement by mass spectrometry. The results are shown in Table 3.

Table 3: MW/z values of representative compounds

| | Compound No. | Structural formula | Molecular formula | Molecular weight | Found $(M+H)^+$ |
|---|---|---|---|---|---|
| 1 | **N6N11D1** | 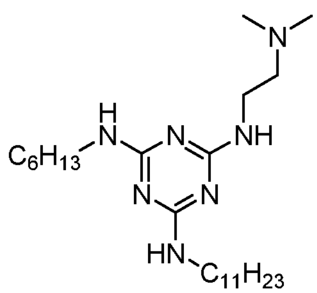 | C24H49N7 | 435.4 | 436.4 |
| 2 | **N6N11D2** | 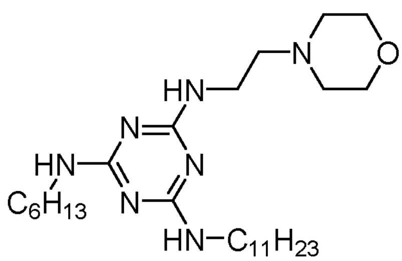 | C26H51N7O | 477.4 | 478.4 |

(continued)

| | Compound No. | Structural formula | Molecular formula | Molecular weight | Found (M+H)+ |
|---|---|---|---|---|---|
| 3 | **N6N11D3** | 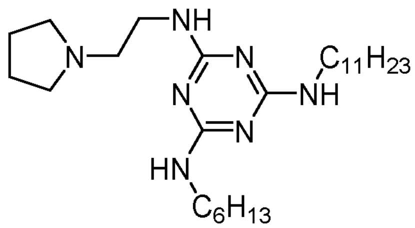 | C26H51N7 | 461.4 | 462.4 |
| 4 | **N6N11D4** | 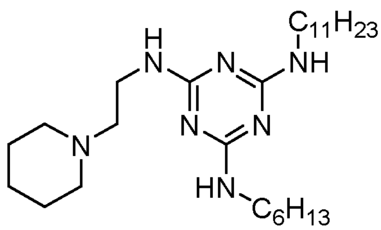 | C27H53N7 | 475.4 | 476.4 |
| 5 | **N6N11D5** | 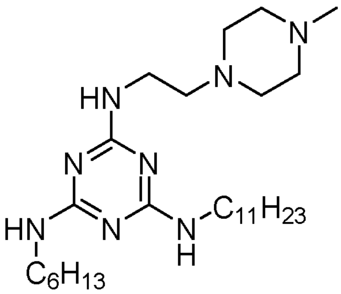 | C27H54N8 | 490.4 | 491.4 |
| 6 | **N6N11D6** | 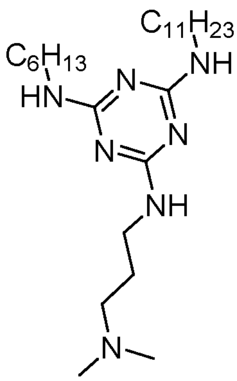 | C25H51N7 | 449.4 | 450.4 |
| 7 | **N6N11D7** | 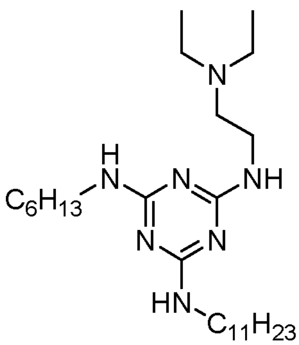 | C26H53N7 | 463.4 | 464.4 |

(continued)

| | Compound No. | Structural formula | Molecular formula | Molecular weight | Found $(M+H)^+$ |
|---|---|---|---|---|---|
| 8 | **N6N11D8** | 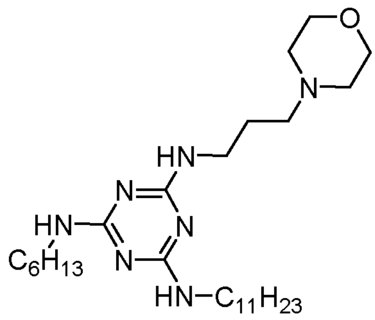 | C27H53N70 | 491.4 | 492.4 |
| 9 | **N6N11D9** | 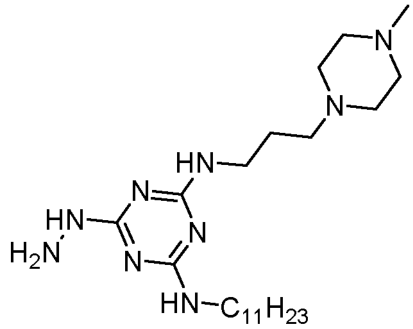 | C22H45N9 | 435.4 | 436.4 |
| 10 | **N6N11D10** | 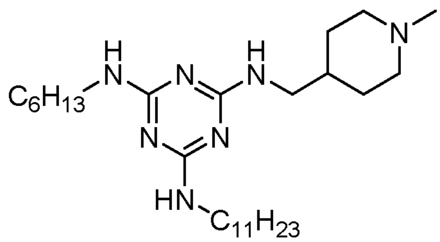 | C27H53N7 | 475.4 | 476.4 |

**Example 1.3.1: One-pot synthesis and characterization of representative amino lipid compound N6N11D6**

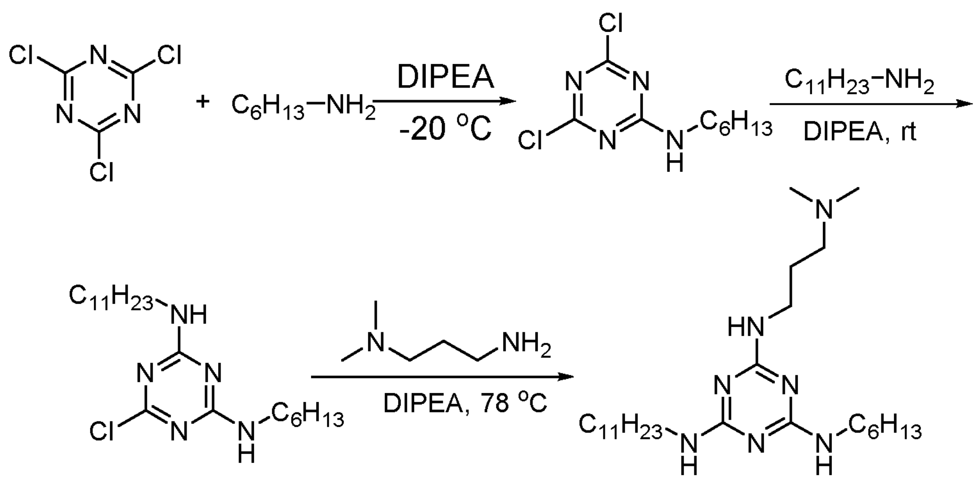


**N6N11D6**

Cyanuric chloride (1 mmol) and THF (10 mL) were added to a 20 mL dry reaction tube and cooled to -20°C under stirring in a cold bath, followed by successive addition of 1-hexylamine (1 mmol) and DIPEA (1.2 mmol), and a large amount of precipitate immediately occurred in the reaction solution. TLC detection was performed after reaction for 5 min, the reaction was complete. After the reaction solution was warmed to room temperature, undecylamine (1 mmol) and DIPEA (1.2 mol) were added, and reacted for 2 h under stirring at room temperature. TLC detection showed no Step I raw material. N,N-dimethylpropanediamine (1.2 mmol) and DIPEA (2 mol) were then added, warmed to 78°C with stirring and reacted for 1 h. TLC detection showed no Step II raw material. Purification by an automatic purification system (forward silica column, eluting with a gradient of 0 to 10% DCM:MeOH) to obtain 395.7 mg of a white solid, with a yield of 88% for the reactions of three steps and a purity of 99%. $^1$H NMR (400 MHz, $CDCl_3$) $\delta$3.54 (m, 2H), 3.05-3.02 (m, 4H), 2.82-2.73 (m, 6H), 2.08 (m, 2H), 1.69 (m, 2H), 1.54 (m, 2H), 1.34 (m, 2H), 1.30 (m, 2H), 1.24 (m, 22H), 0.89-0.85 (m, 6H). ESI-MS calculated for $C_{25}H_{52}N_7^+$ [M+H]$^+$ 450.4, found 450.4.

**Example 1.4: Parallel synthesis and characterization of the library of amino lipid compounds of S6SxOy series**

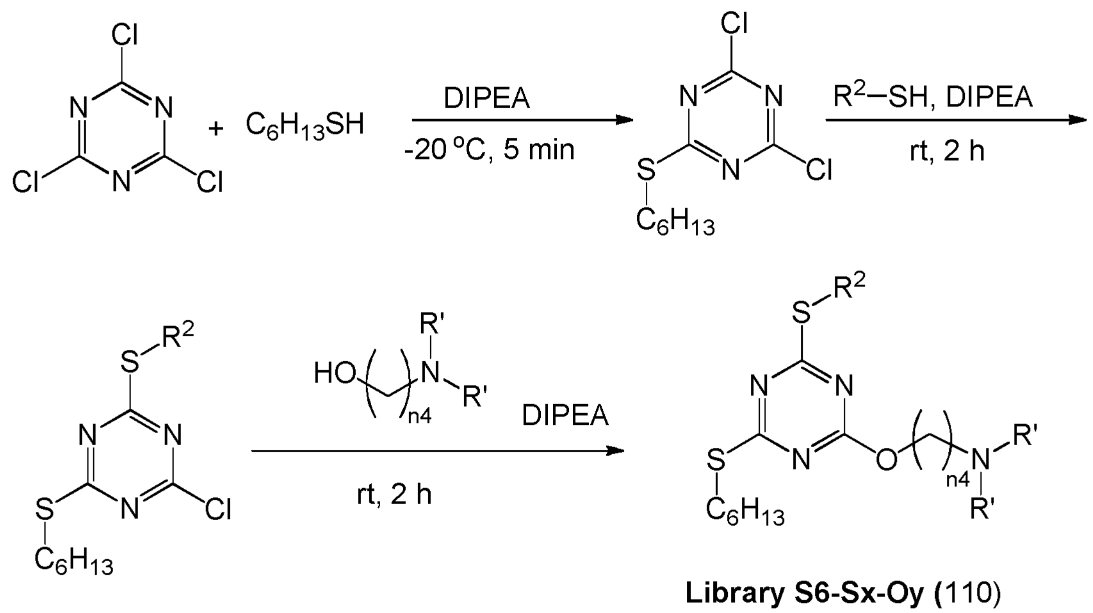


**Library S6-Sx-Oy (**110)

wherein n4=1, 2, 3 or 4; -NR'R' represents a dialkylamino or cyclic amino moiety in O1 to O10 described above; and $R^1$ and $R^2$ are as defined above.

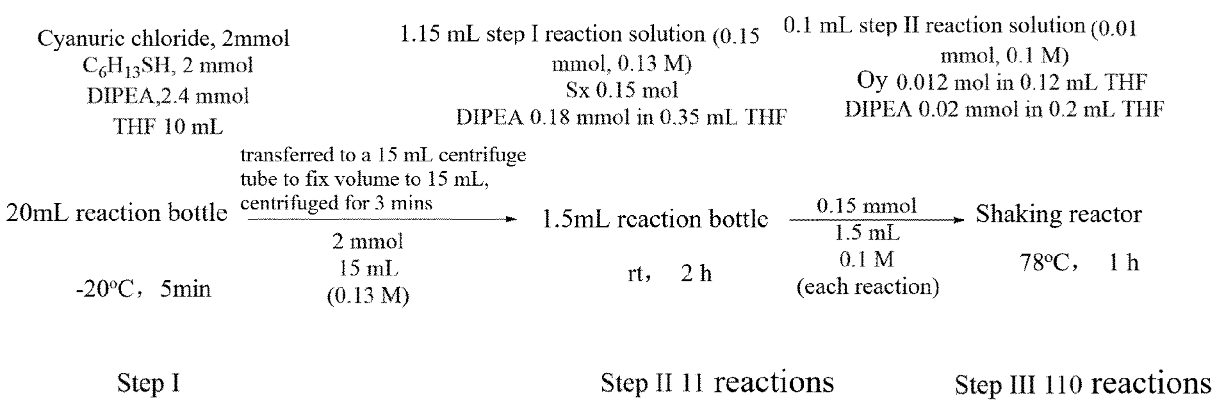


Cyanuric chloride (2 mmol) and THF (10 mL) were added to a 50 mL dry reaction bottle and cooled to -20°C under stirring in a cold bath, followed by successive addition of 1-hexanethiol (2 mmol) and DIPEA (2.4 mmol), and a large amount of precipitate immediately occurred in the reaction solution. TLC detection was performed after reaction for 5 min, the reaction was complete. The reaction solution was transferred to a 15 mL centrifuge tube, fixed to a volume

of 15 mL and then centrifuged in a centrifuge (2000 r/min, 1 min). Step I reaction solution (15 mL, 0.13 M) was then obtained.

The Step I reaction solution was transferred to 11 1.5 mL reaction bottles (each 1.15 mL, 0.15 mmol) with a pipette, and a linear hydrocarbylthiol (0.15 mmol) and a solution of DIPEA in THF (0.35 mL, 0.5 M) were added to the corresponding reaction bottle, and reacted for 2 h under stirring at room temperature. TLC detection showed no Step I raw material

Each Step II reaction solution was transferred to 10 1.5 mL EP tubes (each 0.1mL, 0.01 mmol) with a pipette, and a solution of a hydroxylamine in THF (0.12 mL, 0.012 mmol, 0.1M) and a solution of DIPEA in THF (0.02 mL, 0.1M) were added to the corresponding EP tube, followed reaction for 2 h in a shaking reactor (Thermo-Shaker) at room temperature. TLC detection showed no Step II raw material. After the reaction was complete, the solvent in the reaction tube was evaporated to dryness at normal temperature to obtain 110 amino lipid compounds **S6SxOy.** 10 representative compounds among them were selected for measurement by mass spectrometry. The results are shown in Table 1.4 below.

Table 1.4: MW/z values of representative compounds

| | Compound No. | Structural formula | Molecular formula | Molecular weight | Found $(M+H)^+$ |
|---|---|---|---|---|---|
| 1 | **S19S9O1** | 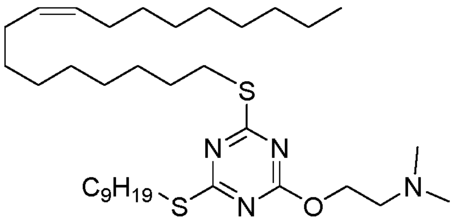 | C34H64N4 OS2 | 608.5 | 609.8 |
| 2 | **S19S9O2** | 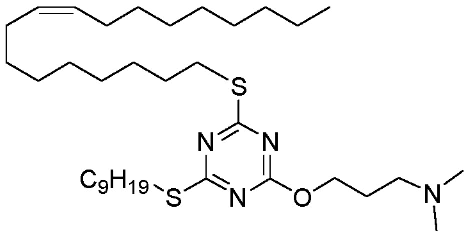 | C35H66N4 OS2 | 622.5 | 623.7 |
| 3 | **S19S9O3** | 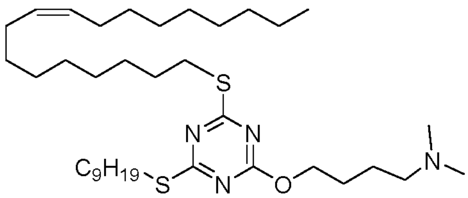 | C36H68N4 OS2 | 636.5 | 637.9 |
| 4 | **S19S9O4** | 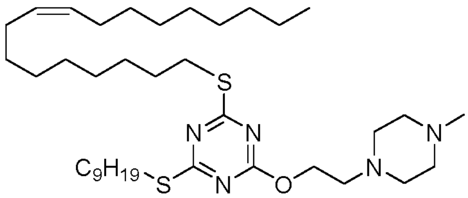 | C37H69N5 OS2 | 663.5 | 664.7 |
| 5 | **S19S9O5** | 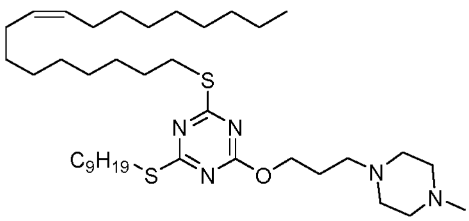 | C38H71N5 OS2 | 677.5 | 678.9 |

(continued)

| | Compound No. | Structural formula | Molecular formula | Molecular weight | Found (M+H)+ |
|---|---|---|---|---|---|
| 6 | **S19S9O6** | 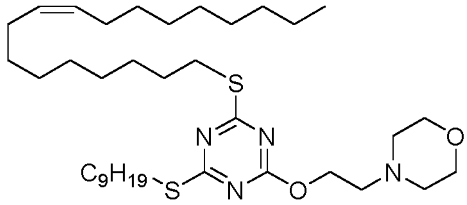 | C36H66N4 O2S2 | 650.5 | 651.8 |
| 7 | **S19S9O7** | 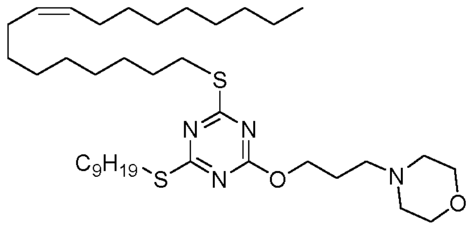 | C37H68N4 O2S2 | 664.5 | 665.8 |
| 8 | **S19S9O8** | 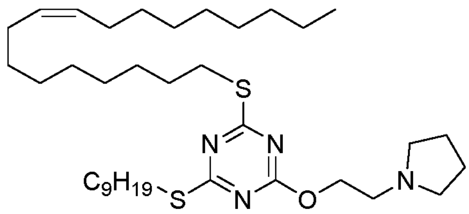 | C36H66N4 OS2 | 634.5 | 635.7 |
| 9 | **S19S9O9** | 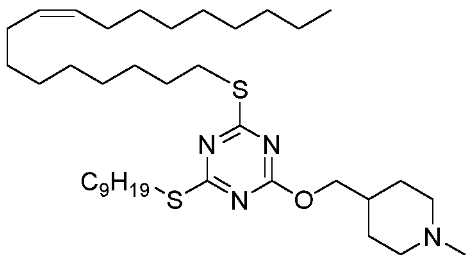 | C37H68N4 OS2 | 648.5 | 649.8 |
| 10 | **S19S9O10** | 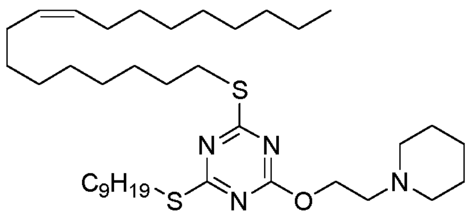 | C37H68N4 OS2 | 648.5 | 649.7 |

**Example 1.4.1: One-pot synthesis and characterization of representative amino lipid compound S19S9O8**

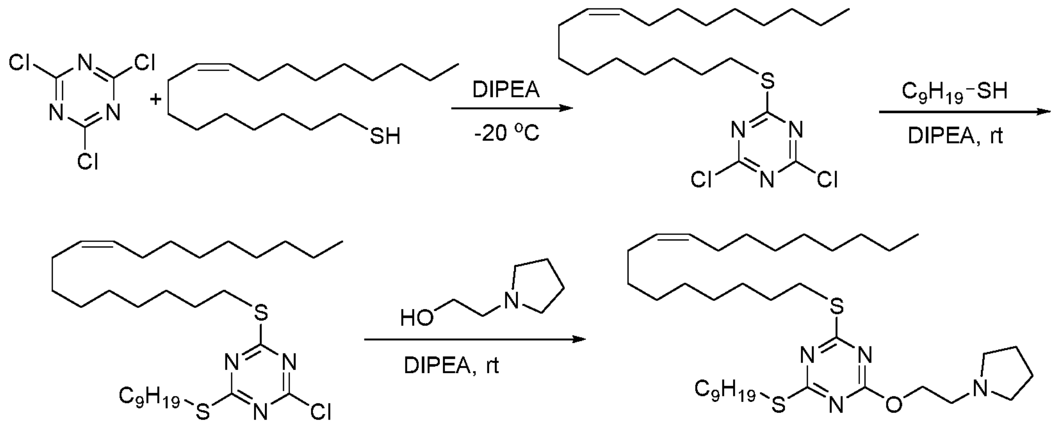


**S19S9O8**

Cyanuric chloride (1 mmol) and THF (10 mL) were added to a 20 mL dry reaction bottle and cooled to -20°C under stirring in a cold bath, followed by successive addition of 1-oleylthiol (1 mmol) and DIPEA (1.2 mmol), and a large amount of precipitate immediately occurred in the reaction solution. TLC detection was performed after reaction for 5 min, the reaction was complete. After the reaction solution was warmed to room temperature, 1-nonylthiol (1 mmol) and DIPEA (1.2 mol) were added, and reacted for 2 h under stirring at room temperature. TLC detection showed no Step I raw material. N-(2-hydroxyethyl)-pyrrolidine (1.2 mmol) and DIPEA (2 mol) were then added, and reacted for 2 h at room temperature. TLC detection showed no Step II raw material. Purification by an automatic purification system (forward silica column, eluting with a gradient of 0 to 10% DCM:MeOH) to obtain 546.2 mg of a white solid, with a yield of 86% for the reactions of three steps and a the purity of 99%. $^1$H NMR (400 MHz, $CDCl_3$) $\delta$ 5.35 (m, 2H), 4.21 (m, 2H), 3.51 (m, 4H), 2.67-2.54 (m, 6H), 2.19 (m, 4H), 1.68 (m, 4H), 1.55-1.23 (m, 38H), 0.88-0.84 (m, 6H). ESI-MS calculated for $C_{36}H_{67}N_4OS_2^+$ $[M+H]^+$ 635.5, found 635.7.

**Example 2: Evaluation on the performances of lipid nanoparticles prepared from amino lipid compounds in *in vivo* delivery of luciferase mRNA**

**1. Preparation of lipid nanoparticles**

**Formulation method 1:**

The amino lipid compound of the invention was mixed with DOPE, cholesterol and PEG2000-DMG in a molar ratio of 45: 10: 42.5: 2.5, and dissolved in absolute ethanol such that the molar concentration of the amino lipid compound was 0.001-0.01 mmol/L. By using a microinjection pump, the resultant ethanol solution and a sodium acetate solution (50 mM, pH = 4.0) in which luciferase mRNA (Fluc mRNA, TriLink) was dissolved were mixed in a volume ratio of 1:3 in a microchannel chip to prepare a crude solution of lipid nanoparticles, which was then dialyzed in 1X PBS at a controlled temperature of 4°C for 6 h using a dialysis box (Fisher, MWCO 20,000) and filtered through a 0.22 μm microporous filter membrane before use. The mass ratio of the amino lipid compound to Fluc mRNA was about 10: 1. The resultant solution of lipid nanoparticles (LNPs) was administered to the tested animals by subcutaneous administration.

Characterization of the lipid nanoparticles:

Characterization of particle size: the particle size and PDI of the prepared lipid nanoparticles were determined by Nano-ZSZEN3600 (Malvern). 40 uL of LNP solution was taken for particle size measurement with circulation for three times, 30 s for each circulation.

Detection of encapsulation rate: the concentration of LNP RNA was detected by Qubit® RNA HS Assay kit. The theoretical RNA concentration was given by the total amount of RNA input divided by the total volume of the final solution.

$$\text{Encapsulation rate} = \frac{\text{Theoretical RNA concentration - Free RNA concentration}}{\text{Theoretical RNA concentration}} \times 100\%$$

Table 4: Characterization data of LNPs prepared from representative amino lipid compounds using Formulation Method 1.

| | Amino lipid No. | Structure | Particle size (nm) | PDI | Encapsulation rate |
|---|---|---|---|---|---|
| 1 | S16N9D3 | 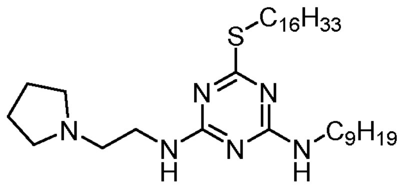 | 87±4 | 0.08 | 98.9% |
| 2 | S15N11D5 | 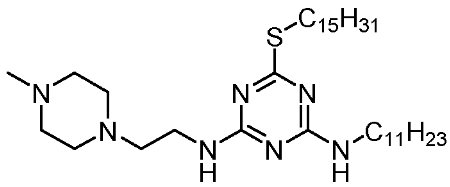 | 79±5 | 0.02 | 99.2% |
| 3 | S12N13D6 | 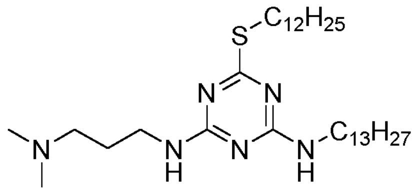 | 89±5 | 0.05 | 99.0% |
| 4 | S16N7D9 | 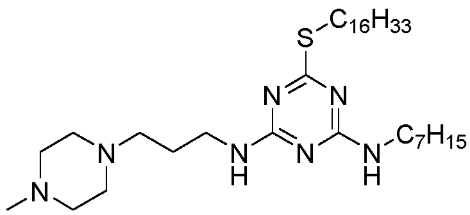 | 102±5 | 0.01 | 98.6% |
| 5 | S16N8D10 | 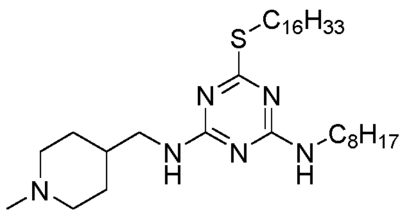 | 90±5 | 0.04 | 97.2% |
| 6 | S7S18D3 | 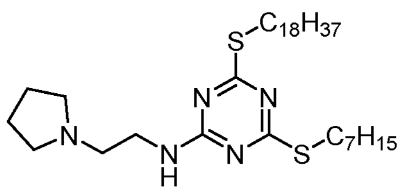 | 121±5 | 0.10 | 99.5% |
| 7 | S9S16D5 | 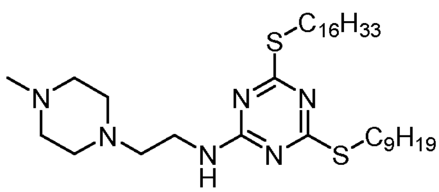 | 152±5 | 0.04 | 99.1% |

(continued)

| | Amino lipid No. | Structure | Particle size (nm) | PDI | Encapsulation rate |
|---|---|---|---|---|---|
| 8 | S11S12D8 | 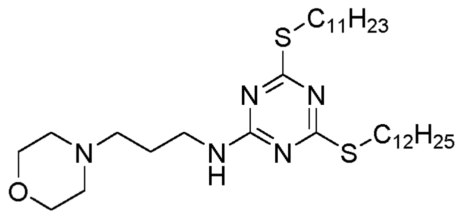 | 80±5 | 0.02 | 98.3% |
| 9 | S8S16D9 | 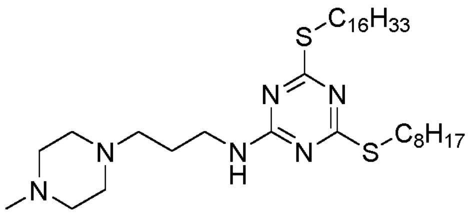 | 96±5 | 0.01 | 98.7% |
| 10 | S10S16D10 | 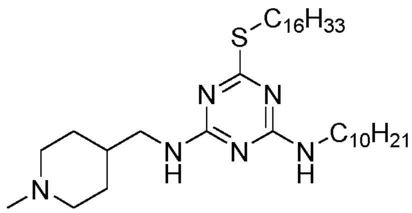 | 136±5 | 0.03 | 99.5% |
| 11 | S19S9O8 | 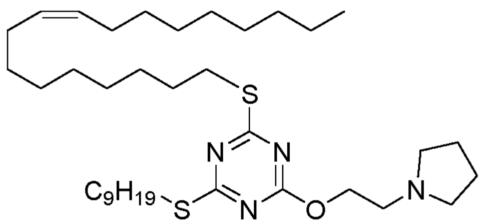 | 88±5 | 0.02 | 99.6% |
| 12 | **S19S9O9** | 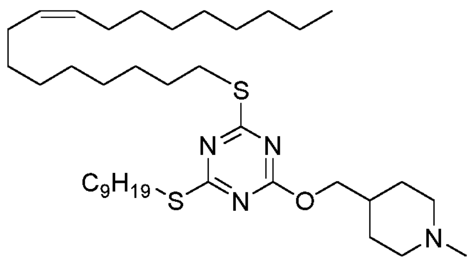 | 110±5 | 0.04 | 99.2% |

**Formulation method 2:**

The preparation method was the same as Formulation method 1, except that amino lipid compound, DSPC, cholesterol and PEG2000-DMG were used in a molar ratio of 50: 10: 38.5: 1.5. The resultant solution of lipid nanoparticles (LNPs) was administered to the tested animals by tail vein and intramuscular injection.

Table 5: Characterization data of LNPs prepared from representative amino lipid compounds using Formulation method 2.

| | Amino lipid No. | Particle size (nm) | PDI | Encapsulation rate |
|---|---|---|---|---|
| 1 | S16N9D3 | 127±5 | 0.06 | 99.1% |
| 2 | S15N11D5 | 95±5 | 0.01 | 98.8% |

(continued)

| | Amino lipid No. | Particle size (nm) | PDI | Encapsulation rate |
|---|---|---|---|---|
| 3 | S12N13D6 | 96±5 | 0.04 | 99.2% |
| 4 | S16N7D9 | 92±5 | 0.05 | 99.2% |
| 5 | S16N8D10 | 120±5 | 0.03 | 98.6% |
| 6 | S7S18D3 | 95±5 | 0.04 | 99.3% |
| 7 | S9S16D5 | 130±5 | 0.05 | 99.6% |
| 8 | S11S12D8 | 90±5 | 0.01 | 98.9% |
| 9 | S8S16D9 | 115±5 | 0.02 | 99.4% |
| 10 | S10S16D10 | 125±5 | 0.04 | 99.2% |
| 11 | S19S9O8 | 88±5 | 0.03 | 99.2% |
| 12 | S19S9O9 | 110±5 | 0.05 | 99.0% |

## 2. Animal tests

Animal Preparation: 6-week aged female BALB/c mice weighed about 20 g were selected and fed in a SPF-grade feeding room. Animal tests were conducted strictly according to the guidelines of national health institutions and the requirements of animal ethics.

*In vivo* delivery: 9 mice were assigned randomly to each group, and the lipid nanoparticle solution was injected by three routes of administration, i.e., subcutaneous, intramuscular and tail vein injection at a dosage of 0.5 mg/kg Fluc mRNA (3 mice for each route of administration). After 12 hours, 200 μL of 10 mg/mL potassium salt of D-fluorescein (PerkinElmer) was injected into each mouse through the tail vein. After 10 min, the mice were placed under an *in vivo* imaging system (IVIS-200, Xenogen) to observe the total fluorescence intensity of each mouse and take photographs for record. The expression intensity of Fluc mRNA delivered by representative amino lipid compounds through the three routes of administration are shown in Table 5-7. DLin-MC3 was used as control.

Table 5: Expression intensity of Fluc mRNA delivered by subcutaneous administration of representative amino lipid compounds.

| Amino lipid No. | Fluorescence intensity |
|---|---|
| S 16N9D3 | 2.7E+07 |
| S15N11D5 | 1.9E+08 |
| S12N13D6 | 8.6E+06 |
| S 16N7D9 | 1.4E+07 |
| CD10 | 3.7E+08 |
| S7S18D3 | 2.9E+08 |
| S9S16D5 | 4.2E+07 |
| S11S12D8 | 1.1E+07 |
| S8S16D9 | 4.2E+06 |
| S10S16D10 | 4.7E+07 |
| S19S9O8 | 1.1E+07 |
| S19S9O9 | 2.8E+07 |
| DLin-MC3 (control) | 3.1E+06 |

Table 6: Expression intensity of Fluc mRNA delivered by intramuscular administration of representative amino lipid compounds.

| No. | Fluorescence intensity |
|---|---|
| S16N9D3 | 4.3E+06 |
| S15N11D5 | 3.7E+06 |
| S12N13D6 | 1.2E+06 |
| S16N7D9 | 4.3E+07 |
| S16N8D10 | 3.9E+06 |
| S7S18D3 | 8.7E+07 |
| S9S16D5 | 5.2E+06 |
| S11S12D8 | 4.7E+06 |
| S8S16D9 | 8.2E+06 |
| S10S16D10 | 3.2E+06 |
| S19S9O8 | 9.1E+07 |
| S19S9O9 | 1.8E+07 |
| DLin-MC3 (control) | 2.7E+07 |

Table 7: Expression intensity of Fluc mRNA delivered by tail vein administration of representative amino lipid compounds.

| No. | Fluorescence intensity |
|---|---|
| S16N9D3 | 5.3E+06 |
| S15N11D5 | 3.7E+06 |
| S12N13D6 | 3.9E+06 |
| S16N7D9 | 5.1E+07 |
| S16N8D10 | 3.2E+06 |
| S7S18D3 | 8.1E+07 |
| S9S16D5 | 7.5E+06 |
| S11S12D8 | 3.8E+06 |
| S8S16D9 | 6.5E+06 |
| S10S16D10 | 2.9E+06 |
| S19S9O8 | 1.1E+08 |
| S19S9O9 | 3.1E+07 |
| DLin-MC3 (control) | 2.7E+07 |

**Example 3: Evaluation on the performances of lipid-nanoparticles prepared from amino lipid compounds in *in vivo* delivery of ovalbumin mRNA and immunity**

Formulation method:
The amino lipid compound of the invention was mixed with DOPE, cholesterol and PEG2000-DMG in a molar ratio of 50: 10: 38.5: 1.5 and dissolved in absolute ethanol such that the molar concentration of the amino lipid compound was 0.001-0.01 mmol/L. Ovalbumin mRNA (OVA mRNA, TriLink) was dissolved in sodium acetate solution (50 mM, pH = 4.0). By using a microinjection pump, the resultant ethanol solution and sodium acetate solution (50 mM, pH = 4.0) were

mixed in a volume ratio of 1:3 in a microchannel chip to prepare a crude solution of lipid nanoparticles, which was then dialyzed in 1X PBS at a controlled temperature of 4°C for 6 h using a dialysis box (Fisher, MWCO 20,000), and filtered through a 0.22 μm microporous filter membrane before use. The mass ratio of the amino lipid compound to ovalbumin mRNA (OVA mRNA) was about 10:1.

Animal Preparation: 6-week aged female BALB/c mice weighed about 20 g were selected and fed in a SPF-grade feeding room. Animal tests were conducted strictly according to the guidelines of national health institutions and the requirements of animal ethics.

*In vivo* delivery: 3 mice were assigned randomly to each group, and the lipid nanoparticle solution was intramuscularly injected into the leg at a dosage of 0.5 mg/kg mRNA (Day 0). After 7 days, one booster injection was performed at the same dosage (Day 7). On Day 21, blood was taken from the tail vein for serological analysis. DLin-MC3 was used as control (Figure 1).

Enzyme-linked immunosorbent assay (ELISA): a flat-bottomed 96-well plate (Nunc) was pre-coated, with a concentration of OVA protein of 0.5 μg protein per well in 50 mM carbonate buffer (pH 9.6), and was allowed to stay overnight at 4°C and then blocked with 5% glycine. Serum obtained from animals receiving the lipid nanoparticle solution containing the mRNA was diluted from $10^2$ to $10^6$ times with PBS-0.05% Tween (PBS-T) at pH 7.4, and added to the wells, incubated at room temperature and placed at 37°C for 1 h. Horseradish peroxidase (HRP, Invitrogen) coupled goat anti-mouse IgG was used for labeling in PBS-T-1% BSA at a dilution rate of 1:10,000. After addition of the HRP substrate, the absorbance at 450 nm was measured in ELISA microplate reader (Bio-Rad). As shown in Figure 1, the IgG antibody titers for S7S18D3, S16N7D9, S19S9O8 and S19S9O9 were significantly superior to that of the control.

**Example 4: Preliminary screening of amino lipid compounds as DNA vectors**

Cell line: HEK293 cells (ATCC CRL-1573™)
Medium: DMEM supplemented with 10% fetal bovine serum (Invitrogen)
Mode of screening: cell transfection on 96-well plates
Detection (readout): percentage of the number of GFP fluorescent cells relative to the total number of cells (the total number of cells was measured using nucleus dye Hoechst - see Figure 2). Lipofectamine2000 (Invitrogen) was used as positive control, according to the manufacturer's instructions.
Method: an 8-channel pipette was used for sample addition. The contents shown were for a single well of a 96-well flat plate.

1. The compound (0.01 mmol) prepared in Example 1 was dissolved in 1 mL of anhydrous ethanol. After ultrasonically dissolved, 10 μL of the resultant amino lipid solution in ethanol was taken and mixed with 5 μL of DOPE solution in ethanol (0.01 m), and then to the obtained mixture was added 35 μL of 0.2M sodium acetate buffer (pH 5.6), and 50 μL of liposome solution was prepared by vortex at a constant rotating speed for 30s. 4 μL was taken from the 50 μL liposome solution and added to 46 μL of 0.02M sodium acetate buffer (pH 5.6), and the final liposome solution was formed by vortex at a constant rotating speed for 30s. 5 μL was taken from the final liposome solution and mixed with 75 ng plasmid DNA (psin-EGFP-IRES-Puro, Clontech) dissolved in 5 μL of 0.02 M sodium acetate buffer, and was allowed to stay at room temperature for 30 min to form a lipid/DNA transfection complex.
2. 10 μL of the lipid/DNA transfection complex was incubated at room temperature for 30 min, followed by addition of 90 μL of fresh resuspended cells ($3\text{-}5\times 10^4$ cells) and mixing with a pipette. 100 μL of cells + lipid /DNA complex was transferred to separate wells of a 96-well culture plate immediately, and placed in an incubator containing 5% $CO_2$ at 37°C.
3. Hoechst33258 (Invitrogen) was added at a final concentration of 0.2 μg/mL to the cells 20 to 24 hours after initial cell transfection, and cultured in darkness at 37°C for 15 min. Afterwards, the cells were washed once with a PBS solution, and then added with medium for culture for 20 to 24 h.
4. The cells were placed in a high-throughput confocal microscope (Molecular Devices ImageXpress), and four visual fields of the image of the cells were captured for each well, and images at three laser wavelengths were captured for each sample: bright field images of cells (Fig. 2(a) and Fig. 2(b)), Hoechst-staining images showing total nuclei (Fig. 2(c) and Fig. 2(d)) and GFP images showing success in transfection with the plasmid DNA and the expression of GFP (Fig. 2(e) and Fig. 2(f)). MetaXpress software was used to separately count the cells in the Hoechst-staining images and GFP images, and then the number of cells expressing GFP was divided by the total number of nuclei to give the absolute cell transfection efficiency. The absolute transfection efficiency was calculated as follows:

$$\text{Absolute transfection efficiency (\%)} = \frac{\text{Number of cells expressing GFP}}{\text{Number of Hoechst-stained cells}}$$

Results: The DNA transfection efficiency in HEK293 cells for 2530 compounds is shown in Table 8.

Table 8: Absolute DNA transfection efficiency in HEK293 cells for 2530 compounds.

| | D1 | D2 | D3 | D4 | D5 | D6 | D7 | D8 | D9 | D10 |
|---|---|---|---|---|---|---|---|---|---|---|
| S6N6 | 0.1% | 0.0% | 0.0% | 0.0% | 0.0% | 0.0% | 0.0% | 0.0% | 0.0% | 0.0% |
| S6N7 | 0.1% | 0.0% | 0.0% | 1.1% | 3.7% | 0.4% | 0.1% | 0.0% | 0.2% | 2.9% |
| S6N8 | 0.2% | 0.3% | 2.0% | 1.7% | 3.4% | 0.6% | 0.9% | 0.0% | 1.2% | 0.4% |
| S6N9 | 0.1% | 0.2% | 0.4% | 0.4% | 7.9% | 1.5% | 1.1% | 0.0% | 0.8% | 4.3% |
| S6N11 | 1.5% | 0.2% | 2.8% | 3.3% | 17.5% | 1.9% | 2.2% | 1.5% | 21.1% | 7.9% |
| S6N12 | 4.7% | 0.9% | 6.2% | 2.0% | 25.9% | 6.0% | 2.2% | 4.0% | 38.8% | 21.3% |
| S6N13 | 6.5% | 1.9% | 9.9% | 2.1% | 24.1% | 12.9% | 2.0% | 4.9% | 38.3% | 51.6% |
| S6N15 | 5.8% | 2.0% | 15.9% | 1.7% | 22.3% | 25.9% | 1.2% | 3.3% | 28.4% | 31.1% |
| S6N16 | 6.6% | 1.4% | 19.8% | 2.4% | 19.6% | 22.8% | 2.6% | 4.2% | 20.6% | 19.7% |
| S6N18 | 21.0% | 2.5% | 21.9% | 3.5% | 15.4% | 21.8% | 5.2% | 8.1% | 14.1% | 13.0% |
| $S6^2N12$ | 0.4% | 0.0% | 0.1% | 0.1% | 0.0% | 0.2% | 0.3% | 0.0% | 0.4% | 0.0% |
| S7N6 | 17.6% | 1.8% | 34.7% | 8.7% | 28.5% | 27.2% | 11.2% | 4.7% | 20.3% | 6.3% |
| S7N7 | 23.6% | 2.5% | 9.2% | 11.3% | 33.5% | 33.5% | 11.4% | 5.3% | 10.9% | 8.6% |
| S7N8 | 15.6% | 2.7% | 9.3% | 15.5% | 22.1% | 22.3% | 26.8% | 2.7% | 14.9% | 9.5% |
| S7N9 | 28.8% | 13.9% | 12.3% | 13.0% | 22.0% | 25.9% | 33.2% | 4.6% | 22.8% | 15.0% |
| S7N11 | 20.3% | 31.1% | 25.3% | 6.9% | 43.7% | 42.1% | 9.6% | 13.9% | 43.9% | 45.9% |
| S7N12 | 18.2% | 25.6% | 17.4% | 9.7% | 35.1% | 54.6% | 5.5% | 17.7% | 46.4% | 45.7% |
| S7N13 | 11.7% | 22.1% | 17.9% | 6.4% | 23.3% | 43.2% | 3.1% | 17.3% | 36.9% | 54.2% |
| S7N15 | 19.5% | 14.1% | 31.4% | 4.9% | 20.4% | 15.9% | 7.2% | 7.9% | 17.6% | 30.9% |
| S7N16 | 20.0% | 12.9% | 21.2% | 2.9% | 18.2% | 26.2% | 6.4% | 8.1% | 12.9% | 15.8% |
| S7N18 | 10.6% | 16.6% | 16.1% | 3.2% | 8.2% | 18.4% | 3.9% | 5.9% | 4.2% | 16.3% |
| $S7^2N12$ | 0.2% | 0.0% | 0.1% | 0.0% | 0.2% | 0.6% | 0.2% | 0.0% | 0.3% | 0.5% |
| S8N6 | 3.4% | 0.2% | 3.2% | 2.6% | 5.0% | 4.3% | 1.8% | 0.0% | 2.2% | 0.5% |
| S8N7 | 3.0% | 0.4% | 3.9% | 3.8% | 3.6% | 8.5% | 1.4% | 0.8% | 3.6% | 1.2% |
| S8N8 | 9.3% | 1.5% | 8.5% | 2.9% | 9.1% | 8.7% | 5.2% | 2.9% | 7.4% | 6.1% |
| S8N9 | 33.6% | 10.7% | 32.7% | 15.5% | 35.9% | 33.3% | 26.0% | 14.8% | 32.1% | 36.3% |
| S8N11 | 42.2% | 25.4% | 52.0% | 14.3% | 52.7% | 99.4% | 24.6% | 13.7% | 63.6% | 46.3% |
| S8N12 | 22.1% | 38.1% | 17.8% | 7.6% | 41.7% | 78.3% | 9.5% | 16.1% | 48.3% | 44.5% |
| S8N13 | 18.3% | 19.7% | 30.5% | 3.8% | 28.3% | 55.1% | 8.9% | 13.3% | 27.9% | 18.7% |
| S8N15 | 8.0% | 4.9% | 9.9% | 0.9% | 10.4% | 23.7% | 2.2% | 7.7% | 12.4% | 22.6% |
| S8N16 | 7.1% | 2.9% | 3.3% | 1.0% | 14.3% | 3.7% | 5.2% | 8.2% | 9.7% | 13.4% |
| S8N18 | 4.8% | 2.6% | 6.7% | 0.9% | 8.6% | 20.6% | 4.1% | 5.1% | 5.3% | 9.6% |
| $S8^2N12$ | 0.0% | 0.0% | 0.1% | 0.0% | 0.8% | 0.5% | 0.1% | 0.0% | 0.7% | 0.8% |

(continued)

| | D1 | D2 | D3 | D4 | D5 | D6 | D7 | D8 | D9 | D10 |
|---|---|---|---|---|---|---|---|---|---|---|
| S9N6 | 16.1% | 2.1% | 27.0% | 20.7% | 22.4% | 8.8% | 9.8% | 7.8% | 11.0% | 9.7% |
| S9N7 | 36.0% | 4.4% | 27.3% | 21.9% | 18.3% | 12.4% | 15.2% | 6.1% | 12.1% | 9.3% |
| S9N8 | 37.3% | 13.9% | 37.6% | 27.3% | 30.3% | 17.9% | 15.7% | 12.2% | 25.7% | 17.2% |
| S9N9 | 35.3% | 24.0% | 54.9% | 18.0% | 47.4% | 60.6% | 49.8% | 14.8% | 50.7% | 34.5% |
| S9N11 | 35.3% | 16.2% | 43.4% | 8.3% | 45.0% | 55.4% | 15.7% | 26.8% | 49.3% | 42.5% |
| S9N12 | 39.3% | 13.0% | 47.3% | 8.0% | 48.2% | 70.5% | 12.4% | 13.6% | 28.8% | 25.8% |
| S9N13 | 25.0% | 11.1% | 31.6% | 6.7% | 27.9% | 31.8% | 6.5% | 11.3% | 19.9% | 29.4% |
| S9N15 | 29.8% | 7.3% | 22.7% | 4.2% | 16.1% | 13.4% | 4.6% | 5.3% | 10.9% | 19.0% |
| S9N16 | 19.2% | 23.5% | 31.3% | 8.1% | 21.7% | 29.7% | 6.4% | 18.4% | 20.3% | 21.8% |
| S9N18 | 24.2% | 10.2% | 33.8% | 9.0% | 22.9% | 47.5% | 6.0% | 26.1% | 15.8% | 11.2% |
| $S9^2N12$ | 0.3% | 0.0% | 0.0% | 0.0% | 0.3% | 1.1% | 0.2% | 0.0% | 0.6% | 1.1% |
| S10N6 | 6.9% | 0.5% | 7.3% | 5.7% | 7.0% | 3.5% | 6.6% | 0.6% | 5.5% | 0.9% |
| S10N7 | 8.2% | 2.4% | 7.4% | 3.6% | 7.5% | 6.2% | 8.1% | 2.6% | 4.5% | 1.1% |
| S10N8 | 7.1% | 3.7% | 3.9% | 2.1% | 13.2% | 10.7% | 2.0% | 3.4% | 5.6% | 0.6% |
| S10N9 | 7.1% | 3.7% | 4.9% | 2.7% | 11.9% | 21.0% | 6.9% | 3.4% | 12.0% | 4.5% |
| S10N11 | 4.8% | 1.5% | 12.6% | 8.7% | 7.2% | 26.5% | 5.0% | 3.8% | 10.8% | 5.4% |
| S10N12 | 1.8% | 3.7% | 7.2% | 4.7% | 2.6% | 19.8% | 5.9% | 5.3% | 3.6% | 6.8% |
| S10N13 | 6.1% | 4.2% | 7.7% | 4.2% | 2.1% | 7.9% | 4.4% | 5.7% | 0.4% | 3.2% |
| S10N15 | 4.8% | 1.4% | 11.5% | 4.1% | 3.2% | 9.1% | 1.7% | 5.1% | 4.1% | 6.9% |
| S10N16 | 7.8% | 1.2% | 8.8% | 4.4% | 3.1% | 13.6% | 4.8% | 3.0% | 5.0% | 8.7% |
| S10N18 | 9.0% | 2.1% | 11.9% | 4.9% | 1.8% | 7.8% | 4.0% | 0.3% | 3.3% | 3.8% |
| $S10^2N12$ | 3.2% | 0.0% | 1.1% | 0.1% | 0.9% | 4.3% | 0.7% | 0.0% | 0.5% | 3.3% |
| S11N6 | 33.1% | 19.0% | 8.3% | 29.0% | 21.6% | 29.3% | 25.0% | 13.3% | 13.5% | 8.8% |
| S11N7 | 27.6% | 11.7% | 19.4% | 4.6% | 22.8% | 34.0% | 11.6% | 13.4% | 16.1% | 28.5% |
| S11N8 | 19.8% | 18.6% | 26.0% | 5.9% | 19.3% | 36.9% | 10.1% | 12.2% | 23.6% | 33.4% |
| S11N9 | 12.1% | 13.6% | 23.1% | 1.1% | 20.5% | 40.1% | 2.0% | 8.6% | 22.4% | 32.5% |
| S11N11 | 17.8% | 6.3% | 23.4% | 1.0% | 10.1% | 22.8% | 6.8% | 2.5% | 4.9% | 5.6% |
| S11N12 | 16.2% | 3.1% | 22.8% | 3.4% | 14.1% | 18.8% | 9.8% | 1.5% | 5.9% | 4.7% |
| S11N13 | 10.6% | 5.5% | 17.5% | 3.5% | 8.4% | 13.6% | 2.8% | 0.9% | 4.9% | 3.8% |
| S11N15 | 12.5% | 1.3% | 24.5% | 9.0% | 9.9% | 8.2% | 5.2% | 0.1% | 7.0% | 7.0% |
| S11N16 | 8.2% | 3.5% | 13.8% | 4.4% | 6.8% | 5.7% | 1.7% | 0.4% | 6.6% | 3.9% |
| S11N18 | 13.8% | 2.8% | 32.1% | 4.4% | 9.3% | 21.7% | 3.4% | 4.4% | 9.3% | 5.7% |
| $S11^2N12$ | 0.0% | 0.0% | 0.0% | 0.0% | 0.1% | 0.3% | 0.1% | 0.0% | 0.0% | 0.0% |
| S12N6 | 0.3% | 0.1% | 0.0% | 0.0% | 0.2% | 1.1% | 6.5% | 0.1% | 0.3% | 0.1% |
| S12N7 | 6.9% | 0.1% | 3.2% | 0.2% | 3.6% | 11.2% | 3.4% | 0.3% | 0.3% | 5.8% |
| S12N8 | 6.0% | 0.1% | 1.5% | 0.0% | 13.8% | 43.1% | 0.1% | 0.0% | 8.9% | 16.3% |
| S12N9 | 4.5% | 0.0% | 7.0% | 0.1% | 20.2% | 37.0% | 0.8% | 0.0% | 10.9% | 11.3% |
| S12N11 | 3.2% | 0.3% | 30.3% | 1.0% | 8.0% | 14.9% | 5.8% | 0.0% | 3.9% | 19.6% |

(continued)

| | D1 | D2 | D3 | D4 | D5 | D6 | D7 | D8 | D9 | D10 |
|---|---|---|---|---|---|---|---|---|---|---|
| S12N12 | 7.9% | 0.0% | 31.4% | 0.1% | 3.5% | 9.1% | 3.7% | 0.0% | 5.1% | 9.3% |
| S12N13 | 1.4% | 0.0% | 3.6% | 0.5% | 1.5% | 7.0% | 12.9% | 0.0% | 2.1% | 2.4% |
| S12N15 | 0.8% | 0.0% | 2.0% | 1.0% | 0.2% | 0.9% | 3.6% | 0.0% | 0.2% | 4.0% |
| S12N16 | 0.5% | 0.0% | 2.6% | 0.7% | 0.7% | 2.1% | 3.6% | 0.0% | 0.4% | 2.4% |
| S12N18 | 0.7% | 0.0% | 3.9% | 0.3% | 0.6% | 0.6% | 0.3% | 0.0% | 0.2% | 3.7% |
| $S12^2N12$ | 0.0% | 0.0% | 0.0% | 0.0% | 0.3% | 0.3% | 0.1% | 0.0% | 0.1% | 0.1% |
| S14N6 | 24.1% | 18.8% | 23.6% | 2.5% | 21.7% | 30.1% | 20.3% | 13.6% | 17.4% | 39.0% |
| S14N7 | 12.5% | 18.7% | 14.4% | 3.7% | 16.3% | 25.0% | 10.5% | 13.3% | 16.1% | 34.6% |
| S14N8 | 8.9% | 12.9% | 24.4% | 2.6% | 19.6% | 33.4% | 1.6% | 9.8% | 15.7% | 26.2% |
| S14N9 | 20.8% | 9.7% | 30.7% | 2.0% | 16.9% | 43.9% | 10.6% | 10.7% | 16.7% | 17.6% |
| S14N11 | 11.8% | 12.5% | 25.2% | 3.1% | 13.3% | 17.0% | 8.6% | 2.2% | 10.2% | 9.0% |
| S14N12 | 15.3% | 1.6% | 21.0% | 3.8% | 9.8% | 6.3% | 1.1% | 2.1% | 4.1% | 5.8% |
| S14N13 | 1.1% | 0.0% | 9.1% | 3.9% | 1.9% | 2.6% | 2.8% | 0.3% | 1.4% | 2.2% |
| S14N15 | 0.4% | 0.1% | 1.0% | 0.4% | 0.4% | 0.8% | 1.1% | 0.3% | 0.7% | 0.7% |
| S14N16 | 0.7% | 0.0% | 1.3% | 1.9% | 0.9% | 0.8% | 1.3% | 0.1% | 0.9% | 0.7% |
| S14N18 | 0.9% | 0.0% | 1.7% | 0.7% | 0.6% | 0.9% | 1.2% | 0.1% | 0.3% | 0.1% |
| $S14^2N12$ | 0.3% | 0.0% | 0.2% | 0.0% | 0.0% | 0.2% | 0.1% | 0.0% | 0.0% | 0.0% |
| S15N6 | 21.2% | 34.1% | 32.4% | 21.6% | 14.9% | 34.2% | 5.1% | 19.8% | 15.4% | 26.5% |
| S15N7 | 18.2% | 23.0% | 19.7% | 8.1% | 12.9% | 32.1% | 13.3% | 14.5% | 17.1% | 33.4% |
| S15N8 | 8.9% | 19.3% | 12.7% | 3.0% | 17.3% | 23.9% | 6.7% | 7.9% | 11.6% | 19.4% |
| S15N9 | 21.3% | 9.7% | 30.1% | 4.5% | 17.5% | 23.1% | 16.4% | 10.1% | 13.4% | 35.1% |
| S15N11 | 12.9% | 19.3% | 20.9% | 8.9% | 11.8% | 12.9% | 9.3% | 6.1% | 11.9% | 16.6% |
| S15N12 | 4.9% | 0.0% | 11.5% | 0.1% | 5.7% | 6.4% | 5.9% | 1.5% | 3.3% | 4.3% |
| S15N13 | 0.4% | 0.0% | 4.2% | 0.0% | 1.9% | 2.7% | 5.5% | 0.2% | 3.2% | 0.0% |
| S15N15 | 0.6% | 0.0% | 0.8% | 0.5% | 0.4% | 0.0% | 0.4% | 0.1% | 0.5% | 0.3% |
| S15N16 | 1.3% | 0.0% | 0.6% | 0.4% | 0.2% | 0.0% | 0.4% | 0.0% | 0.2% | 0.3% |
| S15N18 | 0.6% | 0.0% | 0.7% | 0.4% | 0.3% | 0.3% | 0.9% | 0.3% | 0.4% | 1.2% |
| $S15^2N12$ | 0.0% | 0.0% | 0.1% | 0.0% | 0.7% | 0.0% | 0.2% | 0.0% | 0.9% | 1.0% |
| S16N6 | 8.0% | 16.5% | 35.3% | 6.0% | 18.7% | 24.1% | 5.8% | 11.6% | 9.6% | 14.5% |
| S16N7 | 13.0% | 16.6% | 8.9% | 6.9% | 10.1% | 12.9% | 7.3% | 12.0% | 9.8% | 3.4% |
| S16N8 | 10.0% | 14.6% | 13.2% | 8.4% | 6.7% | 11.1% | 4.6% | 16.5% | 10.9% | 14.9% |
| S16N9 | 7.5% | 7.4% | 13.7% | 9.1% | 7.8% | 11.7% | 5.3% | 6.7% | 9.5% | 6.9% |
| S16N11 | 17.2% | 9.6% | 16.6% | 5.8% | 9.5% | 10.3% | 15.5% | 5.0% | 9.6% | 0.0% |
| S16N12 | 4.2% | 0.0% | 3.9% | 0.0% | 1.9% | 2.7% | 0.7% | 0.0% | 11.4% | 2.1% |
| S16N13 | 1.2% | 0.0% | 1.5% | 0.0% | 0.8% | 1.2% | 3.7% | 0.2% | 5.7% | 2.0% |
| S16N15 | 1.6% | 0.0% | 0.0% | 2.6% | 0.3% | 0.0% | 0.6% | 0.0% | 0.3% | 0.2% |
| S16N16 | 0.7% | 0.0% | 1.6% | 3.4% | 0.5% | 0.1% | 0.2% | 0.0% | 0.0% | 0.2% |
| S16N18 | 0.3% | 0.0% | 0.6% | 0.2% | 0.0% | 0.0% | 0.0% | 0.0% | 0.0% | 1.2% |

(continued)

| | D1 | D2 | D3 | D4 | D5 | D6 | D7 | D8 | D9 | D10 |
|---|---|---|---|---|---|---|---|---|---|---|
| S16[2]N12 | 0.0% | 0.0% | 0.0% | 0.1% | 0.0% | 0.0% | 0.1% | 0.0% | 0.6% | 0.3% |
| S18N6 | 6.9% | 5.9% | 22.1% | 7.2% | 18.1% | 14.5% | 10.4% | 5.5% | 11.0% | 8.9% |
| S18N7 | 6.8% | 3.2% | 15.6% | 3.5% | 28.2% | 20.7% | 4.5% | 3.9% | 9.3% | 13.5% |
| S18N8 | 4.1% | 3.8% | 9.6% | 3.9% | 13.6% | 23.0% | 6.7% | 2.0% | 6.6% | 11.7% |
| S18N9 | 5.3% | 3.4% | 20.4% | 4.0% | 7.7% | 19.5% | 6.7% | 3.0% | 0.0% | 4.5% |
| S18N11 | 3.7% | 1.4% | 4.2% | 10.1% | 6.3% | 5.2% | 2.7% | 1.3% | 2.9% | 2.7% |
| S18N12 | 1.3% | 0.7% | 1.8% | 0.3% | 1.0% | 0.5% | 0.5% | 0.0% | 0.0% | 5.3% |
| S18N13 | 1.3% | 0.0% | 2.8% | 0.0% | 0.0% | 2.6% | 0.0% | 0.0% | 1.8% | 0.9% |
| S18N15 | 0.2% | 0.0% | 0.2% | 0.2% | 0.0% | 0.0% | 0.1% | 0.0% | 0.1% | 0.2% |
| S18N16 | 0.0% | 0.0% | 0.1% | 0.1% | 0.2% | 0.0% | 0.0% | 0.0% | 0.0% | 0.1% |
| S18N18 | 0.1% | 0.1% | 0.0% | 0.0% | 0.0% | 0.4% | 0.0% | 0.0% | 0.0% | 0.0% |
| S18[2]N12 | 0.0% | 0.0% | 0.2% | 0.0% | 0.0% | 1.6% | 0.0% | 0.0% | 0.0% | 0.4% |
| S6S6 | 1.1% | 0.4% | 4.4% | 1.4% | 9.1% | 12.5% | 23.0% | 0.8% | 7.6% | 21.6% |
| S6S7 | 0.5% | 1.2% | 1.4% | 2.0% | 5.7% | 6.9% | 22.2% | 0.0% | 7.5% | 21.6% |
| S6S8 | 1.9% | 2.5% | 2.8% | 1.3% | 15.3% | 7.4% | 15.0% | 0.6% | 10.0% | 24.1% |
| S6S9 | 2.6% | 9.1% | 6.9% | 1.8% | 13.7% | 7.1% | 10.2% | 2.0% | 8.4% | 18.0% |
| S6S10 | 3.2% | 8.3% | 4.4% | 4.5% | 12.4% | 20.9% | 7.7% | 0.0% | 7.9% | 14.9% |
| S6S11 | 6.0% | 18.7% | 12.4% | 6.2% | 32.7% | 32.1% | 10.1% | 4.5% | 16.8% | 38.1% |
| S6S12 | 6.3% | 5.0% | 4.3% | 3.0% | 28.8% | 13.6% | 6.6% | 7.5% | 9.4% | 24.4% |
| S6S14 | 8.3% | 11.1% | 9.2% | 12.0% | 18.6% | 21.9% | 4.7% | 2.3% | 4.1% | 53.3% |
| S6S15 | 5.5% | 8.4% | 14.5% | 7.9% | 13.7% | 36.4% | 3.3% | 11.5% | 8.3% | 34.5% |
| S6S16 | 8.4% | 24.0% | 9.8% | 7.9% | 15.2% | 0.4% | 3.7% | 0.0% | 3.9% | 37.6% |
| S6S18 | 9.8% | 8.1% | 13.8% | 10.4% | 15.1% | 0.0% | 6.4% | 15.9% | 8.7% | 34.7% |
| S7S7 | 7.9% | 2.2% | 9.2% | 0.0% | 4.8% | 10.3% | 9.3% | 0.6% | 7.9% | 5.5% |
| S7S8 | 6.7% | 0.0% | 9.2% | 7.0% | 11.2% | 3.6% | 5.8% | 3.7% | 10.2% | 0.0% |
| S7S9 | 10.8% | 28.8% | 21.5% | 15.1% | 12.5% | 3.4% | 14.5% | 12.2% | 17.1% | 16.3% |
| S7S10 | 21.8% | 23.6% | 19.4% | 8.1% | 10.6% | 4.7% | 20.2% | 18.0% | 23.9% | 25.2% |
| S7S11 | 10.8% | 27.3% | 9.7% | 14.2% | 32.4% | 28.7% | 10.4% | 11.1% | 25.9% | 39.1% |
| S7S12 | 8.2% | 18.0% | 9.9% | 9.5% | 9.6% | 11.7% | 7.1% | 10.9% | 4.8% | 21.3% |
| S7S14 | 7.7% | 15.9% | 8.4% | 9.1% | 10.7% | 4.4% | 4.0% | 11.0% | 3.8% | 26.7% |
| S7S15 | 9.4% | 24.0% | 4.6% | 11.6% | 14.9% | 7.6% | 5.0% | 10.9% | 1.6% | 33.3% |
| S7S16 | 10.0% | 17.6% | 6.3% | 10.9% | 7.9% | 9.0% | 3.5% | 12.8% | 2.3% | 22.2% |
| S7S18 | 3.7% | 7.1% | 11.2% | 8.3% | 14.2% | 5.5% | 7.3% | 16.1% | 13.1% | 48.9% |
| S8S8 | 17.9% | 17.6% | 10.4% | 11.2% | 10.2% | 5.1% | 10.1% | 13.8% | 12.1% | 27.9% |
| S8S9 | 15.6% | 37.0% | 15.1% | 13.2% | 5.8% | 6.3% | 29.3% | 16.0% | 23.5% | 27.9% |
| S8S10 | 16.3% | 18.8% | 8.9% | 8.3% | 19.5% | 9.3% | 11.4% | 13.7% | 25.6% | 26.2% |
| S8S11 | 9.2% | 17.9% | 6.1% | 8.5% | 10.6% | 7.9% | 10.3% | 15.3% | 12.8% | 42.4% |
| S8S12 | 10.7% | 16.1% | 10.9% | 10.8% | 24.8% | 12.6% | 9.3% | 34.4% | 3.5% | 23.5% |

(continued)

| | D1 | D2 | D3 | D4 | D5 | D6 | D7 | D8 | D9 | D10 |
|---|---|---|---|---|---|---|---|---|---|---|
| S8S14 | 8.3% | 18.5% | 13.9% | 9.3% | 29.9% | 16.2% | 7.5% | 17.1% | 16.0% | 41.1% |
| S8S15 | 8.1% | 10.8% | 21.8% | 11.2% | 31.2% | 24.5% | 13.8% | 23.0% | 13.9% | 32.1% |
| S8S16 | 4.6% | 15.7% | 20.7% | 10.2% | 3.7% | 33.0% | 11.8% | 28.6% | 6.7% | 75.2% |
| S8S18 | 7.6% | 0.9% | 14.3% | 15.8% | 9.6% | 8.2% | 9.2% | 18.7% | 17.7% | 19.3% |
| S9S9 | 12.9% | 6.8% | 11.9% | 14.5% | 12.5% | 5.6% | 32.6% | 19.1% | 19.3% | 11.3% |
| S9S10 | 8.6% | 10.7% | 10.3% | 13.3% | 27.6% | 21.2% | 8.3% | 22.5% | 34.1% | 18.7% |
| S9S11 | 9.5% | 17.3% | 10.0% | 15.2% | 11.4% | 41.9% | 8.1% | 21.8% | 8.5% | 32.8% |
| S9S12 | 14.6% | 16.7% | 4.9% | 0.0% | 21.0% | 20.2% | 5.3% | 30.4% | 39.3% | 17.9% |
| S9S14 | 2.1% | 14.3% | 5.1% | 7.2% | 14.6% | 19.2% | 12.5% | 21.0% | 28.4% | 14.5% |
| S9S15 | 2.1% | 10.6% | 2.5% | 11.8% | 8.9% | 47.4% | 5.0% | 22.2% | 25.3% | 56.1% |
| S9S16 | 0.0% | 1.5% | 0.0% | 12.2% | 23.5% | 55.4% | 8.4% | 23.2% | 13.9% | 56.9% |
| S9S18 | 4.9% | 2.3% | 13.9% | 5.5% | 4.0% | 1.7% | 11.8% | 27.0% | 0.0% | 7.0% |
| S10S10 | 7.8% | 4.0% | 13.6% | 5.5% | 14.7% | 23.6% | 7.2% | 14.0% | 13.4% | 18.8% |
| S10S11 | 7.0% | 2.2% | 6.8% | 16.7% | 8.4% | 8.2% | 7.3% | 16.4% | 13.6% | 13.3% |
| S10S12 | 6.6% | 2.4% | 8.2% | 12.0% | 9.8% | 16.2% | 7.2% | 20.9% | 28.1% | 13.7% |
| S10S14 | 8.5% | 1.5% | 7.9% | 5.9% | 15.7% | 26.9% | 2.6% | 18.9% | 3.9% | 23.0% |
| S10S15 | 6.8% | 7.7% | 7.6% | 7.5% | 3.7% | 32.8% | 7.2% | 15.2% | 3.9% | 55.8% |
| S10S16 | 7.2% | 0.1% | 9.5% | 9.1% | 9.4% | 14.1% | 3.1% | 15.7% | 7.8% | 79.4% |
| S10S18 | 9.9% | 1.6% | 11.1% | 6.7% | 3.7% | 22.4% | 9.2% | 13.7% | 6.5% | 43.1% |
| S11S11 | 1.3% | 0.2% | 6.5% | 4.0% | 3.4% | 10.9% | 0.0% | 3.6% | 4.3% | 12.2% |
| S11S12 | 2.3% | 0.1% | 5.4% | 5.6% | 2.8% | 5.2% | 3.1% | 10.7% | 2.4% | 4.8% |
| S11S14 | 2.0% | 0.7% | 6.7% | 3.7% | 5.1% | 6.6% | 2.9% | 8.8% | 4.1% | 58.1% |
| S11S15 | 0.9% | 0.3% | 7.4% | 5.3% | 4.8% | 4.8% | 5.3% | 3.0% | 1.8% | 42.3% |
| S11S16 | 0.5% | 0.0% | 6.2% | 1.7% | 4.9% | 2.7% | 2.2% | 4.7% | 3.6% | 49.0% |
| S11S18 | 0.6% | 0.2% | 4.8% | 3.3% | 1.7% | 3.6% | 3.6% | 7.8% | 0.9% | 20.9% |
| S12S12 | 0.5% | 0.1% | 2.1% | 4.1% | 0.4% | 3.9% | 3.3% | 3.4% | 1.9% | 2.7% |
| S12S14 | 0.6% | 0.1% | 6.5% | 6.7% | 0.2% | 4.0% | 3.3% | 3.7% | 0.8% | 6.5% |
| S12S15 | 0.8% | 0.5% | 2.9% | 3.8% | 0.1% | 4.4% | 1.4% | 3.1% | 2.0% | 9.9% |
| S12S16 | 0.6% | 0.0% | 2.0% | 3.3% | 0.1% | 2.7% | 0.7% | 0.2% | 0.5% | 7.3% |
| S12S18 | 0.0% | 0.0% | 2.1% | 2.6% | 0.7% | 1.0% | 0.7% | 0.4% | 1.0% | 7.5% |
| S14S14 | 0.1% | 0.0% | 0.2% | 0.7% | 0.0% | 0.2% | 2.3% | 0.0% | 0.0% | 0.9% |
| S14S15 | 0.1% | 0.1% | 0.3% | 0.7% | 0.0% | 0.2% | 0.6% | 0.0% | 0.0% | 0.3% |
| S14S16 | 0.0% | 0.0% | 0.4% | 0.6% | 0.1% | 1.2% | 1.1% | 0.0% | 0.0% | 2.7% |
| S14S18 | 0.0% | 0.2% | 2.8% | 2.5% | 0.2% | 1.2% | 0.9% | 0.2% | 0.5% | 1.7% |
| S15S15 | 1.0% | 0.0% | 0.0% | 1.5% | 0.3% | 0.4% | 0.6% | 0.0% | 0.4% | 1.0% |
| S15S16 | 1.7% | 0.0% | 0.1% | 2.0% | 0.2% | 0.2% | 0.6% | 0.0% | 0.3% | 0.6% |
| S15S18 | 0.0% | 0.1% | 2.6% | 0.3% | 0.3% | 1.0% | 1.5% | 0.0% | 0.3% | 0.3% |
| S16S16 | 0.6% | 1.1% | 0.9% | 1.2% | 0.0% | 0.5% | 0.5% | 0.0% | 0.5% | 0.1% |

(continued)

| | D1 | D2 | D3 | D4 | D5 | D6 | D7 | D8 | D9 | D10 |
|---|---|---|---|---|---|---|---|---|---|---|
| S16S18 | 1.2% | 0.0% | 1.9% | 0.3% | 1.7% | 0.2% | 1.0% | 0.3% | 0.4% | 0.8% |
| S18S18 | 0.0% | 0.0% | 0.1% | 0.1% | 0.1% | 0.2% | 0.2% | 0.0% | 0.9% | 0.0% |
| N6N6 | 0.0% | 0.0% | 0.2% | 0.0% | 0.0% | 0.0% | 0.0% | 0.0% | 0.0% | 0.0% |
| N6N7 | 0.0% | 0.0% | 0.4% | 0.0% | 0.0% | 0.0% | 0.0% | 0.0% | 0.0% | 0.0% |
| N6N8 | 0.0% | 0.0% | 0.1% | 0.0% | 0.0% | 0.1% | 0.0% | 0.0% | 0.0% | 0.0% |
| N6N9 | 0.0% | 0.0% | 0.3% | 0.0% | 0.0% | 0.0% | 0.0% | 0.0% | 0.0% | 0.0% |
| N6N11 | 0.0% | 0.0% | 0.3% | 0.0% | 0.1% | 0.2% | 0.0% | 0.0% | 0.0% | 0.0% |
| N6N12 | 0.0% | 0.0% | 0.2% | 0.0% | 0.1% | 0.3% | 0.0% | 0.0% | 0.0% | 0.2% |
| N6N13 | 0.0% | 0.0% | 0.5% | 0.0% | 0.0% | 0.1% | 0.1% | 0.0% | 0.0% | 0.0% |
| N6N15 | 0.0% | 0.0% | 0.6% | 0.1% | 0.0% | 0.3% | 0.0% | 0.3% | 0.1% | 0.2% |
| N6N16 | 0.4% | 0.0% | 0.7% | 0.0% | 0.0% | 0.2% | 0.0% | 0.6% | 0.0% | 0.7% |
| N6N18 | 1.1% | 0.6% | 3.4% | 2.0% | 0.6% | 1.8% | 0.9% | 0.3% | 0.1% | 0.4% |
| $N6^2N12$ | 0.6% | 0.2% | 0.4% | 0.2% | 0.2% | 0.4% | 0.1% | 0.0% | 0.0% | 0.4% |
| N7N7 | 0.0% | 0.0% | 0.0% | 0.0% | 0.0% | 0.0% | 0.0% | 0.0% | 0.0% | 0.1% |
| N7N8 | 0.2% | 0.0% | 0.5% | 0.2% | 0.0% | 0.0% | 0.0% | 0.0% | 0.7% | 0.0% |
| N7N9 | 0.6% | 0.0% | 0.0% | 0.0% | 0.0% | 0.0% | 0.0% | 0.0% | 0.7% | 0.4% |
| N7N11 | 1.3% | 0.1% | 1.0% | 1.4% | 0.6% | 1.1% | 0.0% | 0.0% | 0.1% | 0.7% |
| N7N12 | 0.2% | 0.0% | 3.5% | 0.0% | 0.3% | 0.4% | 0.0% | 0.1% | 0.1% | 1.4% |
| N7N13 | 1.0% | 0.1% | 0.0% | 0.0% | 0.9% | 1.6% | 0.1% | 0.0% | 1.3% | 1.6% |
| N7N15 | 1.0% | 0.0% | 5.2% | 0.2% | 0.0% | 1.6% | 0.0% | 0.2% | 0.6% | 0.6% |
| N7N16 | 0.9% | 0.0% | 2.3% | 0.0% | 0.3% | 0.6% | 0.0% | 0.1% | 0.0% | 1.8% |
| N7N18 | 1.0% | 0.0% | 5.0% | 0.2% | 0.7% | 0.7% | 0.0% | 0.2% | 2.1% | 15.6% |
| $N7^2N12$ | 3.6% | 1.7% | 2.5% | 1.2% | 0.9% | 0.7% | 0.6% | 1.6% | 1.9% | 1.4% |
| N8N8 | 2.3% | 0.0% | 1.8% | 0.0% | 0.0% | 0.0% | 0.0% | 0.0% | 0.7% | 0.0% |
| N8N9 | 0.0% | 0.0% | 0.5% | 0.0% | 0.0% | 0.4% | 0.0% | 0.0% | 0.0% | 0.5% |
| N8N11 | 0.4% | 0.0% | 3.6% | 0.2% | 0.2% | 0.3% | 0.0% | 0.0% | 0.0% | 0.3% |
| N8N12 | 0.6% | 0.0% | 2.4% | 0.0% | 0.6% | 0.4% | 0.0% | 0.0% | 0.0% | 0.7% |
| N8N13 | 2.2% | 0.0% | 0.9% | 0.7% | 0.0% | 0.0% | 0.0% | 0.0% | 1.0% | 2.1% |
| N8N15 | 1.5% | 0.0% | 1.5% | 0.5% | 0.6% | 1.3% | 0.0% | 1.1% | 0.0% | 3.2% |
| N8N16 | 3.8% | 0.8% | 8.5% | 5.3% | 0.2% | 2.4% | 3.1% | 2.3% | 1.0% | 3.4% |
| N8N18 | 1.8% | 1.7% | 9.8% | 8.2% | 1.9% | 2.2% | 2.7% | 1.3% | 1.2% | 1.4% |
| $N8^2N12$ | 0.0% | 0.1% | 0.1% | 0.0% | 0.0% | 0.0% | 0.0% | 0.2% | 0.2% | 0.0% |
| N9N9 | 0.2% | 0.0% | 0.3% | 0.0% | 0.0% | 0.2% | 0.0% | 0.0% | 0.0% | 0.0% |
| N9N11 | 0.0% | 0.0% | 0.0% | 0.0% | 0.0% | 0.0% | 0.0% | 0.0% | 0.0% | 0.0% |
| N9N12 | 0.0% | 0.0% | 0.6% | 0.0% | 0.0% | 0.0% | 0.0% | 0.0% | 0.0% | 0.0% |
| N9N13 | 0.4% | 0.0% | 1.0% | 0.0% | 0.3% | 0.0% | 0.0% | 0.0% | 0.0% | 0.3% |
| N9N15 | 0.5% | 0.0% | 0.5% | 0.0% | 0.2% | 0.0% | 0.0% | 0.2% | 0.9% | 0.0% |
| N9N16 | 2.1% | 0.0% | 2.2% | 0.8% | 0.0% | 2.2% | 0.0% | 1.8% | 0.0% | 4.8% |

(continued)

| | D1 | D2 | D3 | D4 | D5 | D6 | D7 | D8 | D9 | D10 |
|---|---|---|---|---|---|---|---|---|---|---|
| N9N18 | 4.0% | 3.6% | 2.0% | 19.9% | 0.0% | 2.2% | 3.0% | 1.4% | 0.3% | 2.6% |
| N9[2]N12 | 0.0% | 0.0% | 0.4% | 0.0% | 0.0% | 0.3% | 0.0% | 0.0% | 0.0% | 0.0% |
| N11N11 | 0.9% | 0.0% | 1.8% | 0.0% | 0.9% | 0.7% | 0.6% | 0.0% | 0.0% | 0.5% |
| N11N12 | 0.0% | 0.5% | 1.9% | 0.0% | 0.0% | 0.5% | 0.0% | 0.0% | 0.0% | 1.8% |
| N11N13 | 1.4% | 0.0% | 0.0% | 0.0% | 0.1% | 0.8% | 0.5% | 0.0% | 0.0% | 0.0% |
| N11N15 | 1.3% | 0.3% | 0.3% | 0.9% | 0.2% | 1.6% | 0.2% | 1.1% | 0.1% | 0.4% |
| N11N16 | 0.4% | 0.0% | 1.2% | 0.1% | 0.0% | 0.2% | 0.0% | 0.0% | 0.0% | 0.6% |
| N11N18 | 3.4% | 0.3% | 0.6% | 1.9% | 1.4% | 0.4% | 1.1% | 2.2% | 0.0% | 0.2% |
| N112N12 | 0.7% | 0.0% | 1.1% | 0.5% | 1.2% | 0.1% | 0.4% | 0.0% | 0.1% | 0.7% |
| N12N12 | 0.1% | 0.2% | 1.9% | 0.4% | 0.4% | 0.3% | 0.1% | 0.0% | 0.0% | 0.5% |
| N12N13 | 1.2% | 0.1% | 1.7% | 0.0% | 0.3% | 0.5% | 0.2% | 0.3% | 0.3% | 0.2% |
| N12N15 | 1.5% | 0.3% | 1.1% | 1.4% | 0.4% | 0.9% | 0.8% | 1.4% | 1.5% | 0.7% |
| N12N16 | 2.6% | 0.9% | 1.2% | 1.2% | 0.1% | 1.9% | 0.4% | 0.7% | 0.9% | 0.0% |
| N12N18 | 1.4% | 0.6% | 3.5% | 0.9% | 0.0% | 1.5% | 1.2% | 1.5% | 0.1% | 0.0% |
| N12[2]N12 | 0.5% | 0.4% | 3.2% | 2.5% | 0.5% | 3.6% | 2.2% | 0.6% | 3.0% | 0.7% |
| N13N13 | 1.5% | 0.0% | 0.7% | 1.5% | 0.9% | 2.5% | 0.1% | 1.4% | 1.1% | 0.3% |
| N13N15 | 1.5% | 0.0% | 1.4% | 0.0% | 0.2% | 0.3% | 0.8% | 0.0% | 0.2% | 0.0% |
| N13N16 | 0.8% | 0.0% | 0.4% | 1.0% | 0.0% | 1.0% | 1.5% | 0.0% | 0.0% | 0.0% |
| N13N18 | 2.7% | 0.2% | 0.5% | 1.8% | 0.2% | 3.0% | 3.7% | 0.0% | 1.1% | 0.5% |
| N13[2]N12 | 1.2% | 0.1% | 0.6% | 0.8% | 0.0% | 1.3% | 0.2% | 0.0% | 1.7% | 0.0% |
| N15N15 | 2.3% | 0.0% | 1.8% | 1.3% | 0.9% | 1.5% | 0.5% | 0.0% | 0.4% | 1.1% |
| N15N16 | 1.8% | 0.0% | 2.5% | 1.9% | 0.7% | 0.8% | 0.9% | 0.0% | 2.1% | 1.5% |
| N15N18 | 0.0% | 0.0% | 3.0% | 1.6% | 0.0% | 0.6% | 3.2% | 0.9% | 0.7% | 3.5% |
| N15[2]N12 | 0.9% | 0.5% | 2.4% | 2.1% | 2.8% | 6.3% | 1.3% | 0.9% | 1.7% | 0.0% |
| N16N16 | 1.3% | 0.2% | 1.4% | 0.9% | 0.0% | 0.9% | 0.0% | 0.5% | 1.9% | 0.0% |
| N16N18 | 1.2% | 1.2% | 0.4% | 0.6% | 1.1% | 0.0% | 0.0% | 0.0% | 0.0% | 3.0% |
| N16[2]N12 | 0.0% | 0.4% | 2.0% | 3.2% | 0.6% | 0.5% | 1.1% | 0.4% | 0.5% | 1.5% |
| N18N18 | 1.9% | 0.0% | 2.0% | 0.9% | 1.1% | 0.3% | 0.3% | 0.1% | 0.0% | 0.0% |
| N18[2]N12 | 1.0% | 0.0% | 1.5% | 0.9% | 0.7% | 1.3% | 0.2% | 0.4% | 0.6% | 0.7% |
| [2]N12[2]N12 | 0.4% | 0.0% | 1.3% | 0.0% | 0.0% | 0.0% | 0.0% | 0.1% | 0.2% | 1.8% |
| Lipofect amine2000 | 26.5% | | | | | | | | | |

**Example 5: Preliminary screening of amino lipid compounds as mRNA vectors**

Cell line: HEK293 cells (ATCC CRL-1573™)
Medium: DMEM supplemented with 10% fetal bovine serum (Invitrogen)
Screening: cell transfection on 96-well plates
Detection (readout): percentage of the number of GFP fluorescent cells relative to the total number of cells (the total number of cells was determined using nucleus dye Hoechst). Lipofectamine2000 (Invitrogen) was used as the positive control.

Method: the experimental method was basically the same with Example 4, and the mass of EGFP mRNA (TriLink) for transfection was 50 ng per well.
Results: the absolute mRNA transfection efficiency in HEK293 cells for 2530 compounds is shown in Table 9.

Table 9: Absolute mRNA transfection efficiency in HEK293 cells for 2530 compounds.

| | D1 | D2 | D3 | D4 | D5 | D6 | D7 | D8 | D9 | D10 |
|---|---|---|---|---|---|---|---|---|---|---|
| S6N6 | 0.8% | 0.4% | 0.1% | 0.3% | 2.1% | 0.0% | 1.2% | 0.0% | 1.5% | 0.3% |
| S6N7 | 2.3% | 2.7% | 0.2% | 0.0% | 4.3% | 0.2% | 0.0% | 0.4% | 2.6% | 5.5% |
| S6N8 | 3.1% | 0.0% | 0.0% | 0.3% | 5.9% | 0.4% | 3.5% | 0.2% | 2.5% | 1.4% |
| S6N9 | 1.8% | 0.8% | 0.3% | 1.1% | 2.0% | 0.3% | 2.4% | 1.2% | 0.9% | 1.2% |
| S6N11 | 1.0% | 0.8% | 0.7% | 0.9% | 5.3% | 2.5% | 3.8% | 1.1% | 11.9% | 4.0% |
| S6N12 | 0.0% | 0.8% | 4.1% | 0.7% | 4.4% | 3.5% | 1.4% | 2.0% | 4.9% | 9.6% |
| S6N13 | 1.8% | 0.2% | 7.7% | 2.1% | 7.2% | 3.0% | 2.6% | 0.0% | 6.6% | 22.7% |
| S6N15 | 3.6% | 1.2% | 14.4% | 0.4% | 16.4% | 12.1% | 2.2% | 0.1% | 11.9% | 7.9% |
| S6N16 | 3.3% | 2.2% | 37.3% | 0.5% | 35.4% | 40.4% | 3.0% | 0.6% | 29.6% | 41.5% |
| S6N18 | 32.7% | 4.5% | 32.5% | 1.8% | 16.6% | 60.1% | 4.6% | 0.7% | 24.0% | 41.4% |
| $S6^2N12$ | 0.7% | 0.0% | 0.4% | 2.2% | 0.3% | 0.0% | 0.2% | 0.0% | 0.1% | 0.1% |
| S7N6 | 5.1% | 1.2% | 5.8% | 4.5% | 1.9% | 8.8% | 2.3% | 1.8% | 0.8% | 2.0% |
| S7N7 | 4.4% | 1.1% | 6.6% | 3.4% | 1.0% | 1.8% | 3.9% | 1.4% | 2.5% | 0.9% |
| S7N8 | 2.0% | 1.0% | 1.3% | 0.5% | 4.3% | 2.8% | 3.5% | 1.8% | 1.7% | 1.3% |
| S7N9 | 10.0% | 8.7% | 2.8% | 4.9% | 3.1% | 5.9% | 10.5% | 10.6% | 5.2% | 4.6% |
| S7N11 | 5.8% | 16.0% | 9.6% | 1.0% | 8.2% | 18.9% | 11.1% | 20.1% | 13.8% | 25.3% |
| S7N12 | 5.4% | 3.0% | 6.5% | 0.6% | 23.0% | 12.5% | 3.6% | 2.3% | 62.7% | 63.7% |
| S7N13 | 12.8% | 6.2% | 19.1% | 0.7% | 39.5% | 44.9% | 1.2% | 5.8% | 60.4% | 70.8% |
| S7N15 | 21.3% | 3.1% | 33.7% | 2.6% | 38.5% | 37.9% | 18.5% | 2.8% | 32.6% | 67.3% |
| S7N16 | 29.5% | 2.6% | 25.0% | 1.3% | 32.2% | 44.8% | 12.8% | 1.2% | 29.3% | 44.3% |
| S7N18 | 35.7% | 5.2% | 39.8% | 7.9% | 38.5% | 49.2% | 16.7% | 3.0% | 37.1% | 45.8% |
| $S7^2N12$ | 0.7% | 0.4% | 0.4% | 1.0% | 0.2% | 0.9% | 1.2% | 0.6% | 1.0% | 1.4% |
| S8N6 | 2.4% | 0.7% | 2.6% | 2.7% | 1.6% | 9.4% | 0.8% | 1.0% | 0.7% | 0.4% |
| S8N7 | 2.0% | 0.9% | 0.7% | 1.8% | 2.5% | 4.3% | 0.7% | 0.1% | 1.4% | 0.3% |
| S8N8 | 5.8% | 0.9% | 3.0% | 0.6% | 1.3% | 3.5% | 2.5% | 1.3% | 4.5% | 1.2% |
| S8N9 | 21.4% | 8.3% | 3.2% | 2.3% | 11.2% | 6.7% | 9.5% | 3.5% | 57.0% | 30.2% |
| S8N11 | 9.0% | 8.1% | 6.6% | 0.3% | 30.1% | 32.3% | 9.0% | 7.9% | 81.7% | 81.5% |
| S8N12 | 17.9% | 7.0% | 24.6% | 3.5% | 52.5% | 46.0% | 9.5% | 3.8% | 57.6% | 73.1% |
| S8N13 | 15.5% | 7.5% | 35.8% | 4.5% | 44.2% | 58.0% | 14.2% | 1.5% | 46.5% | 75.0% |
| S8N15 | 23.8% | 5.5% | 21.8% | 2.6% | 14.9% | 55.3% | 15.7% | 4.6% | 37.8% | 53.3% |
| S8N16 | 22.5% | 3.4% | 14.3% | 1.9% | 32.3% | 67.0% | 16.8% | 4.9% | 21.2% | 36.4% |
| S8N18 | 19.3% | 3.7% | 28.6% | 3.5% | 29.9% | 40.4% | 12.8% | 1.2% | 29.4% | 20.1% |
| $S8^2N12$ | 0.4% | 0.1% | 0.9% | 1.1% | 0.9% | 1.5% | 1.3% | 0.1% | 0.6% | 1.5% |
| S9N6 | 1.1% | 0.5% | 0.2% | 0.3% | 2.9% | 1.4% | 0.6% | 0.6% | 1.8% | 0.6% |
| S9N7 | 0.3% | 4.2% | 1.4% | 3.0% | 2.6% | 1.2% | 1.6% | 2.2% | 2.8% | 0.9% |

(continued)

| | D1 | D2 | D3 | D4 | D5 | D6 | D7 | D8 | D9 | D10 |
|---|---|---|---|---|---|---|---|---|---|---|
| S9N8 | 2.9% | 22.6% | 10.4% | 6.6% | 6.0% | 7.5% | 11.1% | 5.7% | 9.2% | 16.3% |
| S9N9 | 8.0% | 38.5% | 15.4% | 11.3% | 38.2% | 19.8% | 17.8% | 14.0% | 76.1% | 70.0% |
| S9N11 | 53.0% | 44.8% | 47.8% | 17.4% | 32.1% | 58.8% | 36.8% | 23.5% | 49.2% | 66.0% |
| S9N12 | 30.2% | 15.2% | 49.6% | 9.5% | 24.6% | 39.3% | 25.6% | 11.2% | 36.0% | 57.0% |
| S9N13 | 25.8% | 12.3% | 58.2% | 10.8% | 41.5% | 70.9% | 33.4% | 12.6% | 20.0% | 75.8% |
| S9N15 | 27.4% | 5.9% | 67.9% | 4.9% | 32.9% | 36.5% | 11.7% | 7.2% | 6.3% | 17.5% |
| S9N16 | 25.6% | 1.4% | 50.3% | 2.2% | 16.1% | 46.2% | 10.9% | 2.1% | 16.7% | 30.2% |
| S9N18 | 47.6% | 2.4% | 26.7% | 8.3% | 28.6% | 44.8% | 21.1% | 1.9% | 14.5% | 22.4% |
| $S9^2N12$ | 1.0% | 0.1% | 1.3% | 0.1% | 0.4% | 1.7% | 0.2% | 0.3% | 0.3% | 7.7% |
| S10N6 | 0.4% | 0.1% | 0.4% | 0.1% | 1.1% | 1.5% | 0.6% | 0.2% | 1.1% | 0.5% |
| S10N7 | 0.2% | 0.5% | 1.4% | 0.1% | 3.2% | 4.4% | 1.2% | 0.6% | 0.8% | 1.0% |
| S10N8 | 0.5% | 0.5% | 0.4% | 0.1% | 8.6% | 3.7% | 0.3% | 0.4% | 5.4% | 1.7% |
| S10N9 | 0.5% | 0.5% | 2.7% | 0.2% | 8.5% | 8.7% | 1.6% | 0.1% | 10.5% | 4.2% |
| S10N11 | 6.2% | 0.2% | 17.1% | 0.8% | 11.0% | 10.0% | 3.1% | 0.1% | 11.3% | 8.8% |
| S10N12 | 2.4% | 1.5% | 19.1% | 1.0% | 3.6% | 25.4% | 10.6% | 0.1% | 6.0% | 15.5% |
| S10N13 | 1.7% | 0.2% | 35.0% | 0.3% | 4.6% | 10.2% | 13.7% | 0.2% | 2.7% | 8.5% |
| S10N15 | 2.8% | 1.3% | 57.6% | 1.5% | 1.8% | 3.5% | 2.0% | 0.2% | 5.4% | 13.2% |
| S10N16 | 40.7% | 0.6% | 56.7% | 14.7% | 3.9% | 51.5% | 19.3% | 0.2% | 13.6% | 15.4% |
| S10N18 | 4.8% | 0.0% | 59.6% | 37.7% | 2.3% | 31.8% | 25.9% | 0.1% | 9.0% | 6.0% |
| $S10^2N12$ | 0.4% | 0.0% | 0.1% | 0.1% | 0.1% | 0.1% | 0.0% | 0.0% | 0.3% | 0.2% |
| S11N6 | 4.4% | 5.4% | 18.1% | 2.7% | 26.9% | 1.7% | 4.3% | 2.8% | 12.2% | 6.8% |
| S11N7 | 11.7% | 8.5% | 8.0% | 2.3% | 8.5% | 29.1% | 11.8% | 3.1% | 10.5% | 18.2% |
| S11N8 | 13.1% | 2.4% | 31.8% | 4.3% | 36.2% | 16.8% | 13.4% | 0.3% | 37.6% | 46.1% |
| S11N9 | 14.7% | 2.5% | 24.5% | 4.2% | 41.5% | 45.1% | 5.0% | 1.6% | 60.3% | 87.7% |
| S11N11 | 24.7% | 0.6% | 33.5% | 2.0% | 36.5% | 35.5% | 35.0% | 3.6% | 24.5% | 27.7% |
| S11N12 | 27.3% | 3.1% | 40.5% | 8.9% | 22.8% | 15.9% | 7.8% | 1.0% | 21.7% | 24.7% |
| S11N13 | 12.6% | 1.9% | 25.9% | 15.4% | 11.2% | 10.0% | 5.8% | 3.1% | 9.4% | 10.4% |
| S11N15 | 6.2% | 2.8% | 31.3% | 17.0% | 20.4% | 13.1% | 6.5% | 0.5% | 15.7% | 18.6% |
| S11N16 | 13.0% | 1.1% | 15.7% | 12.3% | 3.9% | 6.2% | 4.4% | 0.7% | 3.9% | 10.4% |
| S11N18 | 15.4% | 0.0% | 40.6% | 8.8% | 4.7% | 14.6% | 10.8% | 0.6% | 14.9% | 9.6% |
| $S11^2N12$ | 0.0% | 0.3% | 0.0% | 0.5% | 0.6% | 3.2% | 0.7% | 0.4% | 0.0% | 0.0% |
| S12N6 | 0.0% | 0.0% | 0.0% | 0.9% | 0.3% | 1.1% | 2.1% | 0.9% | 0.2% | 0.2% |
| S12N7 | 1.5% | 0.4% | 9.2% | 0.5% | 9.9% | 13.0% | 13.1% | 1.5% | 0.6% | 1.7% |
| S12N8 | 11.3% | 1.6% | 12.3% | 1.6% | 22.6% | 30.5% | 3.0% | 2.3% | 20.1% | 12.9% |
| S12N9 | 34.2% | 1.1% | 39.9% | 0.7% | 26.5% | 24.0% | 7.4% | 2.3% | 25.6% | 17.5% |
| S12N11 | 2.6% | 0.3% | 21.2% | 3.7% | 1.1% | 14.0% | 4.4% | 0.4% | 3.3% | 26.6% |
| S12N12 | 1.7% | 0.0% | 10.8% | 2.1% | 3.5% | 10.4% | 3.7% | 0.0% | 0.9% | 31.5% |
| S12N13 | 1.2% | 0.0% | 0.3% | 1.3% | 0.4% | 5.5% | 4.9% | 0.3% | 1.0% | 1.8% |

(continued)

| | D1 | D2 | D3 | D4 | D5 | D6 | D7 | D8 | D9 | D10 |
|---|---|---|---|---|---|---|---|---|---|---|
| S12N15 | 0.5% | 0.0% | 1.5% | 2.0% | 0.8% | 1.6% | 1.3% | 0.0% | 0.0% | 4.1% |
| S12N16 | 0.0% | 0.8% | 1.9% | 1.3% | 3.7% | 1.9% | 6.3% | 0.7% | 1.2% | 3.4% |
| S12N18 | 0.0% | 0.8% | 3.5% | 4.6% | 0.0% | 1.7% | 1.1% | 1.1% | 0.2% | 4.1% |
| $S12^2N12$ | 2.6% | 0.1% | 2.2% | 0.5% | 1.6% | 2.4% | 1.9% | 3.4% | 0.8% | 3.4% |
| S14N6 | 29.1% | 8.8% | 66.7% | 3.0% | 48.0% | 59.9% | 15.7% | 9.2% | 27.2% | 41.9% |
| S14N7 | 25.7% | 4.5% | 64.2% | 4.5% | 59.0% | 90.8% | 36.3% | 3.1% | 50.1% | 55.0% |
| S14N8 | 56.7% | 3.7% | 55.2% | 3.2% | 65.6% | 80.6% | 10.8% | 1.6% | 24.8% | 43.8% |
| S14N9 | 39.0% | 7.4% | 51.6% | 4.0% | 44.9% | 65.8% | 29.8% | 2.3% | 23.7% | 54.2% |
| S14N11 | 14.3% | 3.5% | 30.9% | 3.8% | 19.8% | 20.1% | 17.0% | 2.0% | 15.6% | 21.4% |
| S14N12 | 9.6% | 2.1% | 13.5% | 12.6% | 11.3% | 17.0% | 6.6% | 1.3% | 8.0% | 6.9% |
| S14N13 | 6.0% | 1.1% | 25.9% | 14.5% | 12.9% | 5.2% | 11.7% | 1.4% | 4.8% | 11.7% |
| S14N15 | 1.1% | 0.0% | 3.6% | 1.7% | 0.3% | 0.1% | 0.8% | 0.0% | 0.5% | 3.3% |
| S14N16 | 1.8% | 0.1% | 1.2% | 1.4% | 0.6% | 0.5% | 2.7% | 0.4% | 0.8% | 3.0% |
| S14N18 | 1.8% | 0.1% | 1.5% | 2.2% | 0.6% | 0.7% | 0.4% | 0.3% | 0.3% | 2.6% |
| $S14^2N12$ | 0.1% | 0.0% | 0.2% | 0.2% | 0.9% | 0.1% | 0.1% | 0.1% | 0.1% | 0.2% |
| S15N6 | 37.7% | 4.9% | 33.2% | 1.3% | 45.9% | 37.4% | 2.7% | 3.5% | 30.7% | 18.4% |
| S15N7 | 66.1% | 4.5% | 80.8% | 1.2% | 25.2% | 46.1% | 27.8% | 2.2% | 15.9% | 22.1% |
| S15N8 | 98.3% | 2.2% | 17.0% | 1.6% | 94.7% | 6.9% | 18.0% | 2.9% | 10.8% | 12.9% |
| S15N9 | 96.3% | 1.1% | 15.4% | 1.8% | 22.3% | 61.9% | 9.4% | 1.1% | 18.5% | 12.8% |
| S15N11 | 11.9% | 3.3% | 23.2% | 1.0% | 12.7% | 7.8% | 5.0% | 1.1% | 9.2% | 2.7% |
| S15N12 | 4.8% | 0.1% | 7.5% | 1.6% | 4.3% | 9.6% | 4.7% | 0.3% | 4.4% | 5.1% |
| S15N13 | 1.5% | 0.1% | 3.0% | 0.8% | 1.5% | 0.8% | 5.2% | 0.2% | 2.0% | 5.4% |
| S15N15 | 0.3% | 0.2% | 2.6% | 3.5% | 0.7% | 0.8% | 1.8% | 0.1% | 0.2% | 2.4% |
| S15N16 | 3.2% | 1.4% | 7.6% | 4.8% | 1.4% | 0.2% | 0.5% | 0.4% | 2.7% | 6.1% |
| S15N18 | 3.8% | 0.3% | 8.5% | 5.1% | 1.7% | 1.9% | 0.8% | 0.4% | 0.3% | 4.2% |
| $S15^2N12$ | 0.3% | 0.4% | 1.7% | 0.2% | 0.9% | 2.9% | 1.8% | 2.9% | 0.4% | 14.3% |
| S16N6 | 54.2% | 23.6% | 96.0% | 2.6% | 63.2% | 96.4% | 16.5% | 18.2% | 85.7% | 86.9% |
| S16N7 | 65.1% | 10.0% | 85.0% | 0.8% | 82.6% | 88.3% | 7.8% | 17.7% | 97.9% | 29.6% |
| S16N8 | 40.3% | 19.1% | 97.7% | 1.3% | 72.2% | 91.5% | 6.5% | 17.8% | 71.3% | 70.4% |
| S16N9 | 26.4% | 7.2% | 92.8% | 1.0% | 68.1% | 76.1% | 9.0% | 2.1% | 54.4% | 49.2% |
| S16N11 | 19.8% | 4.1% | 73.8% | 0.5% | 45.0% | 63.0% | 8.3% | 3.6% | 68.4% | 37.8% |
| S16N12 | 3.0% | 1.2% | 1.7% | 0.1% | 3.4% | 2.0% | 1.3% | 0.9% | 12.4% | 9.2% |
| S16N13 | 0.9% | 0.0% | 0.4% | 0.1% | 0.7% | 0.5% | 2.9% | 0.1% | 1.4% | 7.1% |
| S16N15 | 1.4% | 0.2% | 0.0% | 0.1% | 0.1% | 0.1% | 1.1% | 0.0% | 0.2% | 2.6% |
| S16N16 | 0.9% | 0.0% | 1.1% | 3.6% | 0.1% | 0.1% | 1.6% | 0.0% | 0.1% | 2.8% |
| S16N18 | 0.5% | 0.0% | 14.1% | 6.3% | 0.2% | 0.0% | 1.5% | 0.0% | 0.1% | 3.0% |
| $S16^2N12$ | 0.0% | 0.7% | 0.2% | 0.3% | 0.1% | 1.0% | 0.8% | 0.0% | 2.7% | 0.2% |
| S18N6 | 42.6% | 0.4% | 58.9% | 1.5% | 73.2% | 74.8% | 19.5% | 1.0% | 55.4% | 58.8% |

(continued)

| | D1 | D2 | D3 | D4 | D5 | D6 | D7 | D8 | D9 | D10 |
|---|---|---|---|---|---|---|---|---|---|---|
| S18N7 | 63.0% | 1.0% | 97.2% | 0.3% | 67.7% | 87.5% | 6.5% | 3.9% | 86.6% | 45.8% |
| S18N8 | 38.0% | 3.1% | 93.3% | 7.8% | 33.4% | 74.5% | 9.8% | 0.5% | 25.8% | 16.8% |
| S18N9 | 30.3% | 0.4% | 72.3% | 0.2% | 11.6% | 40.1% | 1.9% | 0.2% | 7.6% | 8.9% |
| S18N11 | 1.3% | 0.5% | 30.9% | 0.9% | 1.1% | 5.7% | 10.7% | 1.9% | 4.3% | 4.5% |
| S18N12 | 2.3% | 2.3% | 5.2% | 0.7% | 1.7% | 2.6% | 0.3% | 0.4% | 1.7% | 2.0% |
| S18N13 | 0.2% | 0.0% | 1.0% | 0.7% | 1.4% | 1.0% | 1.5% | 0.0% | 0.0% | 2.9% |
| S18N15 | 0.5% | 0.0% | 0.1% | 1.6% | 0.1% | 0.0% | 0.3% | 0.0% | 0.0% | 0.2% |
| S18N16 | 0.1% | 0.0% | 0.5% | 0.4% | 0.1% | 0.0% | 0.3% | 0.0% | 0.1% | 0.4% |
| S18N18 | 0.0% | 0.0% | 0.1% | 0.2% | 0.0% | 0.0% | 0.0% | 0.0% | 0.1% | 0.1% |
| $S18^2N12$ | 0.0% | 0.0% | 0.1% | 0.3% | 0.0% | 0.1% | 0.0% | 0.0% | 0.0% | 0.0% |
| S6S6 | 0.7% | 2.2% | 1.9% | 0.9% | 4.3% | 12.6% | 19.5% | 0.0% | 8.0% | 14.4% |
| S6S7 | 1.0% | 0.6% | 1.3% | 1.2% | 10.0% | 11.3% | 22.9% | 1.5% | 6.3% | 19.7% |
| S6S8 | 0.7% | 5.0% | 1.6% | 0.5% | 8.7% | 21.1% | 18.9% | 0.2% | 3.0% | 6.6% |
| S6S9 | 2.3% | 6.3% | 2.6% | 0.7% | 8.3% | 10.6% | 4.2% | 5.0% | 5.6% | 14.7% |
| S6S10 | 0.7% | 4.6% | 2.5% | 0.3% | 10.1% | 31.4% | 2.7% | 4.2% | 23.7% | 22.4% |
| S6S11 | 0.4% | 7.4% | 0.7% | 0.0% | 21.1% | 15.7% | 0.5% | 1.5% | 8.5% | 15.9% |
| S6S12 | 0.6% | 1.5% | 1.0% | 0.2% | 14.0% | 24.7% | 8.1% | 11.1% | 1.0% | 41.2% |
| S6S14 | 0.8% | 6.6% | 0.3% | 5.3% | 45.3% | 62.8% | 3.8% | 6.8% | 35.8% | 54.4% |
| S6S15 | 1.2% | 6.9% | 12.0% | 2.0% | 29.7% | 47.0% | 1.3% | 2.8% | 6.5% | 40.0% |
| S6S16 | 0.9% | 16.0% | 16.9% | 5.1% | 60.5% | 34.4% | 2.3% | 43.1% | 38.2% | 85.5% |
| S6S18 | 15.9% | 12.1% | 36.8% | 8.8% | 55.1% | 83.3% | 9.5% | 12.6% | 62.8% | 72.8% |
| S7S7 | 14.9% | 4.9% | 12.2% | 13.5% | 10.5% | 29.3% | 18.4% | 5.0% | 18.3% | 19.3% |
| S7S8 | 16.7% | 35.6% | 2.7% | 5.9% | 16.6% | 6.3% | 42.1% | 5.0% | 43.7% | 32.9% |
| S7S9 | 8.1% | 37.7% | 12.6% | 3.7% | 20.6% | 14.3% | 13.5% | 18.6% | 35.3% | 33.5% |
| S7S10 | 5.6% | 32.4% | 3.3% | 2.7% | 12.2% | 18.1% | 1.7% | 11.4% | 47.1% | 55.8% |
| S7S11 | 4.1% | 24.2% | 11.4% | 8.0% | 42.0% | 68.6% | 4.5% | 23.1% | 80.0% | 60.8% |
| S7S12 | 3.1% | 19.2% | 5.4% | 9.2% | 48.8% | 93.8% | 5.8% | 20.7% | 26.4% | 80.4% |
| S7S14 | 3.1% | 11.0% | 14.6% | 8.3% | 63.7% | 59.7% | 4.1% | 21.2% | 34.5% | 88.7% |
| S7S15 | 3.9% | 17.5% | 12.0% | 6.7% | 58.7% | 90.0% | 5.4% | 25.2% | 9.6% | 90.4% |
| S7S16 | 6.9% | 13.9% | 11.6% | 8.8% | 49.2% | 90.8% | 10.7% | 22.7% | 9.2% | 73.7% |
| S7S18 | 4.1% | 5.1% | 7.9% | 4.5% | 3.8% | 22.0% | 7.1% | 26.7% | 34.3% | 90.5% |
| S8S8 | 11.7% | 54.5% | 26.1% | 9.9% | 45.2% | 45.6% | 25.3% | 48.2% | 53.3% | 50.8% |
| S8S9 | 7.4% | 84.9% | 11.6% | 14.3% | 36.1% | 59.9% | 11.1% | 47.2% | 85.2% | 37.6% |
| S8S10 | 8.6% | 38.8% | 12.0% | 9.2% | 65.6% | 70.2% | 7.6% | 40.9% | 79.4% | 74.2% |
| S8S11 | 3.0% | 43.3% | 4.9% | 10.9% | 87.0% | 92.3% | 3.2% | 22.2% | 97.6% | 67.0% |
| S8S12 | 1.4% | 1.1% | 3.5% | 1.0% | 47.4% | 61.9% | 1.3% | 67.9% | 23.6% | 67.9% |
| S8S14 | 0.7% | 0.4% | 3.4% | 3.1% | 52.7% | 79.5% | 1.3% | 3.4% | 13.5% | 60.5% |
| S8S15 | 0.4% | 0.0% | 0.9% | 2.6% | 40.0% | 45.0% | 0.0% | 8.0% | 22.0% | 64.0% |

(continued)

| | D1 | D2 | D3 | D4 | D5 | D6 | D7 | D8 | D9 | D10 |
|---|---|---|---|---|---|---|---|---|---|---|
| S8S16 | 2.2% | 0.2% | 37.3% | 2.1% | 20.5% | 61.0% | 0.5% | 4.4% | 1.5% | 98.3% |
| S8S18 | 3.9% | 0.0% | 61.5% | 2.0% | 27.5% | 19.1% | 3.8% | 4.2% | 28.1% | 59.6% |
| S9S9 | 0.8% | 3.1% | 4.5% | 2.1% | 37.1% | 33.0% | 3.6% | 14.8% | 19.3% | 34.9% |
| S9S10 | 5.0% | 2.5% | 70.1% | 4.1% | 61.4% | 74.6% | 2.8% | 9.8% | 46.6% | 47.2% |
| S9S11 | 3.0% | 3.3% | 1.9% | 4.1% | 48.6% | 85.1% | 2.5% | 24.4% | 49.7% | 88.8% |
| S9S12 | 0.8% | 0.0% | 5.3% | 0.6% | 57.5% | 61.6% | 3.4% | 13.8% | 60.9% | 82.9% |
| S9S14 | 1.6% | 0.3% | 2.3% | 0.7% | 32.3% | 80.0% | 1.5% | 11.6% | 68.8% | 91.0% |
| S9S15 | 0.0% | 0.0% | 2.5% | 2.1% | 41.3% | 87.2% | 1.2% | 5.4% | 40.4% | 94.8% |
| S9S16 | 0.0% | 0.0% | 25.3% | 1.2% | 47.8% | 71.5% | 1.8% | 11.2% | 41.4% | 66.4% |
| S9S18 | 2.7% | 0.0% | 35.8% | 0.6% | 22.0% | 11.6% | 1.9% | 12.4% | 4.0% | 43.6% |
| S10S10 | 0.7% | 0.4% | 5.0% | 2.5% | 45.9% | 59.1% | 2.0% | 13.8% | 51.3% | 87.1% |
| S10S11 | 1.2% | 0.6% | 4.4% | 0.0% | 61.1% | 83.8% | 1.8% | 8.3% | 60.6% | 93.7% |
| S10S12 | 0.0% | 0.0% | 7.6% | 3.4% | 33.5% | 82.4% | 0.0% | 5.4% | 53.7% | 91.0% |
| S10S14 | 0.1% | 0.2% | 0.6% | 0.2% | 12.9% | 48.6% | 1.3% | 7.5% | 5.7% | 37.7% |
| S10S15 | 0.0% | 0.7% | 19.6% | 0.7% | 9.4% | 24.6% | 0.0% | 11.1% | 3.8% | 19.4% |
| S10S16 | 0.1% | 0.3% | 1.7% | 0.8% | 13.6% | 41.6% | 0.7% | 4.0% | 19.9% | 47.4% |
| S10S18 | 3.5% | 0.2% | 21.5% | 6.2% | 3.5% | 19.9% | 0.3% | 8.0% | 2.4% | 54.6% |
| S11S11 | 12.9% | 0.0% | 3.6% | 0.4% | 28.7% | 25.4% | 0.0% | 1.4% | 8.0% | 13.7% |
| S11S12 | 4.7% | 0.0% | 3.2% | 0.4% | 9.2% | 21.5% | 0.4% | 6.7% | 4.2% | 10.5% |
| S11S14 | 1.9% | 0.0% | 17.9% | 0.2% | 9.1% | 30.0% | 0.7% | 4.2% | 9.9% | 38.5% |
| S11S15 | 0.0% | 0.0% | 5.3% | 0.9% | 17.4% | 15.4% | 0.7% | 2.1% | 3.6% | 75.9% |
| S11S16 | 2.4% | 0.1% | 0.7% | 0.2% | 5.1% | 9.3% | 0.5% | 0.9% | 16.4% | 89.2% |
| S11S18 | 2.7% | 0.1% | 11.6% | 1.4% | 3.8% | 6.0% | 1.1% | 3.3% | 0.3% | 92.0% |
| S12S12 | 0.6% | 0.0% | 11.0% | 36.3% | 3.1% | 2.6% | 1.3% | 3.8% | 1.3% | 12.8% |
| S12S14 | 0.0% | 0.2% | 6.3% | 14.0% | 3.6% | 4.6% | 0.9% | 1.3% | 2.5% | 30.4% |
| S12S15 | 4.1% | 0.0% | 9.7% | 3.3% | 1.4% | 0.0% | 0.0% | 3.2% | 0.9% | 43.1% |
| S12S16 | 0.0% | 0.0% | 0.1% | 6.3% | 0.1% | 1.2% | 0.0% | 0.0% | 0.0% | 13.2% |
| S12S18 | 0.0% | 0.0% | 0.2% | 5.1% | 0.0% | 0.1% | 0.0% | 0.0% | 0.3% | 7.7% |
| S14S14 | 0.0% | 0.0% | 0.1% | 0.8% | 0.0% | 0.2% | 2.8% | 0.0% | 0.0% | 1.5% |
| S14S15 | 0.0% | 0.0% | 0.0% | 0.1% | 0.0% | 0.1% | 0.4% | 0.0% | 0.0% | 0.5% |
| S14S16 | 0.0% | 0.0% | 0.2% | 0.0% | 0.2% | 0.9% | 1.8% | 0.0% | 0.0% | 0.8% |
| S14S18 | 0.0% | 0.0% | 0.1% | 0.8% | 1.0% | 0.2% | 2.7% | 0.3% | 0.6% | 1.5% |
| S15S15 | 0.5% | 0.0% | 0.2% | 1.5% | 0.3% | 0.9% | 0.5% | 0.2% | 0.5% | 0.3% |
| S15S16 | 0.7% | 0.7% | 0.2% | 3.2% | 0.2% | 0.4% | 0.4% | 0.0% | 0.5% | 0.6% |
| S15S18 | 0.0% | 0.5% | 1.9% | 0.0% | 0.4% | 0.0% | 9.3% | 0.5% | 0.0% | 1.8% |
| S16S16 | 0.4% | 0.2% | 0.8% | 2.2% | 0.7% | 0.5% | 0.0% | 1.7% | 0.2% | 2.7% |
| S16S18 | 2.1% | 0.0% | 1.8% | 0.3% | 0.3% | 1.8% | 1.8% | 1.9% | 0.5% | 3.0% |
| S18S18 | 0.0% | 0.1% | 0.1% | 0.1% | 0.1% | 0.5% | 0.0% | 0.0% | 0.1% | 0.0% |

(continued)

| | D1 | D2 | D3 | D4 | D5 | D6 | D7 | D8 | D9 | D10 |
|---|---|---|---|---|---|---|---|---|---|---|
| N6N6 | 0.0% | 0.0% | 0.3% | 0.1% | 0.0% | 0.0% | 0.0% | 0.0% | 0.2% | 0.0% |
| N6N7 | 0.1% | 0.4% | 0.0% | 0.0% | 0.4% | 0.1% | 0.0% | 0.0% | 0.0% | 0.0% |
| N6N8 | 0.0% | 0.1% | 0.4% | 0.0% | 0.0% | 0.0% | 0.1% | 0.0% | 0.0% | 0.0% |
| N6N9 | 0.0% | 0.0% | 0.4% | 0.3% | 0.1% | 0.2% | 0.0% | 0.0% | 0.0% | 0.3% |
| N6N11 | 0.1% | 0.0% | 0.9% | 0.0% | 0.5% | 0.0% | 0.0% | 0.1% | 0.0% | 0.0% |
| N6N12 | 0.2% | 0.0% | 0.5% | 0.0% | 0.0% | 0.1% | 0.0% | 0.1% | 0.0% | 0.0% |
| N6N13 | 0.0% | 0.0% | 1.0% | 0.6% | 0.0% | 0.1% | 0.9% | 0.0% | 0.0% | 0.0% |
| N6N15 | 0.0% | 0.1% | 0.3% | 0.0% | 0.0% | 0.3% | 0.0% | 0.0% | 0.0% | 0.7% |
| N6N16 | 0.0% | 0.1% | 2.8% | 0.0% | 0.1% | 0.4% | 0.5% | 0.0% | 0.0% | 0.8% |
| N6N18 | 3.9% | 0.9% | 3.9% | 0.2% | 0.0% | 0.1% | 0.3% | 0.0% | 0.2% | 0.1% |
| N6$^2$N12 | 0.0% | 0.0% | 0.1% | 0.0% | 0.0% | 0.1% | 0.3% | 0.0% | 0.0% | 0.0% |
| N7N7 | 0.0% | 0.0% | 0.0% | 0.0% | 0.5% | 0.0% | 0.0% | 0.0% | 0.0% | 0.0% |
| N7N8 | 0.0% | 0.3% | 3.7% | 0.0% | 0.0% | 0.0% | 0.0% | 0.0% | 0.2% | 0.6% |
| N7N9 | 0.0% | 0.0% | 0.1% | 0.0% | 0.2% | 0.0% | 0.0% | 0.0% | 0.0% | 0.1% |
| N7N11 | 0.0% | 0.0% | 1.3% | 0.1% | 0.1% | 1.5% | 0.0% | 0.0% | 0.0% | 0.2% |
| N7N12 | 0.1% | 0.0% | 6.3% | 0.6% | 0.0% | 0.2% | 0.0% | 0.0% | 0.0% | 0.8% |
| N7N13 | 0.4% | 0.0% | 7.8% | 0.3% | 0.9% | 2.3% | 0.0% | 0.1% | 0.1% | 1.6% |
| N7N15 | 1.3% | 0.1% | 3.8% | 0.0% | 0.0% | 1.2% | 0.0% | 0.2% | 0.7% | 2.5% |
| N7N16 | 0.5% | 0.1% | 1.3% | 0.0% | 0.0% | 5.0% | 0.4% | 0.0% | 0.0% | 3.1% |
| N7N18 | 2.3% | 0.0% | 0.1% | 0.1% | 0.1% | 1.1% | 0.0% | 0.2% | 0.5% | 73.8% |
| N7$^2$N12 | 3.5% | 0.9% | 6.8% | 3.7% | 2.4% | 4.8% | 1.9% | 2.7% | 5.4% | 2.0% |
| N8N8 | 0.0% | 0.0% | 0.4% | 0.2% | 0.0% | 0.0% | 0.0% | 0.0% | 0.0% | 0.0% |
| N8N9 | 0.8% | 0.3% | 0.5% | 0.0% | 0.0% | 0.0% | 0.0% | 0.0% | 0.0% | 0.2% |
| N8N11 | 0.0% | 0.0% | 4.2% | 0.2% | 0.4% | 0.1% | 0.0% | 0.1% | 0.0% | 0.2% |
| N8N12 | 0.5% | 0.0% | 0.5% | 0.0% | 0.9% | 0.1% | 0.0% | 0.0% | 0.0% | 2.3% |
| N8N13 | 0.0% | 0.1% | 2.7% | 0.2% | 0.0% | 0.0% | 0.0% | 0.2% | 0.0% | 1.9% |
| N8N15 | 0.7% | 0.0% | 7.6% | 0.0% | 0.2% | 0.0% | 0.0% | 0.3% | 0.1% | 3.3% |
| N8N16 | 4.6% | 0.2% | 13.8% | 2.3% | 0.3% | 1.5% | 0.3% | 0.2% | 0.5% | 1.5% |
| N8N18 | 6.4% | 7.0% | 39.9% | 29.2% | 0.9% | 4.1% | 9.3% | 2.8% | 0.0% | 5.0% |
| N8$^2$N12 | 0.2% | 1.4% | 0.9% | 0.2% | 0.4% | 0.9% | 0.0% | 2.0% | 0.4% | 2.7% |
| N9N9 | 0.5% | 0.1% | 0.2% | 0.6% | 0.1% | 0.4% | 0.3% | 0.6% | 0.2% | 0.8% |
| N9N11 | 2.2% | 0.3% | 1.8% | 0.0% | 1.1% | 0.5% | 0.8% | 0.4% | 0.7% | 0.0% |
| N9N12 | 0.5% | 0.8% | 4.3% | 2.4% | 3.8% | 0.2% | 0.0% | 0.0% | 0.1% | 0.4% |
| N9N13 | 2.2% | 0.6% | 4.6% | 0.5% | 0.2% | 0.4% | 0.3% | 0.0% | 2.5% | 2.8% |
| N9N15 | 2.2% | 0.2% | 4.7% | 0.6% | 1.8% | 1.7% | 0.8% | 1.4% | 1.1% | 1.7% |
| N9N16 | 1.5% | 0.7% | 4.3% | 4.5% | 0.0% | 6.2% | 1.1% | 0.0% | 1.1% | 1.2% |
| N9N18 | 5.1% | 25.3% | 4.7% | 1.3% | 0.9% | 2.5% | 0.6% | 0.3% | 3.1% | 1.9% |
| N9$^2$N12 | 4.7% | 1.3% | 2.3% | 0.4% | 1.8% | 0.8% | 0.2% | 0.3% | 3.9% | 2.9% |

(continued)

| | D1 | D2 | D3 | D4 | D5 | D6 | D7 | D8 | D9 | D10 |
|---|---|---|---|---|---|---|---|---|---|---|
| N11N11 | 4.3% | 1.4% | 1.4% | 4.1% | 0.4% | 1.1% | 5.5% | 1.5% | 0.3% | 1.2% |
| N11N12 | 0.3% | 0.5% | 2.7% | 1.2% | 8.0% | 8.1% | 2.4% | 2.8% | 0.3% | 0.4% |
| N11N13 | 5.9% | 0.1% | 3.6% | 0.9% | 0.2% | 0.2% | 0.9% | 0.6% | 7.1% | 0.0% |
| N11N15 | 0.6% | 0.7% | 2.7% | 2.8% | 0.8% | 0.3% | 0.0% | 0.5% | 0.3% | 1.6% |
| N11N16 | 0.6% | 1.0% | 3.6% | 0.0% | 2.5% | 1.4% | 0.7% | 0.4% | 0.9% | 1.1% |
| N11N18 | 7.2% | 0.8% | 2.8% | 2.3% | 0.0% | 0.0% | 4.0% | 1.2% | 1.7% | 1.6% |
| N11$^2$N12 | 1.4% | 0.3% | 3.8% | 2.5% | 1.4% | 0.5% | 0.9% | 1.5% | 0.3% | 1.3% |
| N12N12 | 0.2% | 1.0% | 3.8% | 2.2% | 0.8% | 0.8% | 0.6% | 0.3% | 1.5% | 0.6% |
| N12N13 | 1.6% | 0.0% | 3.1% | 0.0% | 0.0% | 1.0% | 0.3% | 0.0% | 1.8% | 0.2% |
| N12N15 | 1.0% | 0.4% | 6.5% | 1.3% | 3.1% | 2.3% | 1.4% | 0.9% | 0.9% | 1.2% |
| N12N16 | 4.4% | 0.0% | 3.3% | 0.7% | 1.4% | 1.9% | 0.3% | 0.9% | 0.7% | 0.6% |
| N12N18 | 9.4% | 0.9% | 13.4% | 1.9% | 1.9% | 4.8% | 1.4% | 0.5% | 0.7% | 1.0% |
| N12$^2$N12 | 2.8% | 0.3% | 5.8% | 2.6% | 2.2% | 1.2% | 2.0% | 3.2% | 0.7% | 0.9% |
| N13N13 | 0.4% | 0.9% | 3.5% | 3.3% | 1.7% | 0.2% | 1.1% | 0.3% | 0.2% | 2.9% |
| N13N15 | 1.4% | 1.4% | 4.7% | 0.0% | 0.8% | 0.0% | 3.7% | 0.0% | 0.5% | 0.4% |
| N13N16 | 1.5% | 1.4% | 1.6% | 4.0% | 0.4% | 3.8% | 0.6% | 3.3% | 1.4% | 1.1% |
| N13N18 | 13.8% | 0.0% | 21.5% | 3.1% | 0.7% | 14.7% | 1.3% | 0.0% | 0.8% | 6.1% |
| N13$^2$N12 | 4.7% | 0.5% | 7.3% | 4.5% | 4.9% | 3.9% | 2.9% | 2.3% | 2.3% | 0.0% |
| N15N15 | 0.0% | 0.0% | 3.8% | 0.8% | 0.0% | 0.0% | 0.0% | 0.0% | 0.0% | 4.2% |
| N15N16 | 3.6% | 0.0% | 6.1% | 1.6% | 0.0% | 1.3% | 0.0% | 0.0% | 1.7% | 0.0% |
| N15N18 | 0.0% | 0.0% | 8.6% | 1.9% | 0.0% | 8.6% | 8.3% | 0.0% | 0.0% | 7.4% |
| N15$^2$N12 | 0.0% | 0.0% | 0.0% | 2.9% | 3.7% | 9.0% | 0.0% | 0.9% | 0.0% | 3.5% |
| N16N16 | 9.5% | 0.0% | 2.8% | 0.0% | 0.0% | 0.0% | 7.9% | 1.6% | 0.0% | 0.0% |
| N16N18 | 0.0% | 0.0% | 20.8% | 1.8% | 0.0% | 4.4% | 0.0% | 1.0% | 0.0% | 1.8% |
| N16$^2$N12 | 3.4% | 0.0% | 4.0% | 0.0% | 0.0% | 0.0% | 3.0% | 4.3% | 0.0% | 0.0% |
| N18N18 | 2.2% | 0.0% | 40.0% | 0.0% | 0.0% | 4.4% | 0.0% | 0.0% | 0.0% | 0.0% |
| N18$^2$N12 | 0.4% | 0.1% | 3.4% | 1.2% | 0.7% | 2.1% | 0.7% | 2.3% | 0.6% | 2.2% |
| $^2$N12$^2$N12 | 0.1% | 1.9% | 0.0% | 2.6% | 2.7% | 0.2% | 0.3% | 0.0% | 0.0% | 3.3% |
| Lipofect amine2000 | 42.7% | | | | | | | | | |

**Example 6: Transfection of Hela cell line with amino lipid compounds as DNA vectors**

Cell line: Hela cells (ATCC 35241)
Medium: DMEM supplemented with 10% fetal bovine serum (Invitrogen)
Screening: cell transfection on 96-well plates
Detection (readout): percentage of the number of GFP fluorescent cells relative to the total number of cells (the total number of cells was determined using nucleus dye Hoechst). Lipofectamine2000 (Invitrogen) was used as the positive control, according to the manufacturer's instructions.
Method: the experimental method was basically the same with Example 4.
Results: the absolute DNA transfection efficiency in Hela cells for 170 compounds is shown in Table 10.

Table 10: Absolute DNA transfection efficiency in Hela cells for 170 compounds.

| | D1 | D2 | D3 | D4 | D5 | D6 | D7 | D8 | D9 | D10 |
|---|---|---|---|---|---|---|---|---|---|---|
| S8N11 | 21.12% | 12.7% | 26.2% | 7.6% | 26.5% | 50.1% | 12.2% | 6.8% | 31.9% | 23.4% |
| S8N12 | 11.5% | 19.3% | 8.8% | 3.81% | 20.8% | 39.3% | 4.7% | 8.4% | 24.4% | 22.6% |
| S8N13 | 9.7% | 9.83% | 15.7% | 1.8% | 14.7% | 27.6% | 4.5% | 6.6% | 13.9% | 9.5% |
| S9N9 | 17.7% | 12.0% | 27.4% | 8.9% | 23.9% | 30.3% | 24.8% | 7.4% | 25.6% | 17.6% |
| S9N11 | 17.7% | 8.2% | 21.8% | 4.4% | 22.5% | 27.7% | 7.8% | 13.9% | 24.3% | 21.2% |
| S9N12 | 19.7% | 6.5% | 23.7% | 4.1% | 24.1% | 35.5% | 6.8% | 6.8% | 14.4% | 12.9% |
| S11N7 | 13.9% | 5.8% | 9.8% | 2.3% | 11.4% | 17.0% | 5.8% | 6.7% | 8.3% | 14.7% |
| S11N8 | 9.9% | 9.3% | 12.9% | 2.9% | 9.6% | 18.4% | 5.7% | 6.1% | 11.7% | 16.7% |
| S11N9 | 6.0% | 6.8% | 11.5% | 0.5% | 10.6% | 20.3% | 0.9% | 4.3% | 11.8% | 16.2% |
| S7S11 | 5.9% | 13.6% | 4.8% | 7.9% | 16.8% | 14.5% | 5.2% | 5.6% | 12.9% | 19.7% |
| S7S12 | 4.2% | 9.2% | 4.9% | 4.7% | 4.9% | 5.8% | 3.5% | 5.4% | 2.9% | 10.6% |
| S8S11 | 4.59% | 8.9% | 3.3% | 4.5% | 5.9% | 3.9% | 5.7% | 7.6% | 6.8% | 21.8% |
| S8S12 | 5.4% | 8.7% | 5.5% | 5.4% | 12.4% | 6.3% | 4.6% | 17.2% | 1.7% | 11.7% |
| S9S12 | 7.9% | 8.5% | 2.4% | 0.0% | 10.8% | 10.9% | 2.7% | 15.8% | 19.6% | 8.9% |
| S9S14 | 1.4% | 7.3% | 2.4% | 3.6% | 7.3% | 9.6% | 6.5% | 10.8% | 14.8% | 7.2% |
| S9S15 | 1.3% | 5.9% | 1.4% | 5.8% | 4.4% | 23.7% | 2.8% | 11.1% | 12.6% | 28.7% |
| S9S16 | 0.0% | 0.7% | 0.0% | 6.2% | 11.7% | 27.8% | 4.2% | 11.6% | 6.9% | 28.6% |
| Lipofect amine2000 | 12.6% | | | | | | | | | |

**Example 7: Transfection of Hela cell line with amino lipid compounds as mRNA vectors**

Cell line: Hela cells (ATCC 35241)
Medium: DMEM supplemented with 10% fetal bovine serum (Invitrogen)
Screening: cell transfection on 96-well plates
Detection (readout): percentage of the number of GFP fluorescent cells relative to the total number of cells (the total number of cells was determined using nucleus dye Hoechst). A commercially available liposome transfection reagent Lipofectamine2000 (Invitrogen) was used as the positive control group, according to the manufacturer's instructions.
Method: the experimental method was basically the same with Example 4, and the mass of EGFP mRNA for transfection was 50 ng per well.
Results: the absolute mRNA transfection efficiency in Hela cells for 170 compounds is shown in Table 11.

Table 11: Absolute mRNA transfection efficiency in Hela cells for 170 compounds.

| | D1 | D2 | D3 | D4 | D5 | D6 | D7 | D8 | D9 | D10 |
|---|---|---|---|---|---|---|---|---|---|---|
| S8N11 | 21.2% | 12.7% | 26.2% | 7.6% | 26.5% | 50.2% | 12.3% | 6.8% | 31.9% | 23.4% |
| S8N12 | 11.5% | 19.3% | 8.9% | 3.8% | 20.8% | 39.3% | 4.7% | 8.4% | 24.1% | 22.6% |
| S8N13 | 9.7% | 9.8% | 15.7% | 1.8% | 14.7% | 27.6% | 4.5% | 6.6% | 13.4% | 9.5% |
| S9N9 | 17.7% | 12.0% | 27.4% | 8.9% | 23.9% | 30.3% | 24.8% | 7.4% | 25.6% | 17.6% |
| S9N11 | 17.7% | 8.2% | 21.8% | 4.4% | 22.5% | 27.7% | 7.8% | 13.9% | 24.6% | 21.3% |
| S9N12 | 19.6% | 6.5% | 23.7% | 4.1% | 24.1% | 35.5% | 6.8% | 6.8% | 14.4% | 12.9% |
| S11N7 | 13.9% | 5.8% | 9.8% | 2.3% | 11.4% | 17.0% | 5.8% | 6.7% | 8.3% | 14.7% |

(continued)

| | D1 | D2 | D3 | D4 | D5 | D6 | D7 | D8 | D9 | D10 |
|---|---|---|---|---|---|---|---|---|---|---|
| S11N8 | 9.9% | 9.3% | 12.9% | 2.9% | 9.6% | 18.4% | 5.7% | 6.1% | 11.9% | 16.7% |
| S11N9 | 6.0% | 6.8% | 11.5% | 0.5% | 10.6% | 20.3% | 0.9% | 4.3% | 11.8% | 16.2% |
| S7S11 | 5.9% | 13.6% | 4.8% | 7.9% | 16.8% | 14.5% | 5.2% | 5.6% | 12.9% | 19.7% |
| S7S12 | 4.1% | 9.2% | 4.9% | 4.7% | 4.9% | 5.8% | 3.5% | 5.4% | 2.9% | 10.6% |
| S8S11 | 4.9% | 8.6% | 3.3% | 4.5% | 5.9% | 3.9% | 5.7% | 7.6% | 6.8% | 21.8% |
| S8S12 | 5.4% | 8.7% | 5.5% | 5.4% | 12.4% | 6.3% | 4.6% | 17.2% | 1.7% | 11.7% |
| S9S12 | 7.9% | 8.5% | 2.4% | 0.0% | 10.8% | 10.9% | 2.7% | 15.8% | 19.6% | 8.9% |
| S9S14 | 1.4% | 7.3% | 2.5% | 3.6% | 7.32% | 9.6% | 6.2% | 10.8% | 14.8% | 7.5% |
| S9S15 | 1.3% | 5.9% | 1.4% | 5.8% | 4.4% | 23.7% | 2.8% | 11.1% | 12.6% | 28.7% |
| S9S16 | 0.0% | 0.7% | 0.0% | 6.2% | 11.7% | 27.8% | 4.2% | 11.6% | 6.9% | 28.6% |
| Lipofect amine2000 | 21.5% | | | | | | | | | |

**Example 8: Transfection of MCF7 cell line with amino lipid compounds as DNA vectors**

Cell line: MCF7 cells (ATCC HTB-22)
Medium: DMEM supplemented with 10% fetal bovine serum
Screening: cell transfection on 96-well plates
Detection (readout): percentage of the number of GFP fluorescent cells relative to the total number of cells (the total number of cells was determined using nucleus dye Hoechst). Lipofectamine2000 was used as the positive control, according to the manufacturer's instructions.
Method: the experimental method was basically the same with Example 4.
Results: the absolute DNA transfection efficiency in MCF7 cells for 102 compounds is shown in Table 12.

Table 12: Absolute DNA transfection efficiency in MCF7 cells for 102 compounds.

| | D3 | D5 | D6 | D8 | D9 | D10 |
|---|---|---|---|---|---|---|
| S8N11 | 3.6% | 16.7% | 18.0% | 4.4% | 45.4% | 45.3% |
| S8N12 | 13.7% | 29.2% | 25.5% | 2.1% | 32.0% | 40.6% |
| S8N13 | 19.9% | 24.5% | 32.2% | 0.9% | 25.8% | 41.7% |
| S9N9 | 8.6% | 21.2% | 11.0% | 7.8% | 42.3% | 38.9% |
| S9N11 | 26.6% | 17.8% | 32.6% | 13.1% | 27.3% | 36.6% |
| S9N12 | 27.5% | 13.7% | 21.8% | 6.2% | 20.0% | 31.7% |
| S11N7 | 4.4% | 4.7% | 16.2% | 1.7% | 5.8% | 10.1% |
| S11N8 | 17.7% | 20.1% | 9.3% | 0.1% | 20.9% | 25.6% |
| S11N9 | 13.6% | 23.1% | 25.1% | 0.9% | 33.5% | 48.7% |
| S7S11 | 6.4% | 23.3% | 38.1% | 12.9% | 44.5% | 33.8% |
| S7S12 | 3.0% | 27.1% | 52.1% | 11.5% | 14.6% | 44.7% |
| S8S11 | 2.7% | 48.3% | 56.8% | 12.3% | 59.8% | 37.2% |
| S8S12 | 1.9% | 26.3% | 34.4% | 37.7% | 13.1% | 37.7% |
| S9S12 | 3.0% | 32.0% | 34.2% | 7.7% | 33.8% | 46.0% |
| S9S14 | 1.3% | 17.9% | 44.4% | 6.4% | 38.2% | 50.5% |
| S9S15 | 1.4% | 23.0% | 48.4% | 3.0% | 22.5% | 58.8% |

(continued)

| | D3 | D5 | D6 | D8 | D9 | D10 |
|---|---|---|---|---|---|---|
| S9S16 | 14.1% | 26.6% | 39.7% | 6.2% | 23.0% | 36.9% |
| Lipofectamine2000 | 11.7% | | | | | |

**Example 9: Transfection of MCF7 cell line with amino lipid compounds as mRNA vectors**

Cell line: MCF7 cells (ATCC HTB-22)
Medium: DMEM supplemented with 10% fetal bovine serum (Invitrogen)
Screening: cell transfection on 96-well plates
Detection (readout): percentage of the number of GFP fluorescent cells relative to the total number of cells (the total number of cells was determined using nucleus dye Hoechst). Lipofectamine2000 (Invitrogen) was used as the positive control.
Method: the experimental method was basically the same with Example 4, and the mass of EGFP mRNA (TriLink) for transfection was 50 ng per well.
Results: the absolute mRNA transfection efficiency in MCF7 cells for 102 compounds is shown in Table 13.

Table 13: Absolute mRNA transfection efficiency in MCF7 cells for 102 compounds.

| | D3 | D5 | D6 | D8 | D9 | D10 |
|---|---|---|---|---|---|---|
| S8N11 | 8.0% | 9.1% | 6.7% | 0.1% | 6.6% | 4.4% |
| S8N12 | 20.7% | 19.7% | 22.5% | 0.3% | 16.4% | 23.1% |
| S8N13 | 18.1% | 9.2% | 33.4% | 0.4% | 13.3% | 23.0% |
| S9N9 | 12.1% | 8.3% | 30.7% | 2.6% | 21.0% | 29.6% |
| S9N11 | 7.9% | 18.0% | 37.2% | 2.7% | 11.8% | 20.2% |
| S9N12 | 15.9% | 16.6% | 22.4% | 0.7% | 16.4% | 11.2% |
| S11N7 | 5.8% | 3.4% | 4.2% | 3.2% | 5.1% | 9.0% |
| S11N8 | 8.6% | 21.2% | 11.0% | 7.8% | 42.3% | 38.9% |
| S11N9 | 26.6% | 17.8% | 32.6% | 13.1% | 27.3% | 36.6% |
| S7S11 | 6.7% | 16.5% | 26.1% | 1.5% | 3.6% | 22.2% |
| S7S12 | 9.4% | 33.6% | 19.1% | 24.0% | 21.2% | 47.5% |
| S8S11 | 20.4% | 30.6% | 46.3% | 7.0% | 34.9% | 40.4% |
| S8S12 | 6.7% | 32.6% | 50.0% | 14.0% | 5.3% | 50.2% |
| S9S12 | 6.5% | 27.3% | 50.4% | 12.6% | 5.1% | 40.9% |
| S9S14 | 4.4% | 2.1% | 12.2% | 14.8% | 19.1% | 50.3% |
| S9S15 | 1.1% | 27.0% | 47.3% | 13.5% | 27.6% | 49.4% |
| S9S16 | 3.0% | 32.0% | 34.2% | 7.7% | 33.8% | 46.0% |
| Lipofectamine2000 | 17.5% | | | | | |

**Example 10: Screening of amino lipid compounds as DNA vectors in cell lines that are difficult to be transfected (embryonic stem cells)**

Cell line: embryonic stem cells (hESC2, cultured according to reported methods: Stem Cells, 2005, 23, 544-549)
Medium: mTeSR1 (STEMCELL)
Mode of screening: cell transfection on 96-well plates
Detection (readout): percentage of the number of GFP fluorescent cells relative to the total number of cells. Lipofectamine

Stem was used as the positive control.
Method: an 8-channel pipette was used for sample addition. The contents shown were for a single well of a 96-well flat plate.

1. Stem cells were cultured to 3-5 $\times 10^4$ cells per well in a 96-well plate treated with matrigel.
2. The compound (0.01 mmol) prepared in Example 1 was dissolved in 1 mL of anhydrous ethanol. After ultrasonically dissolved, 10 $\mu$L of the resultant amino lipid solution in ethanol was taken and mixed with 5 $\mu$L of DOPE solution in ethanol (0.01 m). The obtained mixture was then added with 35 $\mu$L of 0.2 M sodium acetate buffer (pH 5.6), and vortexed at a constant rotating speed for 30s. Then, 4 $\mu$L was taken from the obtained product and added to 46 $\mu$L of 0.02 M sodium acetate buffer (pH 5.6), and vortexed at a constant rotating speed for 30s to form a liposome solution. 5 $\mu$L of this liposome solution was mixed with 75 ng of plasmid DNA (pSin-EGFP-IRES-Puro) dissolved in 5 $\mu$L of 0.02 M sodium acetate buffer, and allowed to stay at room temperature for 30 min to form a lipid /DNA transfection complex.
3. 10 $\mu$L of the lipid /DNA transfection complex was incubated at room temperature for 30 min, followed by addition of 90 $\mu$L mTeSR1 medium was added and mixing with a pipette. 100 $\mu$L of the mixture was added to separate wells of a 96-well culture plate and placed in an incubator containing 5% $CO_2$ at 37°C.
4. 48 hours after transfection, the cells were digested with 0.05% Trypsin-EDTA. The cells were resuspended in a PBS buffer and filtered. The cell suspension was placed in a flow cytometer and the number of GFP-expressing cells in no less than 2000 cells was measured so as to obtain the absolute efficiency of cell transfection. The relative efficiency of cell transfection was obtained by comparing the cell transfection efficiency with the positive control Lipofectamine Stem.

Results: the absolute DNA transfection efficiency in embryonic stem cells for 60 compounds is shown in Table 14.

Table 14: Absolute DNA transfection efficiency in embryonic stem cells for 60 compounds.

| | D3 | D5 | D6 | D8 | D9 | D10 |
|---|---|---|---|---|---|---|
| S8N11 | 0.2% | 1.1% | 0.2% | 0.7% | 0.5% | 0.7% |
| S9N11 | 0.4% | 3.0% | 1.4% | 0.6% | 6.6% | 2.2% |
| S9N12 | 2.3% | 2.5% | 1.9% | 1.1% | 2.7% | 5.3% |
| S11N8 | 4.3% | 4.0% | 1.7% | 0.0% | 3.6% | 12.6% |
| S11N9 | 3.7% | 0.5% | 1.0% | 0.8% | 1.4% | 0.5% |
| S8S11 | 0.7% | 2.4% | 1.6% | 1.0% | 1.0% | 0.7% |
| S8S12 | 1.5% | 1.7% | 3.3% | 5.9% | 2.9% | 2.6% |
| S9S12 | 5.3% | 4.6% | 10.5% | 11.1% | 7.6% | 14.0% |
| S9S14 | 1.6% | 0.7% | 2.0% | 0.7% | 2.5% | 0.7% |
| S9S15 | 1.8% | 6.2% | 3.7% | 1.9% | 21.6% | 16.8% |
| Lipofectamine Stem | 7.8% | | | | | |

**Example 11: Screening of amino lipid compounds as mRNA vectors in cell lines that are difficult to be transfected (embryonic stem cells)**

Cell line: embryonic stem cells (hESC2, cultured according to reported methods: Stem Cells, 2005, 23, 544-549)
Medium: mTeSR1 (STEMCELL)
Mode of screening: cell transfection on 96-well plate
Detection (readout): percentage of the number of GFP fluorescent cells relative to the total number of cells. Lipofectamine Stem was used as the positive control.
Method: the experimental method was basically the same with Example 10, and the mass of EGFP mRNA for transfection was 50 ng per well.
Results: the absolute mRNA transfection efficiency in embryonic stem cells for 60 compounds is shown in Table 15.

Table 15: Absolute mRNA transfection efficiency in embryonic stem cells for 60 compounds.

| | D3 | D5 | D6 | D8 | D9 | D10 |
|---|---|---|---|---|---|---|
| S8N11 | 2.4% | 1.6% | 1.9% | 1.0% | 1.0% | 0.7% |
| S9N11 | 1.7% | 3.3% | 5.8% | 5.9% | 2.9% | 2.6% |
| S9N12 | 4.6% | 10.5% | 6.1% | 11.1% | 7.6% | 14.0% |
| S11N8 | 12.8% | 6.9% | 2.0% | 1.3% | 34.8% | 35.4% |
| S11N9 | 22.0% | 24.9% | 0.7% | 3.2% | 33.5% | 39.3% |
| S8S11 | 21.4% | 21.1% | 10.3% | 1.6% | 18.1% | 37.4% |
| S8S12 | 17.9% | 24.9% | 7.1% | 0.7% | 16.3% | 24.6% |
| S9S12 | 21.4% | 27.3% | 9.3% | 1.7% | 20.6% | 25.4% |
| S9S14 | 19.7% | 22.5% | 1.7% | 0.3% | 16.4% | 23.1% |
| S9S15 | 9.2% | 33.4% | 2.5% | 0.4% | 13.3% | 23.0% |
| Lipofectamine Stem | 12.8% | | | | | |

**Example 12: Screening of amino lipid compounds as mRNA vectors in cell lines that are difficult to be transfected (cardiomyocytes)**

Cell line: cardiomyocytes (obtained from induced differentiation of hESC according to reported methods: J. Mol. Cell. Cardiol. 2011, 51, 288-298)

Medium: DMEM supplemented with 10% fetal bovine serum (Invitrogen)

Mode of screening: cell transfection on 96-well plates

Detection (readout): percentage of the number of GFP fluorescent cells relative to the total number of cells (the total number of cells was determined using nucleus dye Hoechst). Lipofectamine Stem (Invitrogen) was used as the positive control, according to the manufacturer's instructions.

Method: the experimental method was basically the same with Example 4.

Results: the absolute DNA transfection efficiency in cardiomyocytes for 60 compounds is shown in Table 16.

Table 16: Absolute DNA transfection efficiency in cardiomyocytes for 60 compounds.

| | D3 | D5 | D6 | D8 | D9 | D10 |
|---|---|---|---|---|---|---|
| S8N11 | 0.5% | 3.0% | 1.4% | 2.1% | 0.6% | 6.6% |
| S9N11 | 0.4% | 2.5% | 1.9% | 0.8% | 1.1% | 2.7% |
| S9N12 | 1.1% | 4.0% | 1.7% | 1.5% | 0.0% | 3.6% |
| S11N8 | 0.2% | 9.1% | 6.7% | 1.2% | 0.1% | 6.6% |
| S11N9 | 0.3% | 19.7% | 22.5% | 1.7% | 0.3% | 16.4% |
| S8S11 | 1.9% | 0.5% | 1.0% | 2.2% | 0.8% | 1.4% |
| S8S12 | 0.3% | 2.4% | 1.6% | 1.9% | 1.0% | 1.0% |
| S9S12 | 2.7% | 1.7% | 3.3% | 5.8% | 5.9% | 2.9% |
| S9S14 | 0.5% | 4.6% | 10.5% | 6.1% | 11.1% | 7.6% |
| S9S15 | 0.3% | 12.8% | 6.9% | 2.0% | 1.3% | 24.8% |
| Lipofectamine Stem | 11.2% | | | | | |

**Example 13: Screening of amino lipid compounds as mRNA vectors in cell lines that are difficult to be transfected (cardiomyocytes)**

Cell line: cardiomyocytes (obtained from induced differentiation of hESC according to reported methods: J. Mol. Cell. Cardiol. 2011, 51, 288-298)
Medium: DMEM supplemented with 10% fetal bovine serum (Invitrogen)
Mode of screening: cell transfection on 96-well plates
Detection (readout): percentage of the number of GFP fluorescent cells relative to the total number of cells (the total number of cells was determined using nucleus dye Hoechst). Lipofectamine Stem (Invitrogen) was used as the positive control, according to the manufacturer's instructions.
Method: the experimental method was basically the same with Example 4, and the mass of EGFP mRNA for transfection was 50 ng per well.
Results: the absolute mRNA transfection efficiency in cardiomyocytes for 60 compounds is shown in Table 17.

Table 17: Absolute mRNA transfection efficiency in cardiomyocytes for 60 compounds.

| | D3 | D5 | D6 | D8 | D9 | D10 |
|---|---|---|---|---|---|---|
| S8N11 | 6.2% | 3.7% | 5.3% | 1.9% | 31.6% | 16.8% |
| S9N11 | 16.7% | 18.0% | 5.0% | 4.4% | 45.4% | 45.3% |
| S9N12 | 29.2% | 25.5% | 5.3% | 2.1% | 32.0% | 40.6% |
| S11N8 | 24.5% | 32.2% | 7.9% | 0.9% | 25.8% | 41.7% |
| S11N9 | 8.3% | 30.7% | 8.7% | 2.6% | 21.0% | 29.6% |
| S8S11 | 21.0% | 2.7% | 18.4% | 0.7% | 25.5% | 20.8% |
| S8S12 | 36.7% | 2.5% | 44.9% | 0.7% | 14.0% | 25.6% |
| S9S12 | 65.7% | 1.2% | 9.5% | 0.9% | 69.3% | 3.8% |
| S9S14 | 72.4% | 0.6% | 8.5% | 1.0% | 12.4% | 34.4% |
| S9S15 | 6.6% | 1.9% | 12.9% | 0.6% | 7.1% | 4.3% |
| Lipofectamine Stem | 17.3% | | | | | |

Abbreviation list:

| | |
|---|---|
| DMEM | Basic high-glucose medium |
| DNA | deoxyribonucleic acid |
| DOPE | dioleoylphosphatidylethanolamine |
| DSPC | Distearoyl Phosphatidylcholine |
| PEG2000-DMG | 1,2-dimyristoyl-rac-glycero-3-methoxypolyethylene glycol-2000 |
| GFP | green fluorescent protein |
| EGFP | enhanced GFP |
| KD | kilodalton |
| RNA | ribonucleic acid |
| THF | tetrahydrofuran |
| DIPEA | N,N-diisopropylethylamine |
| PBS | phosphate buffered solution |

Various modifications of the invention in addition to those described herein will become apparent to those skilled in the art from the foregoing description. Such modifications are intended to fall within the scope of the appended claims. All references cited in this application (including all patents, patent applications, journal articles, books and any other publications) are incorporated in their entirety herein by reference.

## Claims

1. An amino lipid compound, wherein the amino lipid compound is a compound represented by Formula I:

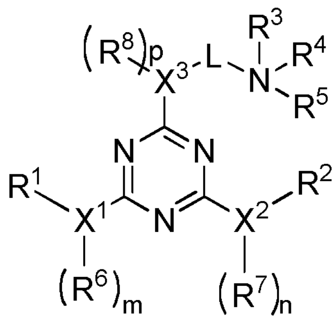

( I )

wherein:

$R^1$ and $R^2$ are the same or different from each other, and are each independently selected from the group consisting of $C_6$-$C_{24}$ alkyl, $C_6$-$C_{24}$ alkenyl, $C_6$-$C_{24}$ alkynyl and $C_4$-$C_{24}$ acyl, wherein the $C_6$-$C_{24}$ alkyl, the $C_6$-$C_{24}$ alkenyl, the $C_6$-$C_{24}$ alkynyl and the $C_4$-$C_{24}$ acyl are optionally substituted with $C_1$-$C_6$ hydrocarbyl;
$X^1$, $X^2$ and $X^3$ are the same or different from each other, and are each independently selected from the group consisting of C, N, O, S, S=O, $S(=O)_2$ and S-S;
when $X^1$ is C, m=2, and two $R^6$ are the same or different from each other; when $X^1$ is N, m=1; when $X^1$ is O, S, S=O, $S(=O)_2$ or S-S, m=0;
when $X^2$ is C, n=2, and two $R^7$ are the same or different from each other; when $X^2$ is N, n=1; when $X^2$ is O, S, S=O, $S(=O)_2$ or S-S, n=0;
when $X^3$ is C, p=2, and two $R^8$ are the same or different from each other; when $X^3$ is N, p=1; when $X^3$ is O, S, S=O, $S(=O)_2$ or S-S, p=0;
$R^3$ and $R^4$ are the same or different from each other, and are each independently selected from $C_1$-$C_{12}$ alkyl, $C_2$-$C_{12}$ alkenyl and $C_2$-$C_{12}$ alkynyl, wherein the $C_1$-$C_{12}$ alkyl, the $C_2$-$C_{12}$ alkenyl and the $C_2$-$C_{12}$ alkynyl are optionally substituted with $C_1$-$C_6$ hydrocarbyl, or $R^3$ and $R^4$ bind to each other to form an optionally substituted 4-10 membered heterocycle containing 1 to 6 heteroatoms selected from nitrogen, sulfur, and oxygen;
$R^5$ is absent, or $R^5$ is hydrogen or $C_1$-$C_{12}$ alkyl to provide a quaternary amine;
$R^6$, $R^7$, $R^8$ are hydrogen or $C_1$-$C_{12}$ alkyl;
L is $C_1$-$C_{12}$ alkylene, $C_2$-$C_{12}$ alkenylene or $C_2$-$C_{12}$ alkynylene, wherein the $C_1$-$C_{12}$ alkylene, the $C_2$-$C_{12}$ alkenylene and the $C_2$-$C_{12}$ alkynylene are optionally substituted with one or more substituents selected from the group consisting of hydrocarbyl, carboxyl, acyl and alkoxy, or L is an optionally substituted 4-10 membered heterocycle containing heteroatoms selected from nitrogen, sulfur, and oxygen.

2. The amino lipid compound according to claim 1, wherein:

$X^1$ is C, $R^6$ is H, and m=2; or $X^1$ is N, $R^6$ is H, and m=1; or $X^1$ is O, S, S=O, $S(=O)_2$ or S-S, and m=0; and/or
$X^2$ is C, $R^7$ is H, and n=2; or $X^2$ is N, $R^7$ is H, and n=1; or $X^2$ is O, S, S=O, $S(=O)_2$ or S-S, n=0; and/or
$X^3$ is C, $R^8$ is H, and p=2; or $X^3$ is N, $R^8$ is H, and p=1; or $X^3$ is O, S, S=O, $S(=O)_2$ or S-S, and p=0.

3. The amino lipid compound according to claim 1, wherein:

$X^1$ and $X^2$ are the same or different from each other, and are each independently N or S;
when $X^1$ is N, m=1; when $X^1$ is S, m=0;
when $X^2$ is N, n=1; when $X^2$ is S, n=0; and/or
$X^3$ is N and p=1, or $X^3$ is O and p=0; and/or
$R^5$ is absent; and/or
L is $C_1$-$C_{12}$ alkylene, wherein the $C_1$-$C_{12}$ alkylene is optionally substituted with $C_1$-$C_6$ hydrocarbyl, for example, L is $(CH_2)_q$, wherein q is an integer of from 1 to 12, such as an integer of from 1 to 8 or from 1 to 6.

4. The amino lipid compound according to claim 3, wherein:

when m=1, $R^6$ is hydrogen; and/or
when n=1, $R^7$ is hydrogen; and/or
$R^8$ is hydrogen.

**5.** The amino lipid compound according to claim 1, 3 or 4, wherein:

$R^1$ and $R^2$ are the same or different from each other, and are each independently $C_6$-$C_{24}$ alkyl or $C_6$-$C_{24}$ alkenyl;
$X^1$ is N, $R^6$ is H, and m=1; or $X^1$ is S, and m=0;
$X^2$ is N, $R^7$ is H, and n=1; or $X^2$ is S, and n=0;
$X^3$ is N, $R^8$ is H, and p=1;
$R^3$ and $R^4$ are the same or different from each other, and are each independently $C_1$-$C_{12}$ alkyl wherein the $C_1$-$C_{12}$ alkyl is optionally substituted with $C_1$-$C_6$ hydrocarbyl, or $R^3$ and $R^4$ bind to each other to form an optionally substituted 4-10 membered heterocycle containing 1 to 6 heteroatoms selected from nitrogen, sulfur, and oxygen; and
$R^5$ is absent.

**6.** The amino lipid compound according to any one of claims 1 to 5, wherein:
L is $C_1$-$C_4$ alkylene, wherein the $C_1$-$C_4$ alkylene is optionally substituted with $C_1$-$C_6$ hydrocarbyl, for example, L is $C_2$-$C_4$ alkylene, such as $(CH_2)_q$, wherein q is 1, 2, 3 or 4.

**7.** The amino lipid compound according to any one of claims 1 to 6, wherein:
$R^1$ and $R^2$ are the same or different from each other, and are each independently $C_6$-$C_{18}$ alkyl or $C_6$-$C_{18}$ alkenyl.

**8.** The amino lipid compound according to any one of claims 1 and 3 to 7, wherein: m=0 or 1, n=0 or 1, and

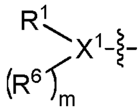 and 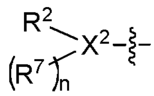

are the same or different from each other, and are each independently one selected from S6, S7, S8, S9, S10, S11, S12, S14, S15, S16, S18, S19, S20, N6, N7, N8, N9, N11, N12, N13, N15, N16, N18 and $^2N_{12}$:

S6: $CH_3(CH_2)_5S$-; S7: $CH_3(CH_2)_6S$-; S8: $CH_3(CH_2)_7S$-;
S9: $CH_3(CH_2)_8S$-; S10: $CH_3(CH_2)_9S$-; S11: $CH_3(CH_2)_{10}S$-;
S12: $CH_3(CH_2)_{11}S$-; S14: $CH_3(CH_2)_{13}S$-; S15: $CH_3(CH_2)_{14}S$-;
S16: $CH_3(CH_2)_{15}S$-; S18: $CH_3(CH_2)_{17}S$-;

S19: 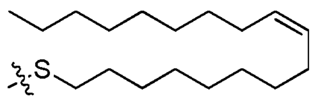; S20: 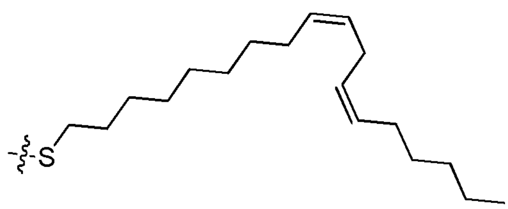;

N6: $CH_3(CH_2)_5NH$-; N7: $CH_3(CH_2)_6NH$-; N8: $CH_3(CH_2)_7NH$-;
N9: $CH_3(CH_2)_8NH$-; N11: $CH_3(CH_2)_{10}NH$-; N12: $CH_3(CH_2)_{11}NH$-;
N13: $CH_3(CH_2)_{12}NH$-; N15: $CH_3(CH_2)_{14}NH$-; N16: $CH_3(CH_2)_{15}NH$-;
N18: $CH_3(CH_2)_{17}NH$-; $^2N_{12}$: $(CH_3(CH_2)_{11})_2N$-.

**9.** The amino lipid compound according to any one of claims 1 and 3 to 8, wherein: p=1, and

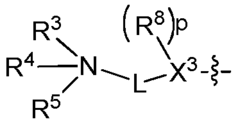

is one selected from D1, D2, D3, D4, D5, D6, D7, D8, D9 and D10:

D1: 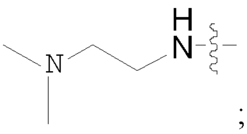; D2: 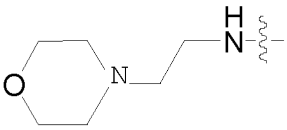; D3: 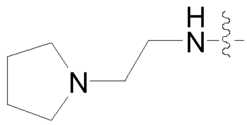;

D4: 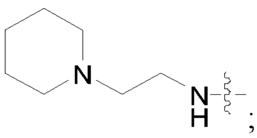; D5: 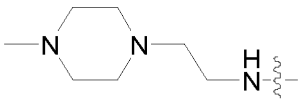; D6: 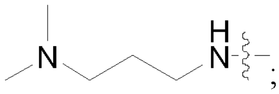;

D7: 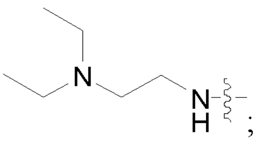; D8: 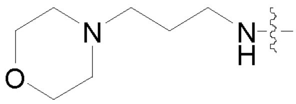; D9: 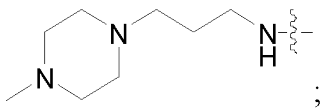;

D10: 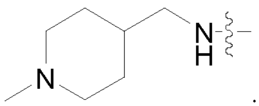.

**10.** The amino lipid compound according to any one of claims 1 and 3 to 9, wherein:

$X^3$ is O;
p is 0;
$R^5$ is absent; and

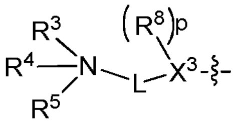

is one selected from O1, O2, O3, O4, O5, O6, O7, O8, O9 and O10:

O1: 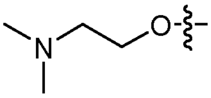; O2: 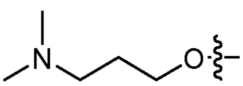; O3: 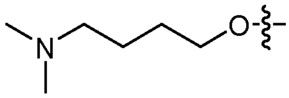;

O4: 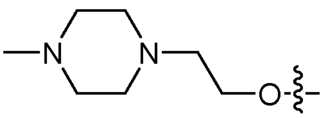; O5: 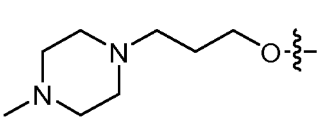; O6: 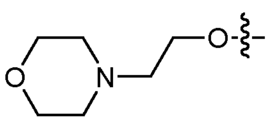;

O7: 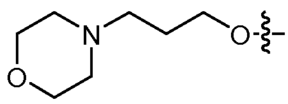 ; O8: 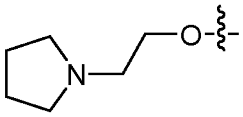 ; O9: 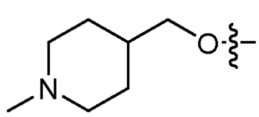 ;

O10: 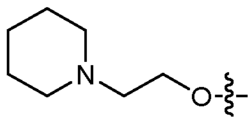 .

**11.** A method for preparing the amino lipid compound according to any one of claims 1 to 10, the method comprising the steps of:

(1) performing a first reaction between cyanuric chloride and a compound represented by $R^1(R^6)_m$-$X^1H$ at a temperature of -40°C to 30°C (preferably -30°C to 30°C) in the presence of a base as an acid-binding agent to obtain a first intermediate of Formula I-1;

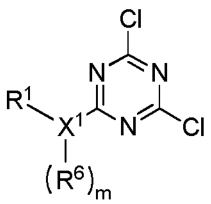

( **I-1** )

(2) with or (preferably) without separating the first intermediate, performing a second reaction between the first intermediate and a compound represented by $R^2(R^7)_n$-$X^2H$ at room temperature or under a heating condition in the presence of a base as an acid-binding agent to obtain a second intermediate of Formula 1-2;

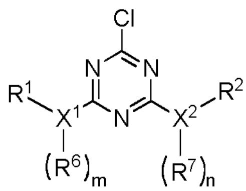

( **I-2** )

(3) with or (preferably) without separating the second intermediate, performing a third reaction between the second intermediate and a diamine represented by $HX^3(R^8)_p$-L-$NR^3R^4R^5$ under a heating condition, preferably further in the presence of a base as an acid-binding agent, to obtain the amino lipid compound of Formula I;

wherein the definitions of $X^1$, $X^2$, $X^3$, $R^1$, $R^2$, $R^3$, $R^4$, $R^5$, $R^6$, $R^7$, $R^8$, m, n and p are the same as the definitions thereof in any one of claims 1 to 10.

**12.** Use of the amino lipid compound according to any one of claims 1 to 10 for preparing lipid particles, wherein the lipid particles are lipid nanoparticles, liposomes, multilayered vesicles or micelles; more preferably, the lipid particles are lipid nanoparticles.

**13.** Lipid particles comprising the amino lipid compound according to any one of claims 1 to 10; preferably, the lipid particles are lipid nanoparticles, liposomes, multilayered vesicles or micelles; more preferably, the lipid particles are lipid nanoparticles.

**14.** The lipid particles according to claim 13, wherein the lipid particles further contain one or more of a helper lipid, a sterol and a bioactive agent;

preferably, the lipid particles further contain polyethylene glycol (PEG)-lipid; and/or

preferably, the molar ratio of the amino lipid compound to the helper lipid in the lipid particles is about (2 to 10):1, preferably about (3 to 8):1, more preferably about (4 to 6):1, such as about 4:1, about 4.5:1 or about 5:1; and/or

preferably, the molar ratio of the amino lipid compound to the sterol in the lipid particles is about (0.5 to 1.5):1, preferably about (1 to 1.4):1, such as about (1 to 1.3):1; and/or

preferably, the molar ratio of the amino lipid compound to the PEG-lipid in the lipid particles is about (9 to 42):1, preferably about (12 to 38):1, more preferably about (16 to 36):1, such as about (18 to 34):1; and/or

preferably, the helper lipid is a non-cationic lipid; more preferably, the helper lipid is a non-cationic phospholipid; and further preferably, the non-cationic lipid is DOPE, DSPC or a combination thereof; and/or

preferably, the sterol is one or more selected from the group consisting of cholesterol, sitosterol, stigmasterol and ergosterol, preferably cholesterol; and/or

preferably, the PEG-lipid is one or more selected from the group consisting of PEG1000-DMG, PEG5000-DMG, PEG2000-DMG and PEG2000-DSPE, preferably PEG2000-DMG; and/or

preferably, the bioactive agent is one or more of a nucleic acid, an antitumor agent, an antibiotic, an immunomodulator, an anti-inflammatory agent, an agent acting on the central nervous system, an antigen or a fragment thereof, a peptide, a protein, an antibody, a vaccine and a small molecule; more preferably, the nucleic acid is RNA, messenger RNA (mRNA), antisense oligonucleotide, DNA, plasmid, ribosomal RNA (rRNA), microRNA (miRNA), transfer RNA (tRNA), small interfering RNA (siRNA) and small nuclear RNA (snRNA).

**15.** Use of the lipid particles according to claim 12 or 13 in the preparation of a medicament for gene therapy, gene vaccination, antisense therapy or a therapy by RNA interference;

preferably, the lipid particles are used as vectors for encapsulating a bioactive agent;

preferably, the gene therapy is useful for the treatment of a cancer and a genetic disease; more preferably, the cancer is one or more of lung cancer, gastric cancer, liver cancer, esophageal cancer, colon cancer, pancreatic cancer, brain cancer, lymphatic cancer, hematologic cancer or prostate cancer, and the genetic disease is one or more of hemophilia, thalassemia and Gaucher disease;

preferably, the gene vaccination is useful for the treatment of a cancer, allergy, toxicity and pathogen infection; more preferably, the pathogen is one or more of viruses, bacteria or fungi.

**16.** Use of the lipid particles according to claim 12 or 13 in the preparation of a medicament for nucleic acid transfer, preferably, the nucleic acid is RNA, messenger RNA (mRNA), antisense oligonucleotide, DNA, plasmid, ribosomal RNA (rRNA), microRNA (miRNA), transfer RNA (tRNA), small interfering RNA (siRNA) and small nuclear RNA (snRNA).

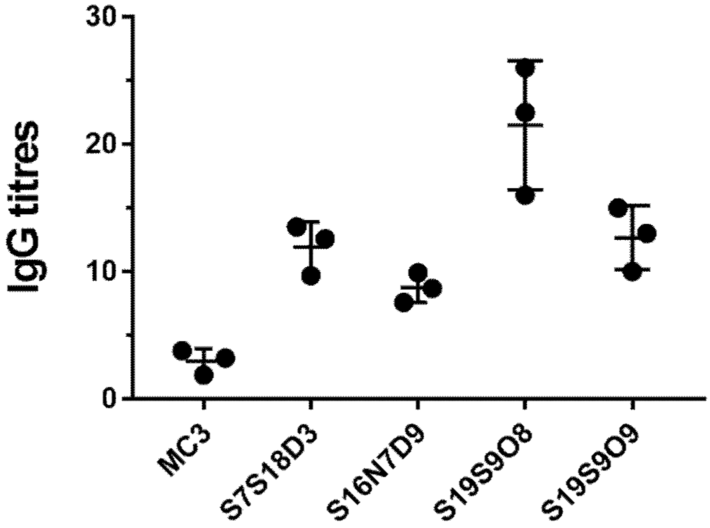
IgG titres
30
20
10
0
MC3
S7S18D3
S16N7D9
S19S9O8
S19S9O9

Fig. 1

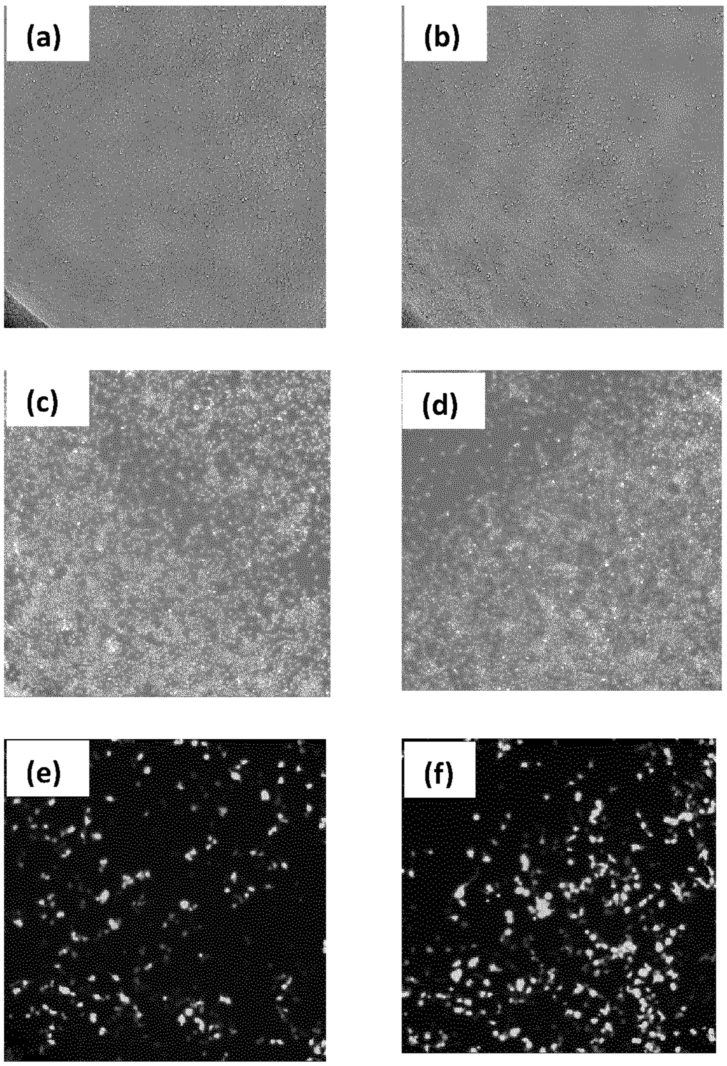
(a)
(b)
(c)
(d)
(e)
(f)

Fig. 2

## INTERNATIONAL SEARCH REPORT

International application No. **PCT/CN2021/077852**

**A. CLASSIFICATION OF SUBJECT MATTER**

C07D 251/52(2006.01)i; C07D 251/46(2006.01)i; C07D 251/70(2006.01)i; A61K 31/53(2006.01)i; A61K 9/127(2006.01)i; C12N 15/88(2006.01)i; A61P 35/00(2006.01)i; A61P 31/12(2006.01)i; A61P 37/00(2006.01)i

According to International Patent Classification (IPC) or to both national classification and IPC

**B. FIELDS SEARCHED**

Minimum documentation searched (classification system followed by classification symbols)

C07D; A61K; C12N 15/88; A61P

Documentation searched other than minimum documentation to the extent that such documents are included in the fields searched

Electronic data base consulted during the international search (name of data base and, where practicable, search terms used)

CNABS, DWPI, SIPOABS, CAPLUS, REGISTRY, MARPAT, CNKI, 万方: 三嗪, triazine, 氨基脂质, 胺基脂质, amino lipid, 载体, vector, 传递, delivery, structure search

**C. DOCUMENTS CONSIDERED TO BE RELEVANT**

| Category* | Citation of document, with indication, where appropriate, of the relevant passages | Relevant to claim No. |
|---|---|---|
| X | CN 102389744 A (DALIAN UNIVERSITY OF TECHNOLOGY) 28 March 2012 (2012-03-28)<br>see description embodiments 1-4 | 1-9 |
| X | US 4629752 A (THE B. F. GOODRICH COMPANY) 16 December 1986 (1986-12-16)<br>see description column 15 lines 20-30 | 1-8 |
| X | US 4269832 A (AMERICAN CYANAMID CO.) 26 May 1981 (1981-05-26)<br>see embodiments 5-11 | 1-7, 9 |
| A | US 2010137539 A1 (LAI LONG-LI et al.) 03 June 2010 (2010-06-03)<br>see entire description | 1-16 |
| X | CN 110669019 A (NORTH UNIVERSITY OF CHINA) 10 January 2020 (2020-01-10)<br>see claim 1 | 1-9 |
| A | WO 2019155094 A1 (INTERNA TECH. B V) 15 August 2019 (2019-08-15)<br>see entire description | 1-16 |
| A | CN 106883158 A (GUANGZHOU INSTITUTES OF BIOMEDICINE AND HEALTH, CHINESE ACADEMY OF SCIENCES) 23 June 2017 (2017-06-23)<br>see entire description | 1-16 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 May 2021** | **08 June 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/077852**

**C. DOCUMENTS CONSIDERED TO BE RELEVANT**

| Category* | Citation of document, with indication, where appropriate, of the relevant passages | Relevant to claim No. |
|---|---|---|
| X | QIAO, Weihong et al. "Synthesis and surface activity properties of symmetric double chains alkylbetaine surfactants derived from s-triazine" *Colloids and Surfaces, A: Physicochemical and Engineering Aspects,* Vol. 405, 05 July 2012 (2012-07-05), ISSN: 0927-7757, pp. 45-50 | 1-9 |
| X | JING, Lishuai et al. "Design and Surface/Interfacial Properties of Asymmetric Triazine Carboxyl Betaine Surfactants" *Journal of Surfactants and Detergents,* Vol. 17, No. 4, 31 July 2017 (2017-07-31), ISSN: 1097--395, pp. 629-636 | 1-9, 11 |

Form PCT/ISA/210 (second sheet) (January 2015)

**INTERNATIONAL SEARCH REPORT**
**Information on patent family members**

International application No.
**PCT/CN2021/077852**

| Patent document cited in search report | | | Publication date (day/month/year) | Patent family member(s) | | | Publication date (day/month/year) |
|---|---|---|---|---|---|---|---|
| CN | 102389744 | A | 28 March 2012 | CN | 102389744 | B | 21 August 2013 |
| US | 4629752 | A | 16 December 1986 | EP | 0209871 | A1 | 28 January 1987 |
| | | | | DE | 3650387 | T2 | 29 February 1996 |
| | | | | AT | 56968 | T | 15 October 1990 |
| | | | | EP | 0209871 | B1 | 26 September 1990 |
| | | | | DE | 3674497 | D1 | 31 October 1990 |
| | | | | EP | 0361536 | B1 | 06 September 1995 |
| | | | | DE | 3650387 | D1 | 12 October 1995 |
| | | | | AT | 127457 | T | 15 September 1995 |
| | | | | JP | S6270372 | A | 31 March 1987 |
| | | | | JP | H08821 | B2 | 10 January 1996 |
| | | | | CA | 1265797 | A | 13 February 1990 |
| | | | | EP | 0361536 | A1 | 04 April 1990 |
| US | 4269832 | A | 26 May 1981 | | None | | |
| US | 2010137539 | A1 | 03 June 2010 | TW | I384010 | B | 01 February 2013 |
| | | | | US | 2013237703 | A1 | 12 September 2013 |
| | | | | TW | 201020277 | A | 01 June 2010 |
| CN | 110669019 | A | 10 January 2020 | | None | | |
| WO | 2019155094 | A1 | 15 August 2019 | AU | 2019218557 | A1 | 20 August 2020 |
| | | | | US | 2021038732 | A1 | 11 February 2021 |
| | | | | EP | 3752164 | A1 | 23 December 2020 |
| | | | | CA | 3088321 | A1 | 15 August 2019 |
| | | | | CN | 111936150 | A | 13 November 2020 |
| CN | 106883158 | A | 23 June 2017 | CN | 106883158 | B | 23 August 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Cell line: embryonic stem cells (hESC2, cultured according to reported methods. *Stem Cells,* 2005, vol. 23, 544-549
- Cell line: cardiomyocytes (obtained from induced differentiation of hESC according to reported methods. *J. Mol. Cell. Cardiol.,* 2011, vol. 51, 288-298